# EUROPEAN PATENT APPLICATION

(11) **EP 4 066 850 A2**
(43) Date of publication of application: **05.10.2022**
(21) Application number: 22150813.8
(22) Date of filing: 22.02.2018
(51) Int. Cl.: A61K 38/19, A61K 38/04, A61K 38/08, A61K 38/10, A61K 38/17, A61K 45/06, A61P 21/00, A61P 9/00, A61P 11/00, A61P 13/12

(54) **PEPTIDES AND METHODS OF TREATING DYSTROPHY-RELATED DISORDERS USING THE SAME**

(30) Priority: 22.02.2017 US 201762462292 P
(62) Divisional of application: 18756867.0
(71) Applicant: Bioincept LLC, Cherry Hill, NJ 08003 (US)
(72) Inventor: BARNEA, Eytan, R., New York N 10016 (US)
(74) Representative: Heath, Abigail

(57) **Abstract**

The disclosure relates to a pharmaceutical composition comprising any one or combination of PIF peptides or analogs or pharmaceutically acceptable salts thereof. Methods of treating a dystrophy-related disorder using the one or a combination of PIF peptide or analogs thereof or pharmaceutically acceptable salts thereof are also disclosed.

## Description

### CROSS-REFERENCE TO RELATED APPLICATIONS

This application claims priority to and the benefit of U.S. Provisional Application No. 62/462,292, filed February 22, 2017, which is hereby incorporated by reference in its entirety.

### FIELD

The present disclosure generally relates to compositions and methods for treatment of Duchenne's Muscular Dystrophy and dystrophy-related disorders. The disclosure also relates to pre-implantation factor (PIF) mutants and methods of treatment using the same, including the treatment of Duchenne's Muscular Dystrophy.

### BACKGROUND

Prelmplantation factor (PIF), a 15-amino acid peptide secreted by viable mammalian embryos, is found in maternal circulation. Synthetic PIF (sPIF) is currently used in clinical trials for treatment of autoimmune hepatitis. Muscular-dystrophy-related disorders, including, Duchenne muscular dystrophy (DMD), is a progressive lethal, X-linked disease of skeletal and cardiac muscles caused by mutation of genes. Patients suffering with one of these disorders suffers from muscle degeneration. In the case of DMD, loss of function of dystrophin leads to damage and ultimately to: (1) muscle fibers waste; and (2) the impairment of satellite cells to undergo asymmetric division, which is essential for muscle regeneration. Degenerating muscles accumulate connective tissue, a process commonly referred to as fibrosis. Present therapeutic strategies are multiple but currently have limited success. As for gene therapy although may be promising several obstacles have to be overcome to make it a clinical reality. Additional strategy is the use of agents that lower inflammation, and improve muscle progenitor cells regeneration. Corticosteroids are the main stay in therapy also sodium cromoglycate, is used to lower inflammation. Other current suggested intervention are clenbutrol to improve muscle function and dantrolene to maintain calcium flux. Each of these treatments do not greatly improve fibrotic tissue and each has poor side effects.

### SUMMARY

Our results suggest that treatment comprising PIF, mutants thereof or pharmaceutical salts thereof promoted differentiation of mouse myoblasts and human satellite cells as demonstrated by increases in MyoD and MyHC expression and a decrease in Pax-7 levels. PIF treatment also increased the myoblasts fusion into multinucleated myotubes. Additionally, PIF treatment increased levels of utrophin, a dystrophin protein homologue via downregulation of let-7. We further examined the therapeutic effects of PIF administration in DMD using mdx mice. PIF administration significantly decreased the serum levels of CK, a marker of muscle damage and the level of collagen type IV in the mdx mice diaphragms, indicating a significant decrease in muscle fibrosis. Considering the known anti-inflammatory effects of PIF, together with its current effects on cell regeneration and inhibition of muscle damage and fibrosis, we propose that PIF can be a potential therapeutic agent for the treatment of DMD.

The present disclosure relates to a method of treating or preventing a dystrophy-related disorder, such as Duchenne's Muscular Dystrophy, in a subject in need thereof, the method comprising administering to the subject at least one pre-implantation factor (PIF) peptide, a mimetic thereof, an analog thereof, or a pharmaceutically acceptable salt thereof in a therapeutically effective amount.

In some embodiments, the step of administering to the subject at least one PIF peptide, a mimetic thereof, an analog thereof, or a pharmaceutically acceptable salt thereof comprises administering a therapeutically effective dose of the at least one PIF molecule, an analog thereof, or a pharmaceutically acceptable salt thereof.

In some embodiments, the step of administering to the subject at least one PIF peptide, a mimetic thereof, an analog thereof, or a pharmaceutically acceptable salt thereof comprises administering a therapeutically effective dose of the PIF peptide, an analog thereof, or pharmaceutically acceptable salt thereof from about 0.001 mg/kg to about 200 mg/kg.

In some embodiments, the step of administering to the subject at least one PIF peptide, a mimetic thereof, an analog thereof, or a pharmaceutically acceptable salt thereof comprises administering a therapeutically effective dose of the PIF peptide, an analog thereof, or pharmaceutically acceptable salt thereof from about 0.5 mg/kg to about 5 mg/kg.

In some embodiments, the at least the PIF peptide, a mimetic thereof, an analog thereof, or pharmaceutically acceptable salt thereof comprises a chemical targeting moiety and/or a radioactive moiety.

In some embodiments, the at least one inhibitor of nuclear translocation of betacatenin or pharmaceutically acceptable salt thereof comprises at least one radioactive moiety comprising at least one or a combination of the following isotopes: ²H, ³H, ¹³C, ¹⁴C, ¹⁵N, ¹⁶O, ¹⁷O, ³¹P, ³²P, ³⁵S, ¹⁸F, and ³⁶Cl.

In some embodiments, the method further comprises administering at least one analgesic and/or one anti-inflammatory compound.

In some embodiments, the method further comprises administering at least one analgesic and or one anti-inflammatory compound before, after, or simultaneously with the administration of a therapeutically effective dose of at least one PIF peptide, an analog thereof or pharmaceutically acceptable salt thereof.

In some embodiments, the therapeutically effective dose is from about 1.0 mg/kg to about 5.5 mg/kg, wherein kg is kilograms of the subject and mg is milligrams of the therapeutically effective dose. In some embodiments, the therapeutically effective dose is from about 1.0 mg/kg to about 5.0 mg/kg, wherein kg is kilograms of the subject and mg is milligrams of the therapeutically effective dose. In some embodiments, the therapeutically effective dose is from about 1.0 mg/kg to about 4.5 mg/kg, wherein kg is kilograms of the subject and mg is milligrams of the therapeutically effective dose. In some embodiments, the therapeutically effective dose is from about 1.0 mg/kg to about 4.0 mg/kg, wherein kg is kilograms of the subject and mg is milligrams of the therapeutically effective dose. In some embodiments, the therapeutically effective dose is from about 1.0 mg/kg to about 3.5 mg/kg, wherein kg is kilograms of the subject and mg is milligrams of the therapeutically effective dose. In some embodiments, the therapeutically effective dose is administered subcutaneously, intravenously, intraperitoneally, topically, orally, sublingually, intranasally, or intramuscularly. In some embodiments, the therapeutically effective dose is administered once a week, twice a week, three times a week, four times a week, or five days per week, and optionally, wherein the dose is administered once in two, three, four or five days in succession. In some embodiments, the therapeutically effective dose is administered once every two weeks, once every three weeks, once every four weeks, once every five weeks, once every six weeks, once every seven weeks, once every eight weeks, once every nine weeks, once every ten weeks, once every eleven weeks, or once every twelve weeks. In some embodiments, the therapeutically effective dose is administered once a month, twice a month, three times a month, four times a month, or five times a month. In some embodiments, the therapeutically effective dose is administered once a year, twice a year, three times a year, four times a year, five times a year, six times a year, seven times a year, eight times a year, nine times a year, ten times per year, or eleven times per year.

In some embodiments, the compositions or pharmaceutical compositions comprise a PIF peptide comprising SEQ ID NO:1, SEQ ID NO:2, and/or SEQ ID NO:3, or pharmaceutically acceptable salts thereof. In some embodiments, the PIF peptide comprises SEQ ID NO:4 or a pharmaceutically acceptable salt thereof. In some embodiments, the PIF peptide comprises SEQ ID NO:5 or a pharmaceutically acceptable salt thereof. In some embodiments, the PIF peptide comprises SEQ ID NO:6 or a pharmaceutically acceptable salt thereof. In some embodiments, the PIF peptide comprises SEQ ID NO:7 or a pharmaceutically acceptable salt thereof. In some embodiments, the PIF peptide comprises SEQ ID NO:8 or a pharmaceutically acceptable salt thereof. In some embodiments, the PIF peptide comprises SEQ ID NO:9 or a pharmaceutically acceptable salt thereof. In some embodiments, the PIF peptide comprises SEQ ID NO:10 or a pharmaceutically acceptable salt thereof. In some embodiments, the PIF peptide comprises SEQ ID NO:11 or a pharmaceutically acceptable salt thereof. In some embodiments, the PIF peptide comprises SEQ ID NO:12 or a pharmaceutically acceptable salt thereof. In some embodiments, the PIF peptide comprises SEQ ID NO:13 or a pharmaceutically acceptable salt thereof. In some embodiments, the PIF peptide comprises SEQ ID NO:14 or a pharmaceutically acceptable salt thereof. In some embodiments, the PIF peptide comprises SEQ ID NO:15 or a pharmaceutically acceptable salt thereof. In some embodiments, the PIF peptide comprises SEQ ID NO:16 or a pharmaceutically acceptable salt thereof. In some embodiments, the PIF peptide comprises SEQ ID NO:17 or a pharmaceutically acceptable salt thereof. In some embodiments, the PIF peptide comprises SEQ ID NO:18 or a pharmaceutically acceptable salt thereof. In some embodiments, the PIF peptide comprises SEQ ID NO:19 or a pharmaceutically acceptable salt thereof. In some embodiments, the PIF peptide comprises SEQ ID NO:20 or a pharmaceutically acceptable salt thereof. In some embodiments, the PIF peptide comprises SEQ ID NO:21 or a pharmaceutically acceptable salt thereof. In some embodiments, the PIF peptide comprises SEQ ID NO:22 or a pharmaceutically acceptable salt thereof. In some embodiments, the PIF peptide comprises SEQ ID NO:23 or a pharmaceutically acceptable salt thereof. In some embodiments, the PIF peptide comprises SEQ ID NO:24 or a pharmaceutically acceptable salt thereof. In some embodiments, the PIF peptide comprises SEQ ID NO:25 or a pharmaceutically acceptable salt thereof. In some embodiments, the PIF peptide comprises SEQ ID NO:26 or a pharmaceutically acceptable salt thereof. In some embodiments, the PIF peptide comprises SEQ ID NO:27 or a pharmaceutically acceptable salt thereof. In some embodiments, the PIF peptide comprises SEQ ID NO:28 or a pharmaceutically acceptable salt thereof. In some embodiments, the PIF peptide comprises SEQ ID NO:29 or a pharmaceutically acceptable salt thereof.

In a further embodiment, a compound of the formula R₁-R₂-R₃-R₄ (SEQ ID NO:31) is provided, wherein R₁ is Pro or a mimetic of Pro, R₂ is Gly or a mimetic of Gly, R₃ is Ser or a mimetic of Ser, and R₄ is Ala or a mimetic of Ala. In alternative embodiments, the compound may comprise one or more of up to 11 additional amino acid residues. Embodiments of the present invention include those peptides derived from pre-implantation embryos that induces TH2 type cytokines like IL-10 synthesis or secretion from lymphocytes or other white blood cells and pharmacophores that binds specifically to PIF receptors (such but not limited to PGSA (A)VRIKPGSANKPSDD or (Q)VRIKPGSANKPSDD) or by substituting with D amino acids or by adding PEG. Preferably such peptides are from preimplantation embryos and increases TH2/TH1 ratio through increased number of lymphocytes containing the desired cytokines and or by preferential secretion or TH2 over THI cytokines into the media. Such pre-implantation embryo-derived peptide may be used to cause a shift from pro- inflammatory to anti- inflammatory activities in lymphocytes. In a further embodiment, the pharmaceutical composition comprises a compound of the formula MVRIK (SEQ ID NO: 32).

In some embodiments, the pharmaceutically acceptable carrier is sterile and pyrogen-free water or aqueous buffer, such as saline or Lactated Ringer's solution.

In some embodiments, the therapeutically effective dose is about 1.0 mg/kg, wherein kg is kilograms of the subject and mg is milligrams of the therapeutically effective dose. In some embodiments, the therapeutically effective dose is about 2.0 mg/kg, wherein kg is kilograms of the subject and mg is milligrams of the therapeutically effective dose. In some embodiments, the therapeutically effective dose is about 3.0 mg/kg, wherein kg is kilograms of the subject and mg is milligrams of the therapeutically effective dose. In some embodiments, the therapeutically effective dose is about 4.0 mg/kg, wherein kg is kilograms of the subject and mg is milligrams of the therapeutically effective dose. In some embodiments, the therapeutically effective dose is about 0.2 mg/kg, wherein kg is kilograms of the subject and mg is milligrams of the therapeutically effective dose. In some embodiments, the therapeutically effective dose is about 0.3 mg/kg, wherein kg is kilograms of the subject and mg is milligrams of the therapeutically effective dose. In some embodiments, the therapeutically effective dose is about 0.4 mg/kg, wherein kg is kilograms of the subject and mg is milligrams of the therapeutically effective dose. In some embodiments, the therapeutically effective dose is about 0.5 mg/kg, wherein kg is kilograms of the subject and mg is milligrams of the therapeutically effective dose. In some embodiments, the therapeutically effective dose is about 0.6 mg/kg, wherein kg is kilograms of the subject and mg is milligrams of the therapeutically effective dose. In some embodiments, the therapeutically effective dose is about 0.7 mg/kg, wherein kg is kilograms of the subject and mg is milligrams of the therapeutically effective dose. In some embodiments, the therapeutically effective dose is about 0.8 mg/kg, wherein kg is kilograms of the subject and mg is milligrams of the therapeutically effective dose.

The present disclosure also relates to a pharmaceutical composition comprising (i) a therapeutically effective dose of one or a combination of PIF peptide or analogs thereof or pharmaceutically acceptable salts thereof; and (ii) a pharmaceutically acceptable carrier.

In some embodiments, the composition further comprises a therapeutically effective dose of one or a plurality of active agents.

In some embodiments, the one or plurality of active agents is one or a combination of compounds chosen from: an anti-inflammatory compound, alpha-adrenergic agonist, antiarrhythmic compound, analgesic compound, and an anesthetic compound, or a hormone therapy.

In some embodiments, the therapeutically effective dose of one or a combination of PIF peptide or mimetics thereof or thereof analogs thereof or pharmaceutically acceptable salts thereof is about 1.0 mg/kg, wherein kg is kilograms of the subject and mg is milligrams of the therapeutically effective dose.

In some embodiments, the therapeutically effective dose of one or a combination of PIF peptide or mimetics thereof or analogs thereof or pharmaceutically acceptable salts thereof is about 2.0 mg/kg, wherein kg is kilograms of the subject and mg is milligrams of the therapeutically effective dose.

In some embodiments, the therapeutically effective dose of one or a combination of PIF peptide or analogs thereof or pharmaceutically acceptable salts thereof is about 3.0 mg/kg, wherein kg is kilograms of the subject and mg is milligrams of the therapeutically effective dose.

In some embodiments, the therapeutically effective dose of one or a combination of PIF peptide or analogs thereof or pharmaceutically acceptable salts thereof is about 4.0 mg/kg, wherein kg is kilograms of the subject and mg is milligrams of the therapeutically effective dose.

In some embodiments, wherein the therapeutically effective dose of one or a combination of PIF peptide or analogs thereof or pharmaceutically acceptable salts thereof is about 0.2 mg/kg, wherein kg is kilograms of the subject and mg is milligrams of the therapeutically effective dose.

In some embodiments, the therapeutically effective dose of one or a combination of PIF peptide or analogs thereof or pharmaceutically acceptable salts thereof is about 0.3 mg/kg, wherein kg is kilograms of the subject and mg is milligrams of the therapeutically effective dose.

In some embodiments, the therapeutically effective dose of one or a combination of PIF peptide or analogs thereof or pharmaceutically acceptable salts thereof is about 0.4 mg/kg, wherein kg is kilograms of the subject and mg is milligrams of the therapeutically effective dose.

In some embodiments, the therapeutically effective dose of one or a combination of PIF peptide or analogs thereof or pharmaceutically acceptable salts thereof is about 0.5 mg/kg, wherein kg is kilograms of the subject and mg is milligrams of the therapeutically effective dose.

In some embodiments, the therapeutically effective dose of one or a combination of PIF peptide or analogs thereof or pharmaceutically acceptable salts thereof is about 0.6 mg/kg, wherein kg is kilograms of the subject and mg is milligrams of the therapeutically effective dose.

In some embodiments, the therapeutically effective dose of one or a combination of PIF peptide or analogs thereof or pharmaceutically acceptable salts thereof is about 0.7 mg/kg, wherein kg is kilograms of the subject and mg is milligrams of the therapeutically effective dose.

In some embodiments, wherein the therapeutically effective dose of one or a combination of PIF peptide or analogs thereof or pharmaceutically acceptable salts thereof is about 0.8 mg/kg, wherein kg is kilograms of the subject and mg is milligrams of the therapeutically effective dose.

In some embodiments, the therapeutically effective dose of one or a combination of PIF peptide or analogs thereof or pharmaceutically acceptable salts thereof is about 0.9 mg/kg, wherein kg is kilograms of the subject and mg is milligrams of the therapeutically effective dose.

In some embodiments, the composition further comprises one or a plurality of stem cells. In some embodiments, the stem cell is an autologous stem cell.

In some embodiments, the pharmaceutical composition is administered via parenteral injection, subcutaneous injection, intravenous injection, intramuscular injection, intraperitoneal injection, transdermally, orally, buccally, ocular routes, intravaginally, by inhalation, by depot injections, or by implants.

In some embodiments, the compositions further comprise one or a combination of active agents chosen from: an anti-inflammatory compound, alpha-adrenergic agonist, antiarrhythmic compound, analgesic compound, and/or an anesthetic compound.

In some embodiments, the one or combination of active agents is selected from Table Y.

The present disclosure also relates to a method of improving the clinical outcome in a subject suffering with, diagnosed with or suspected of having Duchenne's Muscular Dystrophy comprising administering to the subject at least one pharmaceutical composition comprising: pre-implantation factor (PIF) peptide, an analog thereof, or a pharmaceutically acceptable salt thereof; and a pharmaceutically acceptable carrier.

The present disclosure relates to a method of treating Duchenne's Muscular Dystrophy in a human subject, wherein the human subject has been identified as having Duchenne's Muscular Dystrophy, the method comprising: administering to the subject at least one pharmaceutical composition comprising: pre-implantation factor (PIF) peptide, an analog thereof, or a pharmaceutically acceptable salt thereof; and a pharmaceutically acceptable carrier measuring CDK.

Any composition disclosed herein may be used in any method disclosed herein as a monotherapy or as a combination therapy of one or more disclosed compositions.

The present disclosure relates to a method of treating or preventing a dystrophy-related disorder from Table 2, the method comprising administering a therapeutically effective amount of a PIF peptide or pharmaceutically acceptable salt thereof to the subject. The present disclosure relates to a method of treating or preventing DMD, the method comprising administering a therapeutically effective amount of PIF, a modified PIF peptide or a pharmaceutically acceptable salt thereof to the subject.

The present disclosure relates to methods of treating and/or preventing fibrosis in a subject, the method comprising administering a therapeutically effective amount of PIF, a modified PIF peptide or a pharmaceutically acceptable salt thereof to the subject. In some embodiments, the method further comprises administration of one or a plurality of active agents to the subject before, contemporaneously with, or after administration of the therapeutically effective amount of the PIF, a modified PIF peptide or a pharmaceutically acceptable salt thereof. The present disclosure relates to methods of inhibiting the deposition of fibrotic tissue in a subject, the method comprising administering a therapeutically effective amount of PIF, a modified PIF peptide or a pharmaceutically acceptable salt thereof to the subject. In some embodiments, the fibrotic tissue is muscle tissue. In some embodiments, the subject is suffering from a dystrophin-related disorder. In some embodiments, the subject is suffering from a dystrophin-related disorder selected from Table 2.

The present disclosure relates to a method of increasing levels of utrophin in a subject, the method comprising administering a therapeutically effective amount of PIF, a modified PIF peptide or a pharmaceutically acceptable salt thereof to the subject. In some embodiments, the method comprises increasing levels of utrophin at or proximate to the neuromuscular junction within the subject.

The present disclosure also relates to a method of differentiating myoblasts in vitro or in vivo, the method comprising contacting cells in vivo or in vitro with an amount of PIF, a modified PIF peptide or a pharmaceutically acceptable salt thereof sufficient to cause differentiation of myoblasts. In some embodiments, if the method is performed in vivo, the method comprises administering to a subject a therapeutically effective amount of PIF, a modified PIF peptide or a pharmaceutically acceptable salt thereof. In some embodiments, the method further comprises administering to a subject a therapeutically effective amount of a PIF peptide or pharmaceutically acceptable salt thereof such that the myoblasts fuse into myotubes.

The present disclosure relates to a method of increasing myoblast fusion into multinucleated myotubes in a subject, the method comprising administering to the subject an amount of PIF, a modified PIF peptide or a pharmaceutically acceptable salt thereof sufficient to induce the formation of functional myotubes in the subject. In some embodiments, the subject has been diagnosed or is suspected of having a dystrophin-related disorder. In some embodiments, the subject is suffering from muscle degeneration. In some embodiments, the subject has been diagnosed with Duchenne's Muscular Dystrophy.

The present disclosure also relates to a method of decreasing the rate of muscle degeneration in a subject with a dystrophin-related disorder, the method comprising administering to the subject a therapeutically effective amount of PIF, a modified PIF peptide or a pharmaceutically acceptable salt thereof.

### BRIEF DESCRIPTION OF THE DRAWINGS

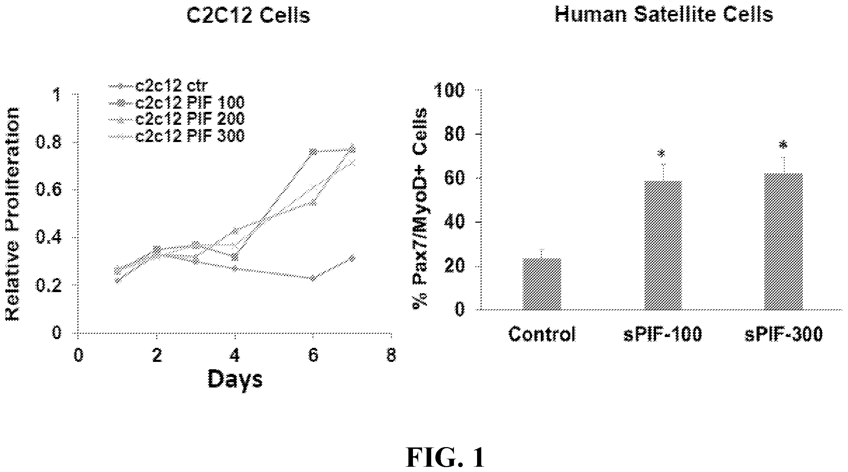

FIG. 1 is a series of graphs showing sPIF-induced proliferation of C2C12 myoblasts (left) and human satellite cells (right).

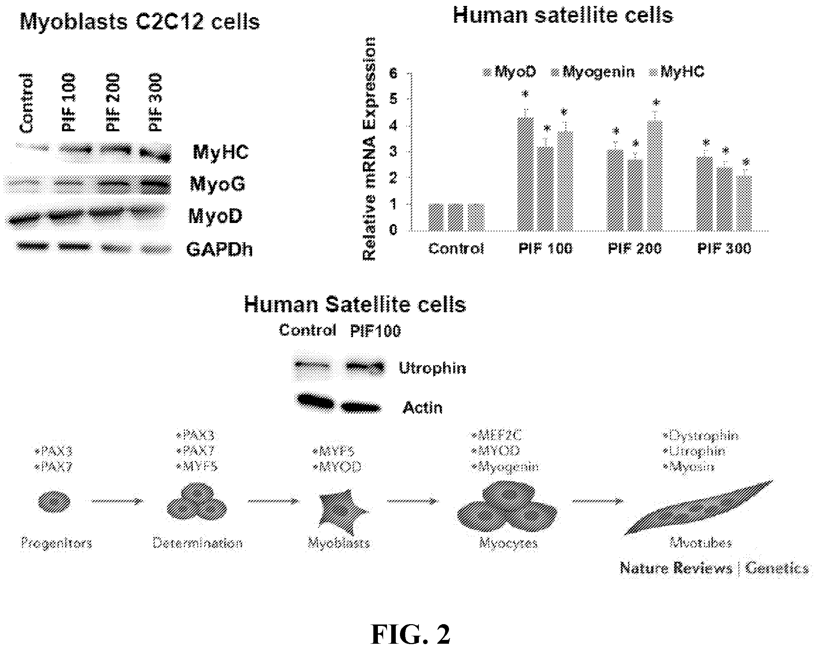

FIG. 2 is a series of graphs and pictures showing sPIF-induced muscle differentiation in C2C12 myoblasts (top left) and human satellite cells (top right). sPIF treatment also increased the level of utrophin (middle), a dystrophin protein homologue, and the myoblasts fusion into multinucleated myotubes (bottom).

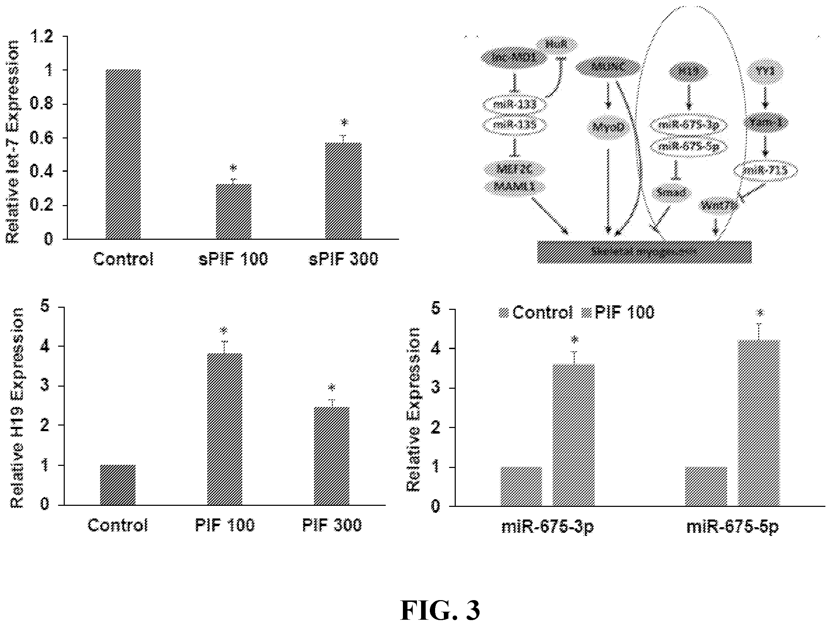

FIG. 3 is a series of graphs showing sPIF downregulation of let-7 (top left) and upregulation of H19 (bottom left) and miR-675 (bottom right) expression. This pathway is shown in the picture on the top right.

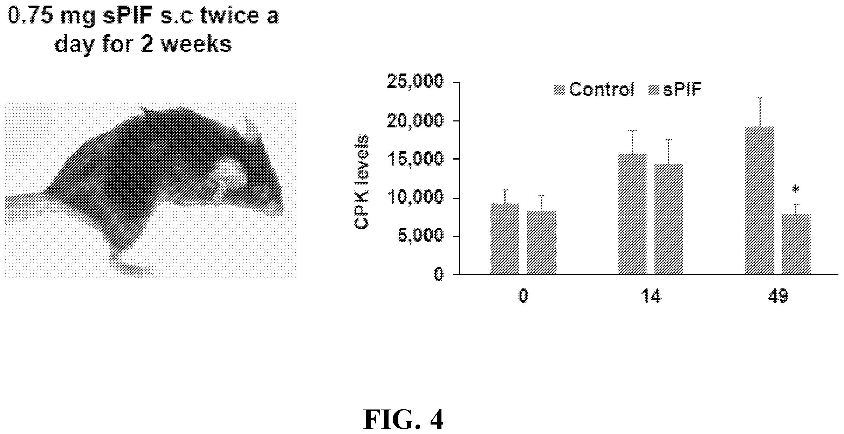

FIG. 4 is a picture of a representative mouse receiving sPIF administration in the mdx mouse model of DMD (left). PIF administration significantly decreased the serum levels of CK (right), a marker of muscle damage.

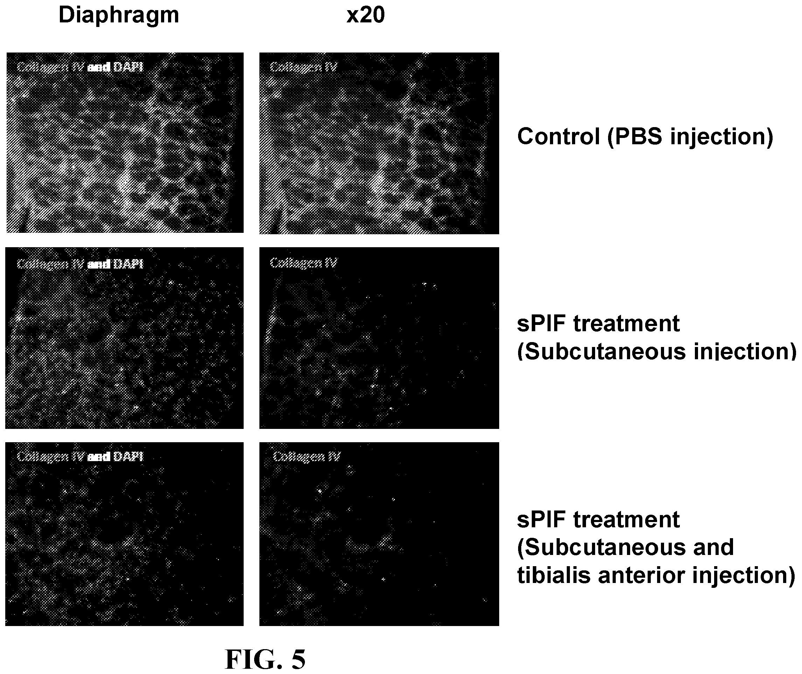

FIG. 5 is a series of representative pictures showing that sPIF treatment decreased tissue fibrosis in the diaphragm of mdx mice, as signified by the level of collagen type IV, indicating a significant decrease in muscle fibrosis.

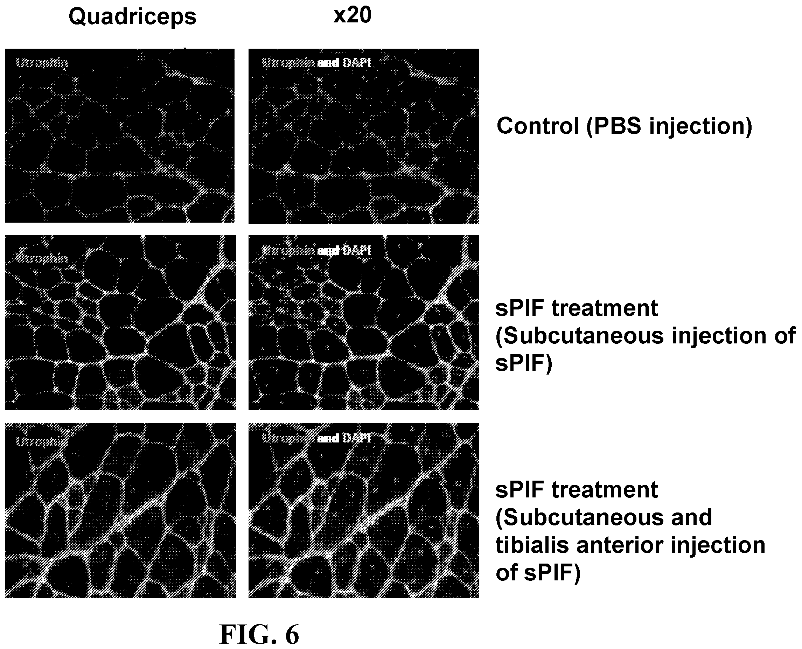

FIG. 6 is a series of representative pictures showing that sPIF treatment increased utrophin in the quadriceps of mdx mice.

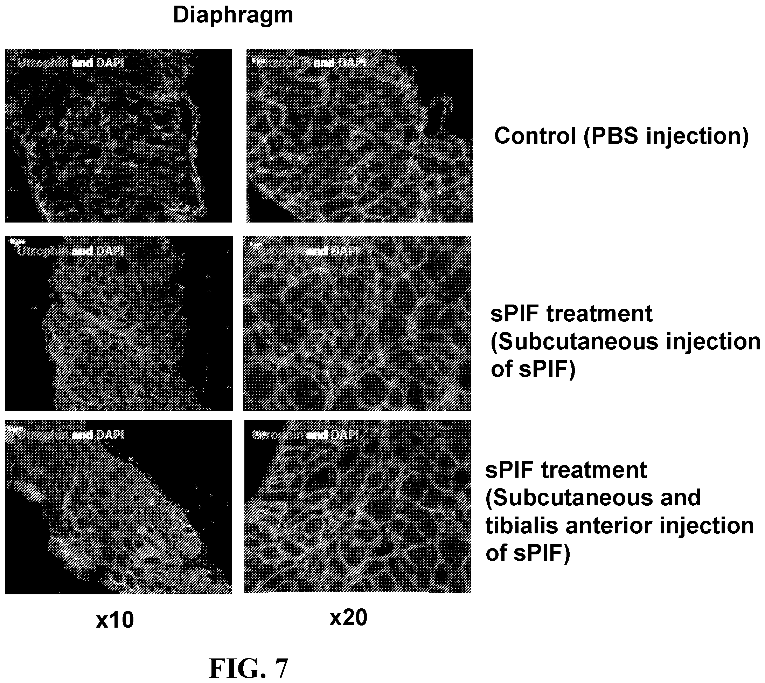

FIG. 7 is a series of representative pictures showing that sPIF treatment increased utrophin in the diaphram of mdx mice.

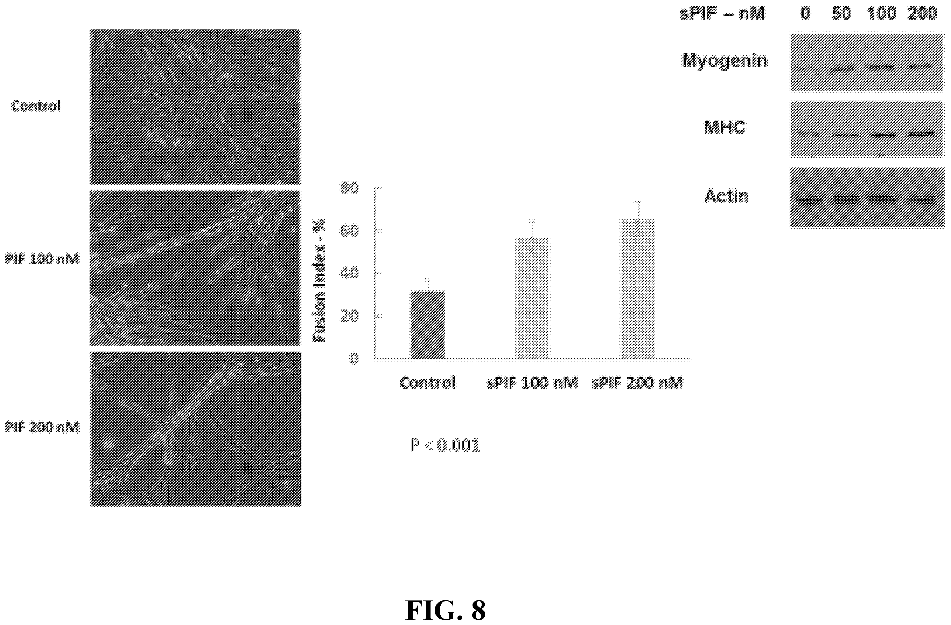

FIG. 8 is a series showing representative microphotographs of mouse myoblasts (left) and a graph of the fusion index percentage (middle), indicating that the effect of PIF on differentiation and fusion is dose dependent. The increase in myogenin levels shown in the Western blot (right) indicate muscle differentiation.

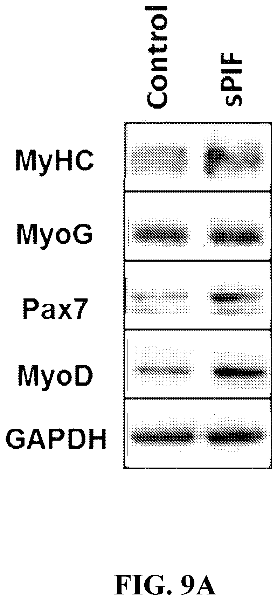

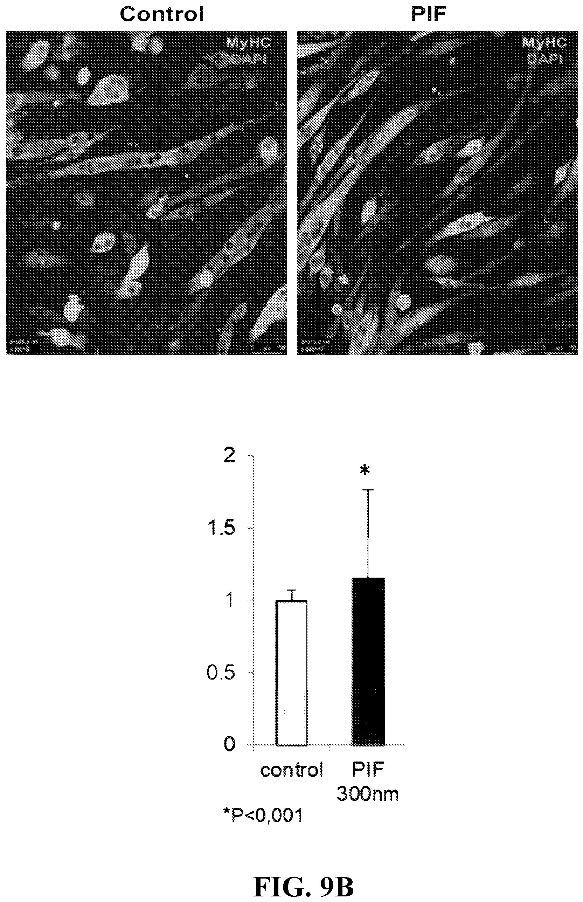

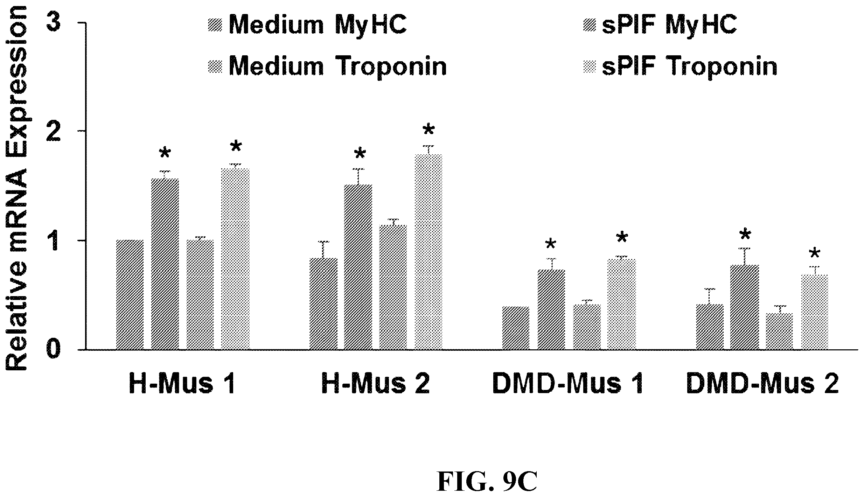

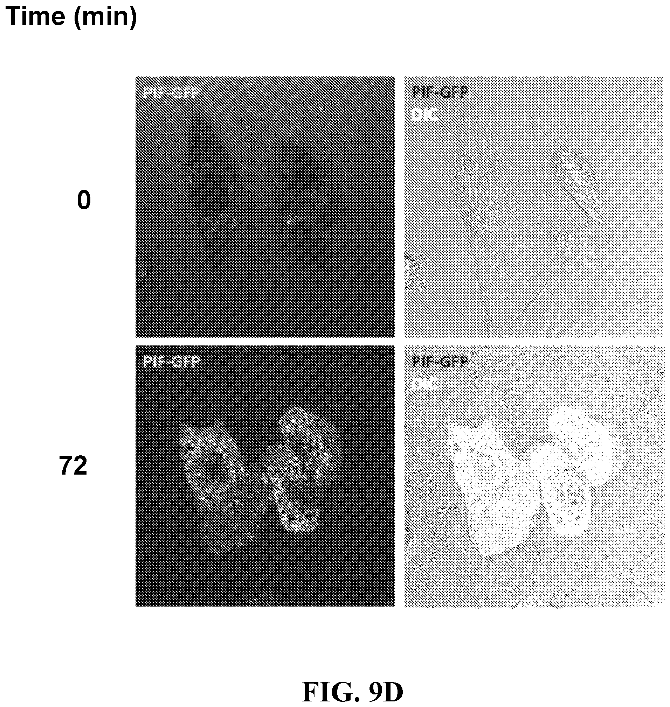

FIG. 9A-FIG. 9D show that sPIF increases the differentiation of mouse and human myoblasts. C2C12 cells were cultured in 2 % HS and treated with sPIF (300 ng/ml) for 5 days. The expression of the different myogenic proteins was determined using Western blot analysis (FIG. 9A). C2C12 cells were cultured in 2% HS and after 5 days stained with DAPI and anti-myosin heavy chain (MyHC) antibody (FITC). Cell fusion was analyzed using confocal microscopy (FIG. 9B). Human myoblasts derived from two DMD patients (DMDmus) and two healthy donors (H-mus) were treated with sPIF 300 ng/ml and analyzed for the expression of MyHC and troponin following 5 days in fusion medium using RT-PCR (FIG. 9C). The internalization of sPIF-FITC to C2C12 cells was analyzed in live cell imaging using confocal microscopy (FIG. 9D). ^{∗}P<0.001.

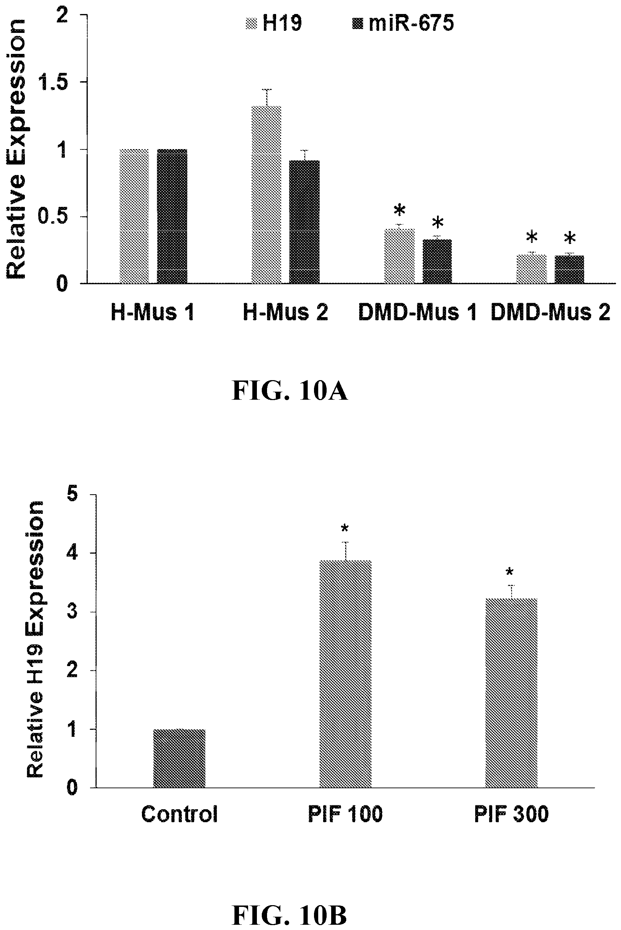

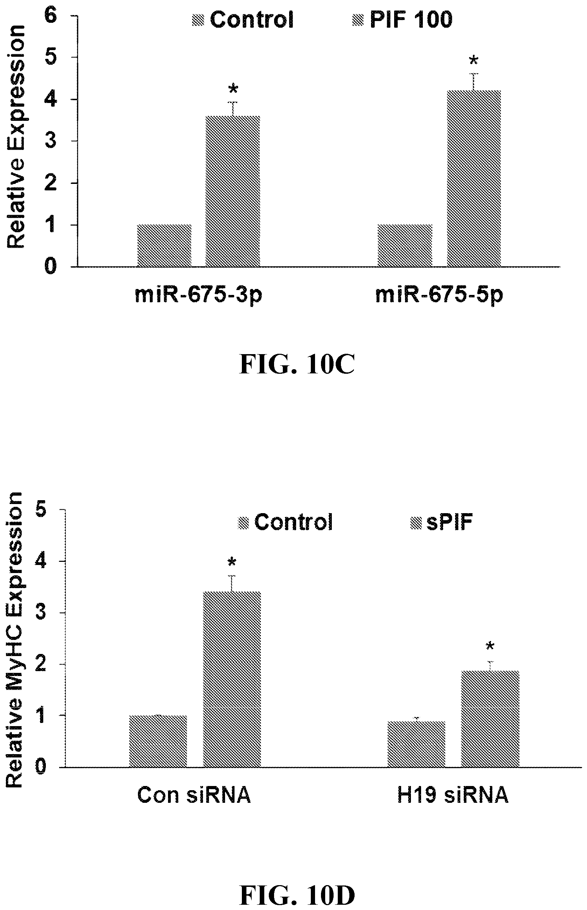

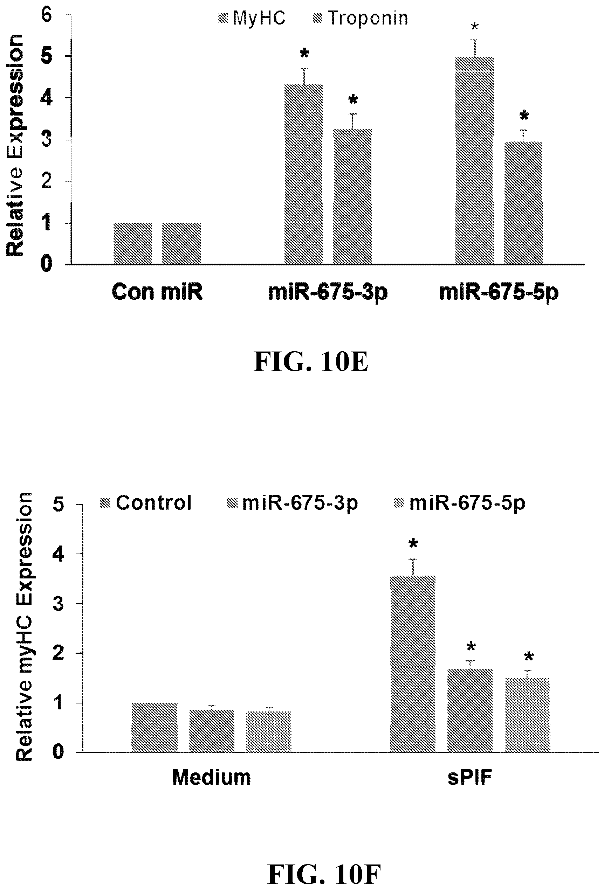

FIG. 10A-FIG. 10F show that sPIF increases the differentiation of DMDmyoblasts via the induction of the H19/miR-675 pathway. The expression of H19 and miR-675 was analyzed in cultured muscle cells derived from healthy donors and DMD patients using RT-PCR (FIG. 10A). sPIF induced the upregulation of both H19 (FIG. 10B) and miR-675-3p and 5p expression (FIG. 10C) as determined by RT-PCR. DMD myoblasts were transfected with a control or H19 siRNA and then treated with sPIF for 7 days. The expression of MyHC was determined by RT-PCR (FIG. 10D). DMD myoblasts were transfected with miR-675-3p or miR-675-5p mimics and the expression of MyHC was determined 7 days later (FIG. 10E). The effects of sPIF on MyHC expression were determined in DMD myoblasts transfected with a control or miR-675 antagomiRs (FIG. 10F). ^{∗}P<0.001.

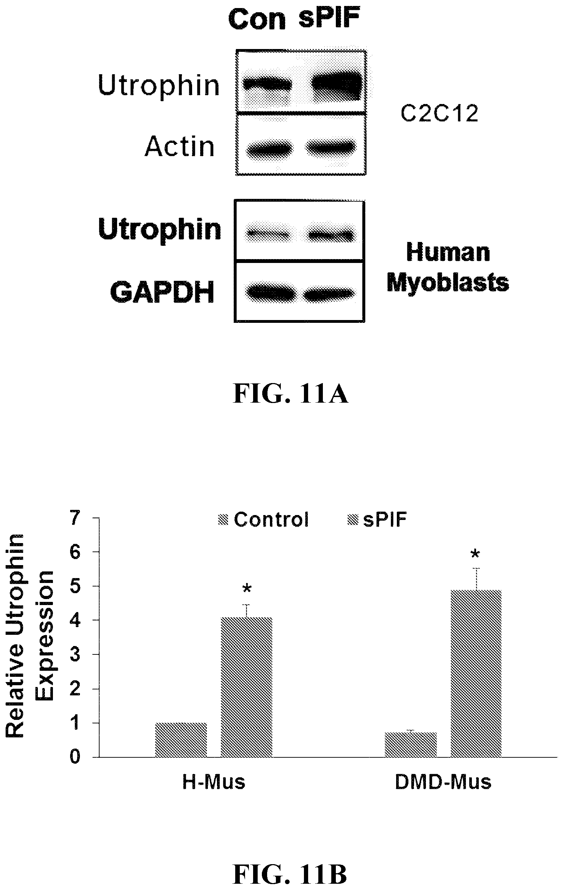

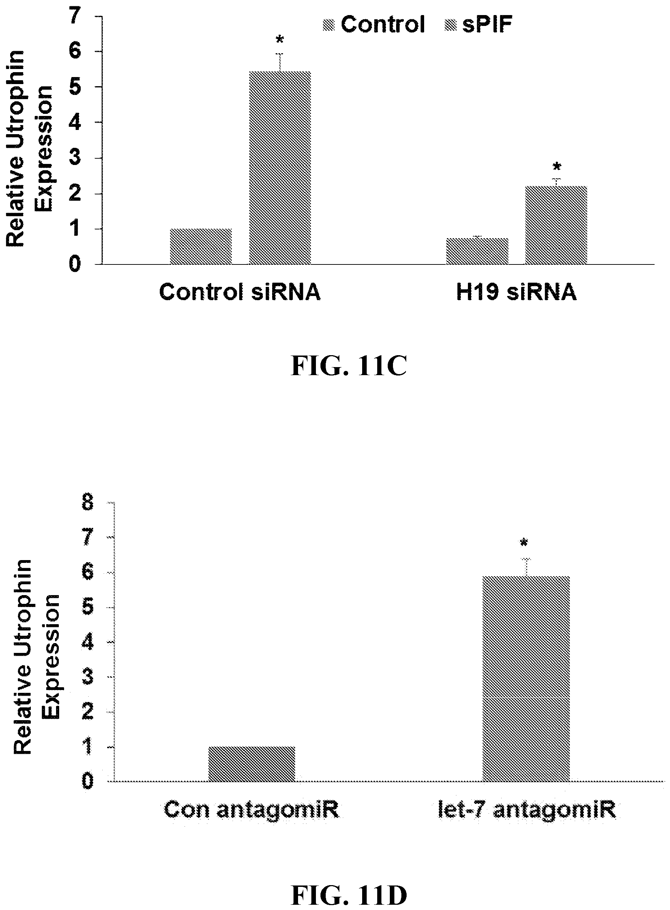

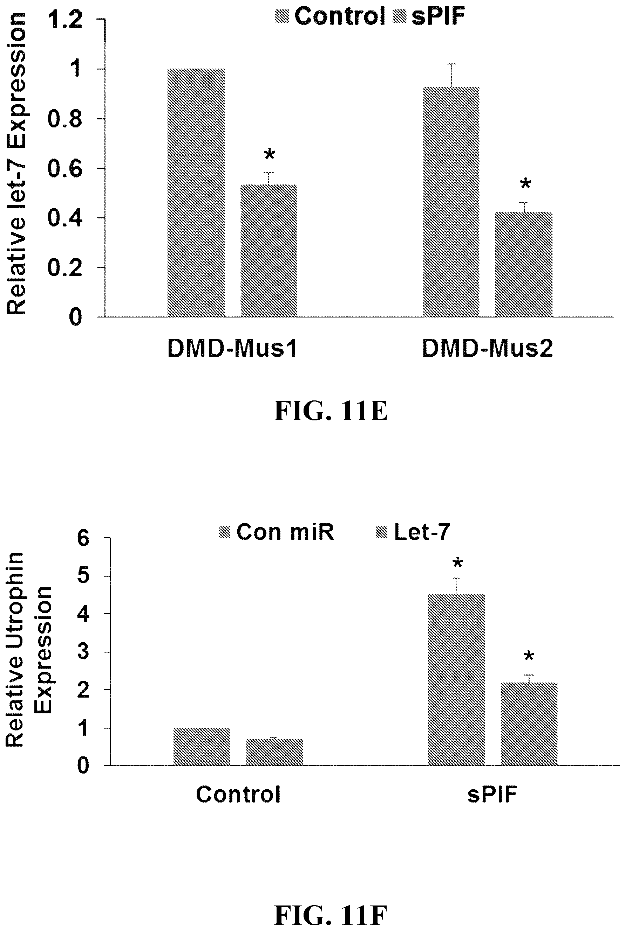

FIG. 11A-FIG. 11F show that sPIF upregulates utrophin expression via the H19/Let-7 pathway. C2C12 and DMD myoblasts were treated with sPIF for 7 days and utrophin expression was determined by Western blot analysis (FIG. 11A). Utrophin expression was also in DMD myoblasts by sPIF treatment as determined by RT-PCR (FIG. 11B). DMD myoblasts were silenced for H19 and sPIF effects were then determined on utrophin expression following 5 days of treatment (FIG. 11C). DMD myoblasts were treated with a control or let-7 antagomiR and utrophin expression was analyzed three days later using RT-PCR (FIG. 11D). The expression of let-7 was determined in DMD myoblasts following sPIF treatment for 3 days by RT-PCR (FIG. 11E). DMD myoblasts were transfected with a control or let-7 mimic and then treated with sPIF for 5 days. Utrophin expression was determined by RT-PCR (FIG. 11F). ^{∗}P<0.001.

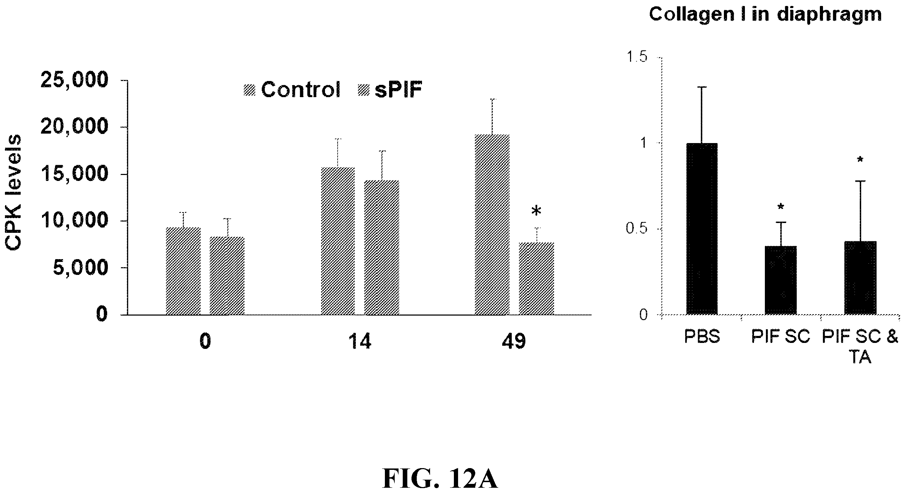

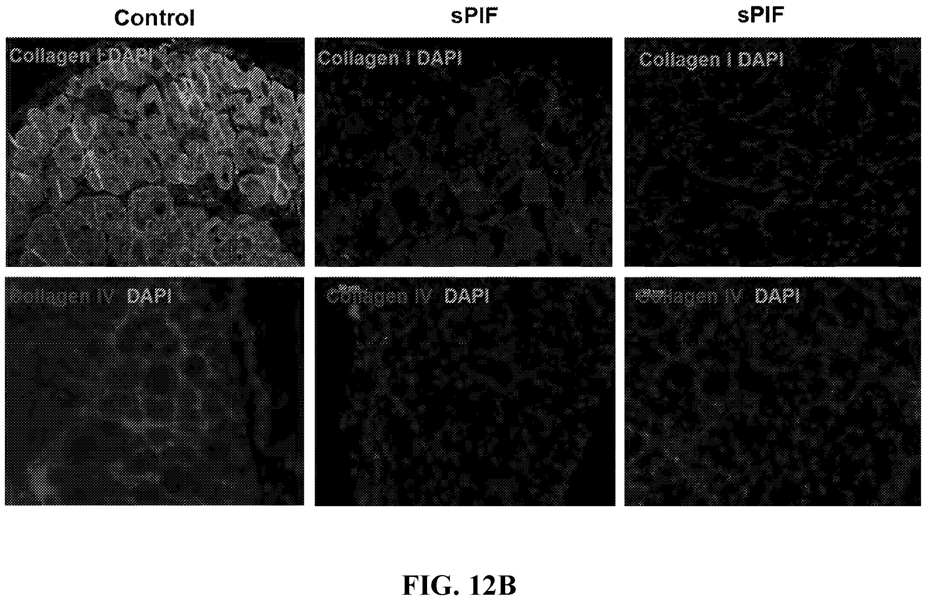

FIG. 12A-FIG. 12B show that sPIF decreased CK levels and tissue fibrosis in mdx mice. Mice were treated with sPIF (0.75mg/kg) and CK levels were analyzed following an additional two weeks (FIG. 12A). Diaphragm muscles from the treated mice were removed and stained for anti-collagen I and collagen IV (FIG. 12B).

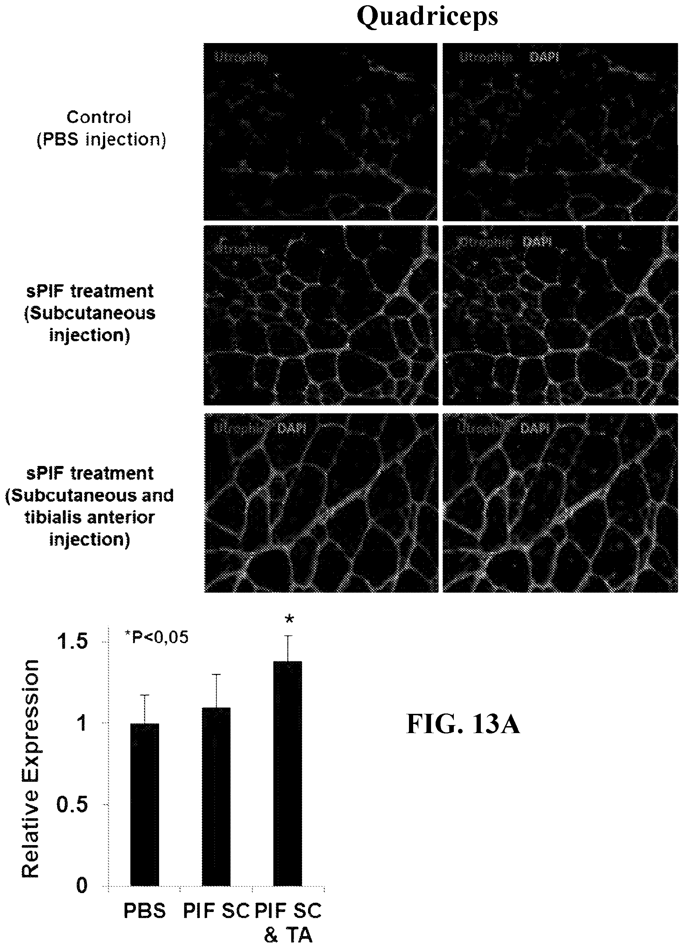

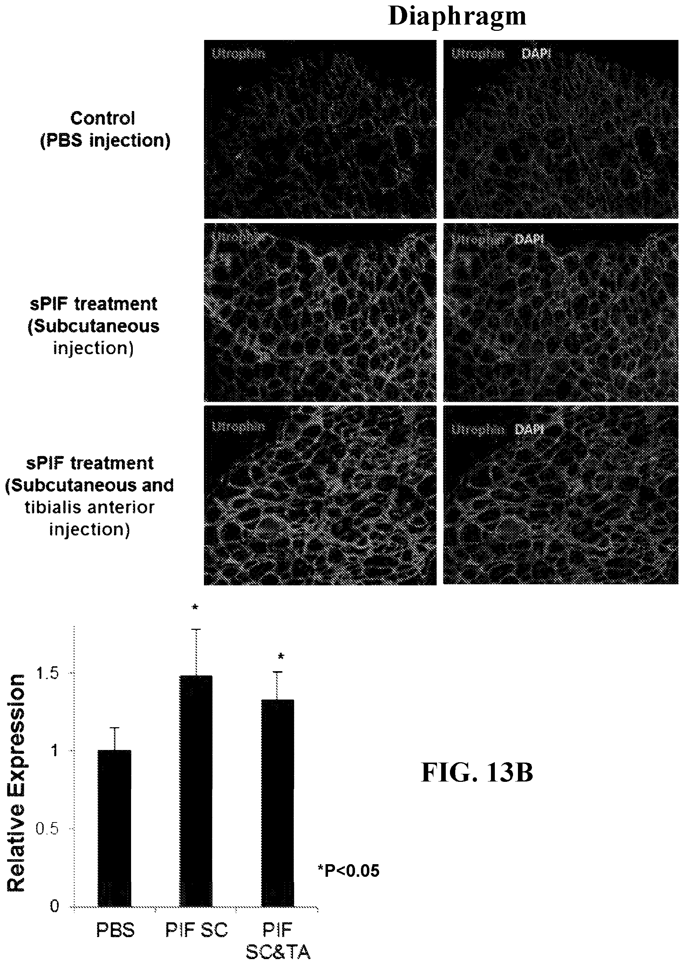

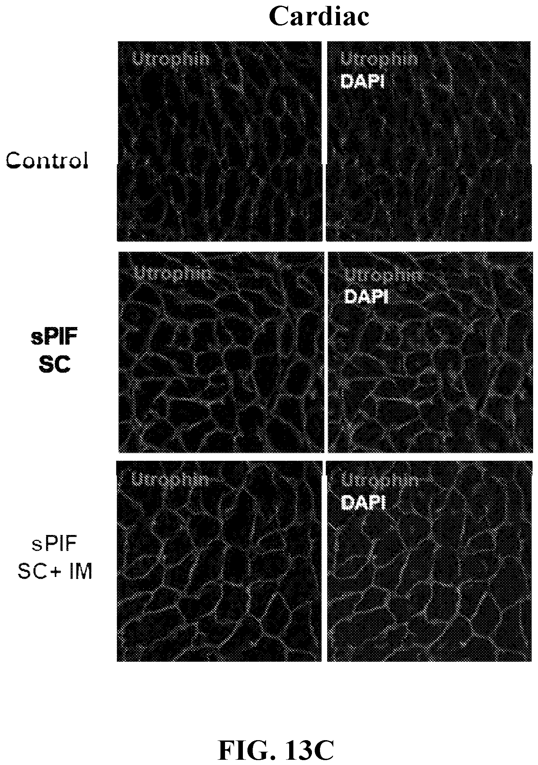

FIG. 13A-FIG. 13C show that sPIF increases the expression of utrophin in MDX mice. mdx mice (6 weeks; n = 8 per group) were injected with sPIF (0.75 mg/kg twice daily for 2 weeks) either S.C. or once S.C and once to the TA muscle. Following 2 additional weeks, the QC muscle (FIG. 13A), diaphragm (FIG. 13B) and cardiac (FIG. 13C) tissues were immunostained with an anti-utrophin antibody and the sections visualized by confocal microscopy. The levels of utrophin were analyzed for fluorescence signal intensity using Cell Profiler software tool and presented as a graph (^{∗}P<0.001)

### DETAILED DESCRIPTION OF THE DISCLOSURE

Before the present compositions and methods are described, it is to be understood that this disclosure is not limited to the particular molecules, compositions, methodologies or protocols described, as these may vary. It is also to be understood that the terminology used in the description is for the purpose of describing the particular versions or embodiments only, and is not intended to limit the scope of the present disclosure which will be limited only by the appended claims. It is understood that these embodiments are not limited to the particular methodology, protocols, cell lines, vectors, and reagents described, as these may vary. It also is to be understood that the terminology used herein is for the purpose of describing particular embodiments only, and is not intended to limit the scope of the present embodiments or claims. The compositions described herein may include D amino acids, L amino acids, a racemic backbone of D and L amino acids, or any mixture thereof at each residue. That is, at each position, the residue may be a D amino acid residue or a L-amino acid residue and each position can be independently D or L of each other position, unless context dictates otherwise.

Unless defined otherwise, all technical and scientific terms used herein have the same meanings as commonly understood by one of ordinary skill in the art. Although any methods and materials similar or equivalent to those described herein can be used in the practice or testing of embodiments of the present disclosure, the preferred methods, devices, and materials are now described. All publications mentioned herein are incorporated by reference. Nothing herein is to be construed as an admission that the disclosure is not entitled to antedate such disclosure by virtue of prior disclosure.

As used herein, the phrase "in need thereof means that the animal or mammal has been identified or suspected as having a need for the particular method or treatment. In some embodiments, the identification can be by any means of diagnosis or observation. In any of the methods and treatments described herein, the animal or mammal can be in need thereof. In some embodiments, the animal or mammal is in an environment or will be traveling to an environment in which a particular disorder or condition is prevalent or more likely to occur.

As used herein, the term "subject," "individual" or "patient," used interchangeably, means any animal, including mammals, such as mice, rats, other rodents, rabbits, dogs, cats, swine, cattle, sheep, horses, or primates, such as humans. As used herein, the term "animal" includes, but is not limited to, humans and non-human vertebrates such as wild animals, rodents, such as rats, ferrets, and domesticated animals, and farm animals, such as horses, pigs, cows, sheep, goats. In some embodiments, the animal is a mammal. In some embodiments, the animal is a human. In some embodiments, the animal is a non-human mammal.

As used herein, the terms "a" or "an" means that "at least one" or "one or more" unless the context clearly indicates otherwise. It must also be noted that as used herein and in the appended claims, the singular forms "a", "an", and "the" include plural reference unless the context clearly dictates otherwise. Thus, for example, reference to "a cell" is a reference to one or more cells and equivalents thereof known to those skilled in the art, and so forth.

As used herein, the term "about" means that the numerical value is approximate and small variations would not significantly affect the practice of the disclosed embodiments. Where a numerical limitation is used, unless indicated otherwise by the context, "about" means the numerical value can vary by 10% and remain within the scope of the disclosed embodiments. Where a numerical value is used with the term "about" the numerical value without the term "about" is also disclosed and can be used without the term "about."

As used herein, the terms "comprising" (and any form of comprising, such as "comprise", "comprises", and "comprised"), "having" (and any form of having, such as "have" and "has"), "including" (and any form of including, such as "includes" and "include"), or "containing" (and any form of containing, such as "contains" and "contain"), are inclusive or open-ended and do not exclude additional, unrecited elements or method steps.

As used herein, the phrase "integer from X to Y" means any integer that includes the endpoints. That is, where a range is disclosed, each integer in the range including the endpoints is disclosed. For example, the phrase "integer from X to Y" discloses about 1, 2, 3, 4, or 5 as well as the range from about 1 to about 5.

As used herein, the term "mammal" means any animal in the class Mammalia such as rodent (i.e., a mouse, a rat, or a guinea pig), a monkey, a cat, a dog, a cow, a horse, a pig, or a human. In some embodiments, the mammal is a human.

As used herein, the phrase "therapeutically effective amount" means the amount of active compound or pharmaceutical agent that elicits the biological or medicinal response that is being sought in a tissue, system, animal, individual or human by a researcher, veterinarian, medical doctor or other clinician. The therapeutic effect is dependent upon the disorder being treated or the biological effect desired. As such, the therapeutic effect can be a decrease in the severity of symptoms associated with the disorder and/or inhibition (partial or complete) of progression of the disorder, or improved treatment, healing, prevention or elimination of a disorder, or side-effects. The amount needed to elicit the therapeutic response can be determined based on the age, health, size and sex of the subject. Optimal amounts can also be determined based on monitoring of the subject's response to treatment.

As used herein, the terms "treat," "treated," or "treating" can refer to therapeutic treatment and/or prophylactic or preventative measures wherein the object is to prevent or slow down (lessen) an undesired physiological condition, disorder or disease, or obtain beneficial or desired clinical results. For purposes of the embodiments described herein, beneficial or desired clinical results include, but are not limited to, alleviation of symptoms; diminishment of extent of condition, disorder or disease; stabilized (i.e., not worsening) state of condition, disorder or disease; delay in onset or slowing of condition, disorder or disease progression; amelioration of the condition, disorder or disease state or remission (whether partial or total), whether detectable or undetectable; an amelioration of at least one measurable physical parameter, not necessarily discernible by the patient; or enhancement or improvement of condition, disorder or disease. Treatment can also include eliciting a clinically significant response without excessive levels of side effects. Treatment also includes prolonging survival as compared to expected survival if not receiving treatment. Thus, "treatment of Duchenne's Muscular Dystrophy " means an activity that prevents, alleviates or ameliorates any of the primary phenomena or secondary symptoms associated with a dystrophy-related disorder, including Duchenne's Muscular Dystrophy.

This application describes compounds and methods of administering those compounds to a subject in need thereof. In some embodiments, "preimplantation factor" or "PIF" may also refer to synthetic PIF-1, which replicates the native peptide's effect and exerts potent immune modulatory effects on activated peripheral blood mononuclear cell (PBMC) proliferation and cytokine secretion, acting through novel sites on PBMCs and having an effect which is distinct from known immunosuppressive drugs. In some embodiments, "preimplantation factor" or "PIF" or "PIF analog" refers to an amino acid selected from SEQ ID NO:1, SEQ ID NO:2, SEQ ID NO:3, SEQ ID NO:4, SEQ ID NO:5, SEQ ID NO:13, SEQ ID NO:14, SEQ ID NO:15, SEQ ID NO:16, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO: 31, SEQ ID NO:32, or peptidomimetics or mutants thereof, and combinations thereof that are about 75, 80, 81, 82, 83, 84 85, 86, 87, 88, 89, 90, 91, 92, 93, 94, 95, 96, 97, 98, or 99% homologous to any such amino acid. In some embodiments, the terms "PIF" "PIF peptide," or "PIF analog" refers to an amino acid sequence of Table 1 or any peptidomimetics, mutant or analog thereof, that is from about 75% to about 100% homologous to any sequence of Table 1, optionally fused or unfused to one or more other amino acid sequences at its carboxy and/or its amino terminal ends. Pharmaceutical compositions of the present disclosure relate to any or all of the compounds or PIF peptides disclosed herein or their respective pharmaceutically effective salts or polymorphs.

Without being bound by any particular theory, the compounds described herein may act as agonists of PIF-mediated signal transduction via the receptor or receptors of PIF. Thus, these compounds modulate signaling pathways that provide significant therapeutic benefit in the treatment of, but not limited to, Duchenne's Muscular Dystrophy. The compounds of the present disclosure may exist in unsolvated forms as well as solvated forms, including hydrated forms. The compounds of the present disclosure also are capable of forming both pharmaceutically acceptable salts, including but not limited to acid addition and/or base addition salts. Furthermore, compounds of the present disclosure may exist in various solid states including an amorphous form (non-crystalline form), and in the form of clathrates, prodrugs, polymorphs, bio-hydrolyzable esters, racemic mixtures, non-racemic mixtures, or as purified stereoisomers including, but not limited to, optically pure enantiomers and diastereomers. In general, all of these forms can be used as an alternative form to the free base or free acid forms of the compounds, as described above and are intended to be encompassed within the scope of the present disclosure.

A "polymorph" refers to solid crystalline forms of a compound. Different polymorphs of the same compound can exhibit different physical, chemical and/or spectroscopic properties. Different physical properties include, but are not limited to stability (e.g., to heat or light), compressibility and density (important in formulation and product manufacturing), and dissolution rates (which can affect bioavailability). Different physical properties of polymorphs can affect their processing. The disclosure relates to compositions comprising a polymorph of any of the disclosed PIF peptides or salts thereof.

As noted above, the compounds of the present disclosure can be administered, *inter alia,* as pharmaceutically acceptable salts, esters, amides or prodrugs. The term "salts" refers to inorganic and organic salts of compounds of the present disclosure. The salts can be prepared in situ during the final isolation and purification of a compound, or by separately reacting a purified compound in its free base or acid form with a suitable organic or inorganic base or acid and isolating the salt thus formed. Representative salts include the hydrobromide, hydrochloride, sulfate, bisulfate, nitrate, acetate, oxalate, palmitiate, stearate, laurate, borate, benzoate, lactate, phosphate, tosylate, citrate, maleate, fumarate, succinate, tartrate, naphthylate, mesylate, glucoheptonate, lactobionate, and laurylsulphonate salts, and the like. The salts may include cations based on the alkali and alkaline earth metals, such as sodium, lithium, potassium, calcium, magnesium, and the like, as well as non-toxic ammonium, quaternary ammonium, and amine cations including, but not limited to, ammonium, tetramethylammonium, tetraethylammonium, methylamine, dimethylamine, trimethylamine, triethylamine, ethylamine, and the like. See, for example, S. M. Berge, et al., "Pharmaceutical Salts," J Pharm Sci, 66: 1-19 (1977). The term "salt" refers to acidic salts formed with inorganic and/or organic acids, as well as basic salts formed with inorganic and/or organic bases. Examples of these acids and bases are well known to those of ordinary skill in the art. Such acid addition salts will normally be pharmaceutically acceptable although salts of non-pharmaceutically acceptable acids may be of utility in the preparation and purification of the compound in question. Salts include those formed from hydrochloric, hydrobromic, sulphuric, phosphoric, citric, tartaric, lactic, pyruvic, acetic, succinic, fumaric, maleic, methanesulphonic and benzenesulphonic acids.

In some embodiments, salts of the compositions comprising either a PIF or PIF analog or PIF mutant may be formed by reacting the free base, or a salt, enantiomer or racemate thereof, with one or more equivalents of the appropriate acid. In some embodiments, pharmaceutical acceptable salts of the present disclosure refer to analogs having at least one basic group or at least one basic radical. In some embodiments, pharmaceutical acceptable salts of the present disclosure comprise a free amino group, a free guanidino group, a pyrazinyl radical, or a pyridyl radical that forms acid addition salts. In some embodiments, the pharmaceutical acceptable salts of the present disclosure refer to analogs that are acid addition salts of the subject compounds with (for example) inorganic acids, such as hydrochloric acid, sulfuric acid or a phosphoric acid, or with suitable organic carboxylic or sulfonic acids, for example aliphatic mono- or di-carboxylic acids, such as trifluoroacetic acid, acetic acid, propionic acid, glycolic acid, succinic acid, maleic acid, fumaric acid, hydroxymaleic acid, malic acid, tartaric acid, citric acid or oxalic acid, or amino acids such as arginine or lysine, aromatic carboxylic acids, such as benzoic acid, 2-phenoxybenzoic acid, 2-acetoxybenzoic acid, salicylic acid, 4-aminosalicylic acid, aromatic-aliphatic carboxylic acids, such as mandelic acid or cinnamic acid, heteroaromatic carboxylic acids, such as nicotinic acid or isonicotinic acid, aliphatic sulfonic acids, such as methane-, ethane-or 2-hydroxyethane-sulfonic acid, or aromatic sulfonic acids, for example benzene-, p-toluene- or naphthalene-2-sulfonic acid. When several basic groups are present mono- or poly-acid addition salts may be formed. The reaction may be carried out in a solvent or medium in which the salt is insoluble or in a solvent in which the salt is soluble, for example, water, dioxane, ethanol, tetrahydrofuran or diethyl ether, or a mixture of solvents, which may be removed in vacuo or by freeze drying. The reaction may also be a metathetical process or it may be carried out on an ion exchange resin. In some embodiments, the salts may be those that are physiologically tolerated by a patient. Salts according to the present disclosure may be found in their anhydrous form or as in hydrated crystalline form (i.e., complexed or crystallized with one or more molecules of water).

Examples of pharmaceutically acceptable esters of the compounds of the present disclosure include C₁-C₈ alkyl esters. Acceptable esters also include C₅-C₇ cycloalkyl esters, as well as arylalkyl esters such as benzyl. C₁-C₄ alkyl esters are commonly used. Esters of compounds of the present disclosure may be prepared according to methods that are well known in the art. Examples of pharmaceutically acceptable amides of the compounds of the present disclosure include amides derived from ammonia, primary C₁-C₈ alkyl amines, and secondary C₁-C₈ dialkyl amines. In the case of secondary amines, the amine may also be in the form of a 5 or 6 membered heterocycloalkyl group containing at least one nitrogen atom. Amides derived from ammonia, C₁-C₃ primary alkyl amines and C₁-C₂ dialkyl secondary amines are commonly used. Amides of the compounds of the present disclosure may be prepared according to methods well known to those skilled in the art.

"Administering" when used in conjunction with a therapeutic means to administer a therapeutic directly into or onto a target tissue or to administer a therapeutic to a patient whereby the therapeutic positively impacts the tissue to which it is targeted. Thus, as used herein, the term "administering", when used in conjunction with PIF, can include, but is not limited to, providing PIF peptide into or onto the target tissue; providing PIF peptide systemically to a patient by, e.g., intravenous injection whereby the therapeutic reaches the target; providing PIF peptide in the form of the encoding sequence thereof to the target (e.g., by so-called gene-therapy techniques). "Administering" a composition may be accomplished by parenteral, oral or topical administration.

As used herein, the term "therapeutic" means an agent utilized to treat, combat, ameliorate, prevent or improve an unwanted condition, symptom or disease of a subject. In part, embodiments of the present disclosure are directed to treating, ameloriating, preventing or improving autoimmune disease.

A "therapeutically effective amount" or "effective amount" or "physiologically relevant amount" of a composition is an amount calculated to achieve a desired effect, *i.e.,* to effectively inhibit or reduce symptoms and/or complications associated with a dystrophin-related disorder, such as Duchenne's Muscular Dystrophy. Effective amounts of compounds of the present disclosure can objectively or subjectively reduce or decrease the severity or frequency of symptoms associated with Duchenne's Muscular Dystrophy. The specific dose of a compound administered according to this disclosure to obtain therapeutic and/or prophylactic effects will, of course, be determined by the particular circumstances surrounding the case, including, for example, the compound administered, the route of administration, and the condition being treated. The compounds are effective over a wide dosage range and, for example, dosages per day will normally fall within the range of from about 0.01 mg/kg to about 100 mg/kg, more preferably about 0.1 mg/kg to about 1 mg/kg. In some embodiments, the therapeutically effective dose of PIF or PIF analog or peptide is about 0.1mg/kg, 0.2mg/kg, 0.3mg/kg, 0.4mg/kg, 0.5mg/kg, 0.6mg/kg, 0.7mg/kg, 0.8mg/kg, 0.9mg/kg, and 1mg/kg, 5 mg/kg, 6 mg/kg, 7 mg/kg 8 mg/kg, 9 mg/kg, 10 mg/kg, 12 mg/kg, where "mg" is milligram of PIF analog or peptide "kg" is kilogram of the subject.

In some embodiments, the pharmaceutical compositions comprise a therapeutically effective amount of PIF peptide or analog but the composition is free of SEQ ID NO: 1 or a pharmaceutically acceptable salt thereof.

It will be understood that the effective amount administered will be determined by the physician in the light of the relevant circumstances including the condition to be treated, the choice of compound to be administered, and the chosen route of administration, and therefore the above dosage ranges are not intended to limit the scope of the disclosure in any way. A therapeutically effective amount of compound of this disclosure is typically an amount such that when it is administered in a physiologically tolerable excipient composition, it is sufficient to achieve an effective systemic concentration or local concentration in the tissue. In some embodiments, the term "therapeutically effective amount" as used herein, refers to that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated. In one aspect, the therapeutically effective amount is that which may treat or alleviate the disease or symptoms of the disease at a reasonable benefit/risk ratio applicable to any medical treatment. However, it is to be understood that the total daily usage of the compounds and compositions described herein may be decided by the attending physician within the scope of sound medical judgment. The specific therapeutically-effective dose level for any particular patient will depend upon a variety of factors, including the disorder being treated and the severity of the disorder; activity of the specific compound employed; the specific composition employed; the age, body weight, general health, gender and diet of the patient: the time of administration, route of administration, and rate of excretion of the specific compound employed; the duration of the treatment; drugs used in combination or coincidentally with the specific compound employed; and like factors well known to the researcher, veterinarian, medical doctor or other clinician of ordinary skill.

It is also appreciated that the therapeutically effective amount, whether referring to monotherapy or combination therapy, is advantageously selected with reference to any toxicity, or other undesirable side effect, that might occur during administration of one or more of the compounds described herein. Further, it is appreciated that the co-therapies described herein may allow for the administration of lower doses of compounds that show such toxicity, or other undesirable side effect, where those lower doses are below thresholds of toxicity or lower in the therapeutic window than would otherwise be administered in the absence of a co-therapy.

As used herein, the term "composition" generally refers to any product comprising the specified ingredients in the specified amounts, as well as any product which results, directly or indirectly, from combinations of the specified ingredients in the specified amounts. It is to be understood that the compositions described herein may be prepared from isolated compounds described herein or from salts, solutions, hydrates, solvates, and other forms of the compounds described herein. It is also to be understood that the compositions may be prepared from various amorphous, non-amorphous, partially crystalline, crystalline, and/or other morphological forms of the compounds described herein. It is also to be understood that the compositions may be prepared from various hydrates and/or solvates of the compounds described herein. Accordingly, such pharmaceutical compositions that recite compounds described herein are to be understood to include each of, or any combination of, the various morphological forms and/or solvate or hydrate forms of the compounds described herein.

Illustratively, compositions may include one or more carriers, diluents, and/or excipients. The compounds described herein, or compositions containing them, may be formulated in a therapeutically effective amount in any conventional dosage forms appropriate for the methods described herein. The compounds described herein, or compositions containing them, including such formulations, may be administered by a wide variety of conventional routes for the methods described herein, and in a wide variety of dosage formats, utilizing known procedures (see generally, Remington: The Science and Practice of Pharmacy, (21st ed., 2005)).

"Immune-modulating" refers to the ability of a compound of the present disclosure to alter (modulate) one or more aspects of the immune system. The immune system functions to protect the organism from infection and from foreign antigens by cellular and humoral mechanisms involving lymphocytes, macrophages, and other antigen-presenting cells that regulate each other by means of multiple cell-cell interactions and by elaborating soluble factors, including lymphokines and antibodies, that have autocrine, paracrine, and endocrine effects on immune cells.

"Dystrophy-related disorder" or "dystrophin-related disorder" as used herein refers to various diseases that arise from an abnormal expression of the proteins or peptides responsible for degeneration of myotubules. This may be restricted to certain organs or involve a particular tissue in different portions of the subject. A large number of dystrophin-related disorders are recognized, including, but not limited to, those disorders identified in Table 2:

**TABLE 2. Types of Muscular Dystrophy (MD)**

| **Type** | **Subtypes** | **Sub-subtypes** |
|---|---|---|
| Myotonic | Type 1 | |
| | Type 2 | |
| Duchenne | | |
| Becker | | |
| Limb-girdle | Type 1 (dominantly inherited) | Bethlem myopathy |
| | | Desmin myopathy/LGMDIE |
| | | Myofibrillar myopathy |
| | | ZASP-related myopathy (a form of myofibrillar |
| | | myopathy) |
| | | LGMD1A |
| | | LGMD1B |
| | | LGMD1C |
| | | LGMD1D |
| | | LGMD1F |
| | | LGMD1G |
| | | LGMD1H |
| | Type 2 (recessive) | Calpainopathy/LGMD2A |
| | | Dysferlinopathy/LGMD2B |
| | | Sarcoglycanopathies/ LGMD2C, LGMD2D, |
| | | LGMD2E, LGMD2F |
| | | LGMD2G |
| | | LGMD2H |
| | | LGMD2I |
| | | LGMD2J |
| | | LGMD2K |
| | | LGMD2L |
| | | LGMD2M |
| | | LGMD2N |
| | | LGMD2O |
| | | LGMD2Q |
| Facioscapulohumeral | Adult-onset Infantile-onset | |
| Congenital | CMD with adducted thumbs, ophthalmoplegia, and intellectual disability | |
| | CMD with cardiomyopathy | |
| | CMD with central nervous system atrophy and absence of large myelinated | |
| | fibers in peripheral nervous system | |
| | CMD with cerebellar atrophy | |
| | CMD with desmin inclusions | |
| | CMD with integrin alpha 7 mutations | |
| | CMD with joint hyperlaxity | |
| | CMD with familial junctional epidermolysis bullosa | |
| | CMD with muscle hypertrophy; also called MDC1C | |
| | CMD with muscle hypertrophy and respiratory failure; also called MDC1B | |
| | CMD with muscle hypertrophy and severe intellectual disability; also called | |
| | MDC1D | |
| | CMD with myasthenic syndrome | |
| | CMD with (early) spinal rigidity | |
| | CMD with spinal rigidity and lamin A/C abnormality | |
| | CMD with spinal rigidity and selenoprotein deficiency | |
| | CMD with structural abnormalities of mitochondria | |
| | Fukuyama CMD; also called MDDGA4 | |
| | Merosin-deficient CMD; also called MDC1A | |
| | Merosin-positive CMD; this is an old term referring to a variety of CMD types | |
| | in which merosin is normal | |
| | Santavuori muscle-eye-brain disease | |
| | Ullrich CMD | |
| | Walker-Warburg syndrome: MDDGA type | |
| | Walker-Warburg syndrome: MDDGA1 type | |
| | Walker-Warburg syndrome: MDDGA2 type | |
| | Walker-Warburg syndrome: MDDGA7 type | |
| | Walker-Warburg syndrome: MDDGA8 type | |
| | Walker-Warburg syndrome: MDDGA10 type | |
| | Walker-Warburg syndrome: MDDGA11 type | |
| | Walker-Warburg syndrome: MDDGA12 type | |
| Oculopharyngeal | | |
| Distal | Distal myopathy with vocal cord and pharyngeal weakness | |
| | Finnish (tibial) distal myopathy | |
| | Gowers-Laing distal myopathy | |
| | Hereditary inclusion-body myositis type 1/HIBM1 | |
| | Miyoshi distal myopathy | |
| | Nonaka distal myopathy/HIBM2 | |
| | Welander's distal myopathy | |
| | ZASP-related myopathy | |
| Emery - Dreifuss | | |

In some embodiments, the dystrophin-related disorder is a muscular dystrophy. In some embodiments, the dystrophin-related disorder is Duchenne's Muscular Dystrophy (DMD). In some embodiments, the dystrophin-related disorder is congenital muscular dystrophy. In some embodiments, the dystrophin-related disorder is myotonic muscular dystrophy. In some embodiments, the dystrophin-related disorder is Becker muscular dystrophy. In some embodiments, the dystrophin-related disorder is limb-girdle muscular dystrophy. In some embodiments, the dystrophin-related disorder is facioscapulohumeral muscular dystrophy. In some embodiments, the dystrophin-related disorder is oculopharyngeal muscular dystrophy. In some embodiments, the dystrophin-related disorder is distal muscular dystrophy. In some embodiments, the dystrophin-related disorder is Emery-Dreifuss muscular dystrophy.

In some embodiments, the dystrophin-related disorder is muscular dystrophyinduced fibrosis. In some embodiments, the dystrophin-related disorder is radiation-induced fibrosis. In some embodiments, the dystrophin-related disorder is surgically-induced fibrosis.

"Inflammatory response" or "inflammation" is a general term for the local accumulation of fluid, plasma proteins, and white blood cells initiated by physical injury, infection, or a local immune response. Inflammation is an aspect of many diseases and disorders, including but not limited to diseases related to immune disorders, viral infection, arthritis, autoimmune diseases, collagen diseases, allergy, asthma, pollinosis, and atopy. Inflammation is characterized by rubor (redness), dolor (pain), calor (heat) and tumor (swelling), reflecting changes in local blood vessels leading to increased local blood flow which causes heat and redness, migration of leukocytes into surrounding tissues (extravasation), and the exit of fluid and proteins from the blood and their local accumulation in the inflamed tissue, which results in swelling and pain, as well as the accumulation of plasma proteins that aid in host defense. These changes are initiated by cytokines produced by activated macrophages. Inflammation is often accompanied by loss of function due to replacement of parenchymal tissue with damaged tissue (e.g., in damaged myocardium), reflexive disuse due to pain, and mechanical constraints on function, e.g., when a joint swells during acute inflammation, or when scar tissue bridging an inflamed joint contracts as it matures into a chronic inflammatory lesion.

"Anti-inflammatory" refers to the ability of a compound to prevent or reduce the inflammatory response, or to soothe inflammation by reducing the symptoms of inflammation such as redness, pain, heat, or swelling. Inflammatory responses can be triggered by injury, for example injury to skin, muscle, tendons, or nerves. Inflammatory responses can also be triggered as part of an immune response. Inflammatory responses can also be triggered by infection, where pathogen recognition and tissue damage can initiate an inflammatory response at the site of infection. Generally, infectious agents induce inflammatory responses by activating innate immunity. Inflammation combats infection by delivering additional effector molecules and cells to augment the killing of invading microorganisms by the frontline macrophages, by providing a physical barrier preventing the spread of infection, and by promoting repair of injured tissue. "Inflammatory disorder" is sometimes used to refer to chronic inflammation due to any cause.

As used herein, the term "neuromuscular junction" (or "myoneural junction") is a chemical synapse formed by the contact between a motor neuron and a muscle fiber. It is at the neuromuscular junction that a motor neuron is able to transmit a signal to the muscle fiber, causing muscle contraction.

As used herein, "conservative" amino acid substitutions may be defined as set out in Tables A, B, or C below. The PIF peptides of the disclosure comprise peptide or peptidimimetics or salts thereof comprising conservative substitutions (from either nucleic acid or amino acid sequences) have been introduced by modification of polynucleotides encoding polypeptides of the disclosure or by simple protein or peptide synthesis on solid substrates. Amino acids can be classified according to physical properties and contribution to secondary and tertiary protein structure. A conservative substitution is recognized in the art as a substitution of one amino acid for another amino acid that has similar properties. In some embodiments, the conservative substitution is recognized in the art as a substitution of one nucleic acid for another nucleic acid that has similar properties, or, when encoded, has similar binding affinities. Exemplary conservative substitutions are set out in Table A.

**Table A -- Conservative Substitutions I**

| Side Chain Characteristics | | Amino Acid |
|---|---|---|
| Aliphatic | | |
| | Non-polar | G A P I L V F |
| | Polar - uncharged | C S T M N Q |
| | Polar - charged | D E K R |
| Aromatic | | H F W Y |
| Other | | N Q D E |

Alternately, conservative amino acids can be grouped as described in Lehninger, (Biochemistry, Second Edition; Worth Publishers, Inc. NY, N.Y. (1975), pp. 71-77) as set forth in Table B.

**Table B -- Conservative Substitutions II**

| Side Chain Characteristic | | Amino Acid |
|---|---|---|
| Non-polar (hydrophobic) | | |
| | Aliphatic: | A L IV P . |
| | Aromatic: | F W Y |
| | Sulfur-containing: | M |
| | Borderline: | GY |
| Uncharged-polar | | |
| | Hydroxyl: | STY |
| | Amides: | N Q |
| | Sulfhydryl: | C |
| | Borderline: | GY |
| Positively Charged (Basic): | | K R H |
| Negatively Charged (Acidic): | | DE |

Alternately, exemplary conservative substitutions are set out in Table C.

**Table C -- Conservative Substitutions III**

| Original Residue | Exemplary Substitution |
|---|---|
| Ala (A) | Val Leu Ile Met |
| Arg (R) | Lys His |
| Asn (N) | Gln |
| Asp (D) | Glu |
| Cys (C) | Ser Thr |
| Gln (Q) | Asn |
| Glu (E) | Asp |
| Gly (G) | Ala Val Leu Pro |
| His (H) | Lys Arg |
| Ile (I) | Leu Val Met Ala Phe |
| Leu (L) | Ile Val Met Ala Phe |
| Lys (K) | Arg His |
| Met (M) | Leu Ile Val Ala |
| Phe (F) | Trp Tyr Ile |
| Pro (P) | Gly Ala Val Leu Ile |
| Ser (S) | Thr |
| Thr (T) | Ser |
| Trp (W) | Tyr Phe Ile |
| Tyr (Y) | Trp Phe Thr Ser |
| Val (V) | Ile Leu Met Ala |

It should be understood that the inhibitors described herein are intended to include nucleic acids and, where the inhibitors include polypeptide, polypeptides bearing one or more insertions, deletions, or substitutions, or any combination thereof, of amino acid residues as well as modifications other than insertions, deletions, or substitutions of amino acid residues.

As used herein, the terms "peptide," "polypeptide" and "protein" are used interchangeably and refer to two or more amino acids covalently linked by an amide bond or non-amide equivalent. The peptides of the disclosure can be of any length. For example, the peptides can have from about two to about 100 or more residues, such as, about 4 to about 15, about 12 to about 15, about 8 to about 18, about 18 to about 25, about 15 to about 50,about 50 to about 75, or about 75 to about 100 or more amino acids in length. Preferably, peptides are from about 4 to about 18 residues in length. The peptides of the disclosure also include 1- and d-isomers, and combinations of 1- and d-isomers. The peptides can include modifications typically associated with posttranslational processing of proteins, for example, cyclization (e.g., disulfide or amide bond), phosphorylation, glycosylation, carboxylation, ubiquitination, myristylation, or lipidation. In some embodiments, the compositions or pharmaceutical compositions of the disclosure relate to analogs of any PIF sequence set forth in Table 1 that share no less than about 70%, about 75%, about 79%, about 80%, about 85%, about 86%, about 87%, about 90%, about 93%, about 94% about 95%, about 96%, about 97%, about 98%, about 99% homology with any one or combination of PIF sequences set forth in Table 1. In some embodiments, PIF or PIF peptide may refer to an amino acid sequence selected from SEQ ID NOs: 1, 2, 3, 4, 5, 6, 7, 8, 9, 10, 11, 12, 13 ,14 ,15, 16 ,17, 18, 19, 20, 21, 22, 23, 24, 25, 26, 27, 28, 29 or a functional fragment thereof that is about 70%, 75%, 80%, 85%, 90%, 91%, 92%, 93%, 94%, 95%, 96%, 97%, 98%, or 99% homologous to any such amino acid sequence. In some embodiments, PIF may refer to an amino acid sequence comprising, consisting essentially of, or consisting of a sequence that is at least 70%, 75%, 80%, 85%, 86%, 87%, 90%, 91%, 92%, 93%, 94%, 95%, 96%, 97%, 98%, or 99% homologous to SEQ ID. NO: 20. In some embodiments, PIF may refer to an amino acid sequence comprising, consisting essentially of, or consisting of a sequence that is at least 70%, 75%, 80%, 85%, 86%, 87%, 90%, 91%, 92%, 93%, 94%, 95%, 96%, 97%, 98%, or 99% homologous to SEQ ID. NO: 21. In some embodiments, PIF may refer to an amino acid sequence comprising, consisting essentially of, or consisting of a sequence that is at least 70%, 75%, 80%, 85%, 86%, 87%, 90%, 91%, 92%, 93%, 94%, 95%, 96%, 97%, 98%, or 99% homologous to SEQ ID. NO: 22. In some embodiments, PIF may refer to an amino acid sequence comprising, consisting essentially of, or consisting of a sequence that is at least 70%, 75%, 80%, 85%, 86%, 87%, 90%, 91%, 92%, 93%, 94%, 95%, 96%, 97%, 98%, or 99% homologous to SEQ ID. NO: 23. In some embodiments, PIF may refer to an amino acid sequence comprising, consisting essentially of, or consisting of a sequence that is at least 70%, 75%, 80%, 85%, 86%, 87%, 90%, 91%, 92%, 93%, 94%, 95%, 96%, 97%, 98%, or 99% homologous to SEQ ID. NO: 24. In some embodiments, PIF may refer to an amino acid sequence comprising, consisting essentially of, or consisting of a sequence that is at least 70%, 75%, 80%, 85%, 86%, 87%, 90%, 91%, 92%, 93%, 94%, 95%, 96%, 97%, 98%, or 99% homologous to SEQ ID. NO: 25. In some embodiments, PIF may refer to an amino acid sequence comprising, consisting essentially of, or consisting of a sequence that is at least 70%, 75%, 80%, 85%, 86%, 87%, 90%, 91%, 92%, 93%, 94%, 95%, 96%, 97%, 98%, or 99% homologous to SEQ ID. NO: 26. In some embodiments, PIF may refer to an amino acid sequence comprising, consisting essentially of, or consisting of a sequence that is at least 70%, 75%, 80%, 85%, 86%, 87%, 90%, 91%, 92%, 93%, 94%, 95%, 96%, 97%, 98%, or 99% homologous to SEQ ID. NO: 27. In some embodiments, PIF may refer to an amino acid sequence comprising, consisting essentially of, or consisting of a sequence that is at least 70%, 75%, 80%, 85%, 86%, 87%, 90%, 91%, 92%, 93%, 94%, 95%, 96%, 97%, 98%, or 99% homologous to SEQ ID. NO: 28. In some embodiments, PIF may refer to an amino acid sequence comprising, consisting essentially of, or consisting of a sequence that is at least 70%, 75%, 80%, 85%, 86%, 87%, 90%, 91%, 92%, 93%, 94%, 95%, 96%, 97%, 98%, or 99% homologous to SEQ ID. NO: 29. In some embodiments, the PIF mutant comprises a sequence selected from: XVZIKPGSANKPSD, XVZIKPGSANKPS XVZIKPGSANKP XVZIKPGSANK XVZIKPGSAN, XVZIKPGSA, XVZIKPGS, XVZIKPG, XVZIKP, XVZIK, XVZI, XVZ wherein X is a non-natural amino acid or a naturally occurring amino acid. In some embodiments, the PIF mutant comprises a sequence selected from: XVZIKPGSANKPSD, XVZIKPGSANKPS XVZIKPGSANKP XVZIKPGSANK XVZIKPGSAN, XVZIKPGSA, XVZIKPGS, XVZIKPG, XVZIKP, XVZIK, XVZI, XVZ wherein X is a non-natural amino acid or a naturally occurring amino acid except that X is not methionine if Z is arginine, and Z is not arginine if X is methionine. In some embodiments, the PIF analog or mutant is synthetic or synthetically made.

Peptides disclosed herein further include compounds having amino acid structural and functional analogs, for example, peptidomimetics having synthetic or non-natural amino acids (such as a norleucine) or amino acid analogues or non-natural side chains, so long as the mimetic shares one or more functions or activities of compounds of the disclosure. The compounds of the disclosure therefore include "mimetic" and "peptidomimetic" forms. As used herein, a "non-natural side chain" is a modified or synthetic chain of atoms joined by covalent bond to the α-carbon atom, β-carbon atom, or γ-carbon atom which does not make up the backbone of the polypeptide chain of amino acids. The peptide analogs may comprise one or a combination of non-natural amino-acids chosen from: norvaline, tert-butylglycine, phenylglycine, He, 7-azatryptophan, 4-fluorophenylalanine, N-methyl-methionine, N-methyl-valine, N-methyl-alanine, sarcosine, N-methyl-tert-butylglycine, N-methyl-leucine, N-methyl-phenylglycine, N-methyl-isoleucine, N-methyl-tryptophan, N-methyl-7-azatryptophan, N-methyl-phenylalanine, N-methyl-4-fluorophenylalanine, N-methyl-threonine, N-methyl-tyrosine, N-methyl-valine, N-methyl-lysine, homocysteine, and Tyr; Xaa2 is absent, or an amino acid selected from the group consisting of Ala, D-Ala, N-methyl-alanine, Glu, N-methyl-glutamate, D-Glu, Gly, sarcosine, norleucine, Lys, D-Lys, Asn, D-Asn, D-Glu, Arg, D-Arg, Phe, D-Phe, N-methyl-phenylalanine, Gin, D-Gln, Asp, D-Asp, Ser, D-Ser, N-methyl-serine, Thr, D-Thr, N-methyl-threonine, D-Pro D-Leu, N-methyl-leucine, D-Ile, N-methyl-isoleucine, D-Val, N-methyl-valine, tert-butylglycine, D-tert-butylglycine, N-methyl-tert-butylglycine, Trp, D-Trp, N-methyl-tryptophan, D-Tyr, N-methyl-tyrosine, 1-aminocyclopropanecarboxylic acid, 1-aminocyclobutanecarboxylic acid, 1-aminocyclopentanecarboxylic acid, 1-aminocyclohexanecarboxylic acid, 4-aminotetrahydro-2H-pyran-4-carboxylic acid, aminoisobutyric acid, (5)-2-amino-3-(1H-tetrazol-5-yl)propanoic acid, Glu, Gly, N-methyl-glutamate, 2-amino pentanoic acid, 2-amino hexanoic acid, 2-amino heptanoic acid, 2-amino octanoic acid, 2-amino nonanoic acid, 2-amino decanoic acid, 2-amino undecanoic acid, 2-amino dodecanoic acid, octylglycine, tranexamic acid, aminovaleric acid, and 2-(2-aminoethoxy)acetic acid. The natural side chain, or R group, of an alanine is a methyl group. In some embodiments, the non-natural side chain of the composition is a methyl group in which one or more of the hydrogen atoms is replaced by a deuterium atom. Non-natural side chains are disclosed in the art in the following publications: WO/2013/172954, WO2013123267, WO/2014/071241, WO/2014/138429, WO/2013/050615, WO/2013/050616, WO/2012/166559, US Application No. 20150094457, Ma, Z., and Hartman, M.C. (2012). In Vitro Selection of Unnatural Cyclic Peptide Libraries via mRNA Display. In J.A. Douthwaite & R.H. Jackson (Eds.), Ribosome Display and Related Technologies: Methods and Protocols (pp. 367-390). Springer New York., all of which are incorporated by reference in their entireties.

The terms "mimetic," "peptide mimetic" and "peptidomimetic" are used interchangeably herein, and generally refer to a peptide, partial peptide or non-peptide molecule that mimics the tertiary binding structure or activity of a selected native peptide or protein functional domain (e.g., binding motif or active site). These peptide mimetics include recombinantly or chemically modified peptides, as well as non-peptide agents such as small molecule drug mimetics, as further described below. In some embodiments, PIF is a PIF mimetic or PIF peptidometic with one or a plurality of modifications disclosed herein. The term "analog" refers to any polypeptide comprising at least one α-amino acid and at least one non-native amino acid residue, wherein the polypeptide is structurally similar to a naturally occurring full-length PIF protein and shares the biochemical or biological activity of the naturally occurring full-length protein upon which the analog is based. In some embodiments, PIF is a analog with one or a plurality of modifications disclosed herein. In some embodiments, the compositions, pharmaceutical compositions and kits comprise a peptide or peptidomimeic sharing share no less than about 70%, about 75%, about 79%, about 80%, about 85%, about 86%, about 87%, about 90%, about 93%, about 94% about 95%, about 96%, about 97%, about 98%, about 99% homology with any one or combination of PIF sequences set forth in Table 1; and wherein one or a plurality of amino acid residues is a non-natural amino acid residue or an amino acid residue with a non-natural sidechain. In some embodiments, peptide or peptide mimetics are provided, wherein a loop is formed between two cysteine residues. In some embodiments, the peptidomimetic may have many similarities to natural peptides, such as: amino acid side chains that are not found among the known 20 proteinogenic amino acids, non-peptide-based linkers used to effect cyclization between the ends or internal portions of the molecule, substitutions of the amide bond hydrogen moiety by methyl groups (N-methylation) or other alkyl groups, replacement of a peptide bond with a chemical group or bond that is resistant to chemical or enzymatic treatments, N- and C-terminal modifications, and conjugation with a non-peptidic extension (such as polyethylene glycol, lipids, carbohydrates, nucleosides, nucleotides, nucleoside bases, various small molecules, or phosphate or sulfate groups). As used herein, the term "cyclic peptide mimetic" or "cyclic polypeptide mimetic" refers to a peptide mimetic that has as part of its structure one or more cyclic features such as a loop, bridging moiety, and/or an internal linkage. As used herein, the term "bridging moiety" refers to one or a series of bonded atoms that covalently link one or a plurality of amino acid side chains to one another within an amino acid sequence.

In some embodiments, peptide or peptide mimetics are provided, wherein the loop comprises a bridging moiety selected from the group consisting of: 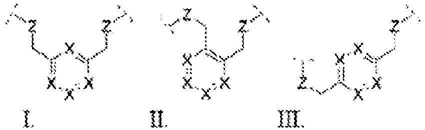 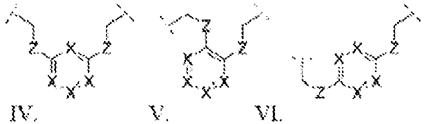 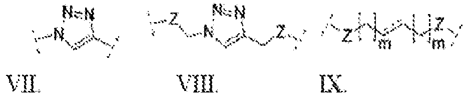 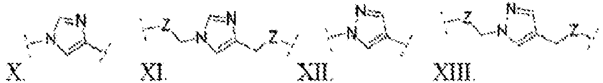 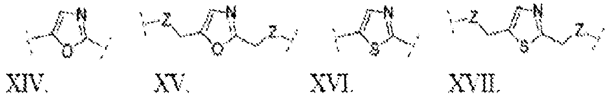 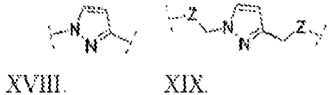 wherein each X is independently N or CH, such that no ring contains more than 2 N; each Z is independently a bond, NR, 0, S, CH2, C(O)NR, NRC(O), S(O)vNR, NRS(O)v; each m is independently selected from 0, 1, 2, and 3; each vis independently selected from 1 and 2; each R is independently selected from Hand C₁-C₆; and each bridging moiety is connected to the peptide by independently selected C₀-C₆ spacers.

In some embodiments, the PIF peptides of the disclosure are modified to produce peptide mimetics by replacement of one or more naturally occurring side chains of the 20 genetically encoded amino acids (or D amino acids) with other side chains, for instance with groups such as alkyl, lower alkyl, cyclic 4-, 5-, 6-, to 7 membered alkyl, amide, amide lower alkyl, amide di (lower alkyl), lower alkoxy, hydroxy, carboxy and the lower ester derivatives thereof, and with 4-, 5-, 6-, to 7 membered heterocyclics. For example, proline analogs can be made in which the ring size of the proline residue is changed from 5 members to 4, 6, or 7 members. Cyclic groups can be saturated or unsaturated, and if unsaturated, can be aromatic or nonaromatic. Heterocyclic groups can contain one or more nitrogen, oxygen, and/or sulphur heteroatoms. Examples of such groups include the furazanyl,furyl, imidazolidinyl, imidazolyl, imidazolinyl, isothiazolyl, isoxazolyl, morpholinyl (*e.g*. morpholino ), oxazolyl, piperazinyl (*e.g.* 1-piperazinyl), piperidyl (*e.g.* 1-piperidyl, piperidino ), pyranyl, pyrazinyl, pyrazolidinyl, pyrazolinyl, pyrazolyl, pyridazinyl, pyridyl, pyrimidinyl, pyrrolidinyl (*e.g*. 1-pyrrolidinyl), pyrrolinyl, pyrrolyl, thiadiazolyl, thiazolyl, thienyl, thiomorpholinyl (*e.g.* thiomorpholino ), and triazolyl. These heterocyclic groups can be substituted or unsubstituted. Where a group is substituted, the substituent can be alkyl, alkoxy, halogen, oxygen, or substituted or unsubstituted phenyl. Peptidomimetics may also have amino acid residues that have been chemically modified by phosphorylation, sulfonation, biotinylation, or the addition or removal of other moieties.

In a further embodiment, PIF is a compound of the formula R₁-R₂-R₃-R₄-R₅-R₆-R₇-R₈-R₉-R₁₀-R₁₁-R₁₂-R₁₃-R₁₄-R₁₅, wherein R₁ is Met or a mimetic of Met or salt thereof, R₂ is Val or a mimetic of Val or salt thereof, R₃ is Arg or a mimetic of Arg, or any amino acid or salt thereof, R₄ is Ile or a mimetic of Ile or salt thereof, R₅ is Lys or a mimetic of Lys or salt thereof, R₆ is Pro or a mimetic of Pro or salt thereof, R₇ is Gly or a mimetic of Gly or salt thereof, R₈ is Ser or a mimetic of Ser or salt thereof, R₉ is Ala or a mimetic of Ala or salt thereof, R₁₀ is Asn or a mimetic of Asn or salt thereof, R₁₁ is Lys or a mimetic of Lys or salt thereof, R₁₂ is Pro or a mimetic of Pro or salt thereof, R₁₃ is Ser or a mimetic of Ser or salt thereof, R₁₄ is Asp or a mimetic of Asp or salt thereof and R₁₅ is Asp or a mimetic of Asp or salt thereof. In a further embodiment, a compound comprising the formula R₁-R₂-R₃-R₄-R₅-R₆-R₇-R₈- R₉-R₁₀-R₁₁-R₁₂-R₁₃-R₁₄-R₁₅, wherein R₁ is a mimetic of the naturally occurring residue at position 1 or salt thereof of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₂ is a mimetic of the naturally occurring residue at position 2 or salt thereof of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₃ is a mimetic of the naturally occurring residue at position 3 or salt thereof of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₄ is a mimetic of the naturally occurring residue at position 4 or salt thereof of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₅ is a mimetic or salt thereof of the naturally occurring residue at position 5 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₆ is a mimetic or salt thereof of the naturally occurring residue at position 6 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₇ is a mimetic or salt thereof of the naturally occurring residue at position 7 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₈ is a mimetic or salt thereof of the naturally occurring residue at position 5 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₉ is a mimetic or salt thereof of the naturally occurring residue at position 9 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₁₀ is a mimetic or salt thereof of the naturally occurring residue at position 10 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₁₁ is a mimetic or salt thereof of the naturally occurring residue at position 11 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₁₂ is a mimetic or salt thereof of the naturally occurring residue at position 12 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₁₃ is a mimetic or salt thereof of the naturally occurring residue at position 13 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₁₄ is a mimetic or salt thereof of the naturally occurring residue at position 14 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences; wherein R₁₅ is a mimetic or salt thereof of the naturally occurring residue at position 15 of SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, or SEQ ID NO:29 or the residue at that position of such sequences.

In some embodiments, the pharmaceutical composition comprising the formula R₁-R₂-R₃-R₄-R₅-R₆-R₇-R₈- R₉-R₁₀-R₁₁-R₁₂-R₁₃-R₁₄-R₁₅-R₁₆-R₁₇-R₁₈, wherein R₁ is Ser or a mimetic of Ser or salt thereof, R₂ is Gly or a mimetic of Gly or salt thereof, R₃ is Ile or a mimetic of Ile or salt thereof, R₄ is Val or a mimetic of Val or salt thereof, R₅ is Ile or a mimetic of Ile or salt thereof, R₆ is Tyr or a mimetic of Tyr or salt thereof, R₇ is Gln or a mimetic of Gln or salt thereof, R₈ is Tyr or a mimetic of Tyr or salt thereof, R₉ is Met or a mimetic of Met or salt thereof, R₁₀ is Asp or a mimetic of Asp or salt thereof, R₁₁ is Asp or a mimetic of Asp or salt thereof, R₁₂ is Arg or a mimetic of Arg or salt thereof, R₁₃ is Tyr or a mimetic of Tyr or salt thereof, R₁₄ is Val or a mimetic of Val or salt thereof, R₁₅ is Gly or a mimetic of Gly or salt thereof, R₁₆ is Ser or a mimetic of Ser or salt thereof, R₁₇ is Asp or a mimetic of Asp or salt thereof; and R₁₈ is Leu or a mimetic of Leu or salt thereof; and a compound comprising the formula R₁-R₂-R₃-R₄-R₅-R₆-R₇-R₈- R₉, wherein R₁ is Val or a mimetic of Val or salt thereof, R₂ is Ile or a mimetic of Ile or salt thereof, R₃ is Ile or a mimetic of Ile or salt thereof, R₄ is Ile or a mimetic of Ile or salt thereof, R₅ is Ala or a mimetic of Ala or salt thereof, R₆ is Gln or a mimetic of Gln or salt thereof, R₇ is Tyr or a mimetic of Tyr or salt thereof, R₈ is Met or a mimetic of Met or salt thereof, and R₉ is Asp or a mimetic of Asp or salt thereof, is provided. In some embodiments, R₃ is not Arg or a mimetic of Arg or a salt thereof.

A variety of techniques are available for constructing peptide mimetics with the same or similar desired biological activity as the corresponding native but with more favorable activity than the peptide with respect to solubility, stability, and/or susceptibility to hydrolysis or proteolysis (see, e.g., Morgan & Gainor, Ann. Rep. Med. Chern. 24,243-252,1989). Certain peptidomimetic compounds are based upon the amino acid sequence of the peptides of the disclosure. Often, peptidomimetic compounds are synthetic compounds having a three dimensional structure (*i.e*. a "peptide motif") based upon the three-dimensional structure of a selected peptide. The peptide motif provides the peptidomimetic compound with the desired biological activity, *i.e.,* binding to PIF receptors, wherein the binding activity of the mimetic compound is not substantially reduced, and is often the same as or greater than the activity of the native peptide on which the mimetic is modeled. Peptidomimetic compounds can have additional characteristics that enhance their therapeutic application, such as increased cell permeability, greater affinity and/or avidity and prolonged biological half-life.

Peptidomimetic design strategies are readily available in the art (see, e.g., Ripka & Rich, Curr. Op. Chern. Bioi. 2,441-452,1998; Hruby et al., Curr. Op.Chem. Bioi. 1,114-119,1997; Hruby & Baise, Curr.Med. Chern. 9,945-970,2000). One class of peptidomimetics a backbone that is partially or completely non-peptide, but mimics the peptide backbone atom-for atom and comprises side groups that likewise mimic the functionality of the side groups of the native amino acid residues. Several types of chemical bonds, e.g., ester, thioester, thioamide, retroamide, reduced carbonyl, dimethylene and ketomethylene bonds, are known in the art to be generally useful substitutes for peptide bonds in the construction of protease-resistant peptidomimetics. Another class of peptidomimetics comprises a small nonpeptide molecule that binds to another peptide or protein, but which is not necessarily a structural mimetic of the native peptide. Yet another class of peptidomimetics has arisen from combinatorial chemistry and the generation of massive chemical libraries. These generally comprise novel templates which, though structurally unrelated to the native peptide, possess necessary functional groups positioned on a nonpeptide scaffold to serve as "topographical" mimetics of the original peptide (Ripka & Rich, 1998, *supra*)*.*

A list of PIF amino acid sequences are provided below in Table 1. Antibodies to various PIF peptides and scrambled PIF peptides are also provided.

**Table 1. PIF Peptides**

| (SEQ ID NO) | Peptide | Amino Acid Sequence |
|---|---|---|
| SEQ ID NO: 1 | nPIF-1₁₅ | MVRIKPGSANKPSDD |
| isolated native, matches region of Circumsporozoite protein (Malaria) | | |
| SEQ ID NO:2 | nPIF -1₍₁₅₋ₐₗₜₑᵣ₎ | MVRIKYGSYNNKPSD |
| isolated native, matches region of Circumsporozoite protein (Malaria) | | |
| SEQ ID NO:3 | nPIF-1₍₁₃₎ | MVRIKPGSANKPS |
| isolated native, matches region of Circumsporozoite protein (Malaria) | | |
| SEQ ID NO:4 | nPIF-1₍₉₎ | MVRIKPGSA |
| isolated native, matches region of Circumsporozoite protein (Malaria) | | |
| SEQ ID NO:5 | scrPIF-1₁₅ | GRVDPSNKSMPKDIA |
| synthetic, scrambled amino acid sequence from region of Circumsporozoite protein Malaria | | |
| SEQ ID NO:6 | nPIF-2₍₁₀₎ | SQAVQEHAST |
| isolated native, matches region of human retinoid and thyroid hormone receptor-SMRT | | |
| SEQ ID NO:7 | nPIF-2₍₁₃₎ | SQAVQEHASTNMG |
| isolated native, matches region of human retinoid and thyroid hormone receptor (SMRT) | | |
| SEQ ID NO:8 | scrPIF-2₍₁₃₎ | EVAQHSQASTMNG |
| synthetic, scrambled amino acid sequence from region of human retinoid and thyroid hormone receptor SMRT | | |
| SEQ ID NO:9 | scrPIF-2₍₁₄₎ | GQASSAQMNSTGVH |
| SEQ ID NO:10 | nPIF-3₍₁₈₎ | SGIVIYQYMDDRYVGSDL |
| isolated native, matches region of Rev Trans | | |
| SEQ ID NO:11 | Neg control for negPIF-1₍₁₅₎ | GMRELQRSANK |
| synthetic, scrambled amino acid sequence from region of Circumsporozoite protein Malaria | | |
| SEQ ID NO: 12 | nPIF-4₍₉₎ | VIIIAQYMD |
| isolated native, matches region of Rev Trans | | |
| antibody of native isolated nPIF-1₁₅ | AbPIF-1₍₁₅₎ | |
| (SEQ ID NO: 13) | sPIF-1₍₁₅₎ | MVRIKPGSANKPSDD |
| synthetic, amino acid sequence from region of Circumsporozoite protein Malaria | | |
| (SEQ ID NO:14) | sPIF-2₍₁₃₎ | SQAVQEHASTNMG |
| synthetic, amino acid sequence from of human retinoid and thyroid hormone receptor SMRT | | |
| (SEQ ID NO: 15) | sPIF-3₍₁₈₎ | SGIVIYQYMDDRYVGSDL |
| synthetic, amino acid sequence from region of Circumsporozoite protein Malaria | | |
| (SEQ ID NO: 16) | sPIF-1₍₉₎ | MVRIKPGSA |
| synthetic, amino acid sequence from region of Circumsporozoite protein Malaria | | |
| antibody of native isolated nPIF-2₍₁₃₎ | AbPIF-2₍₁₃₎ | |
| antibody of native isolated nPIF -3₍₁₈₎ | AbPIF-3₍₁₈₎ | |
| (SEQ ID NO: 17) | sPIF-4₍₉₎ | VIIIAQYMD |
| Synthetic | | |
| SEQ ID NO: 18 Synthetic | sPIF-1₍₅₎ | MVRIK |
| SEQ ID NO: 19 Synthetic | sPIF-1₍₄₎ | PGSA |
| SEQ ID NO: 20 | PIF (-3) | MVXIKPGSANKPSDD |
| SEQ ID NO: 21 | PIF (-1) | XVRIKPGSANKPSDD |
| SEQ ID NO: 22 | PIF (-1, -3) | XVXIKPGSANKPSDD |
| SEQ ID NO: 23 | PIF (-6) | MVRIKXGSANKPSDD |
| SEQ ID NO: 24 | PIF (-4) | MVRXKPGSANKPSDD |
| SEQ ID NO: 25 | PIF (-2) | MXRIKPGSANKPSDD |
| SEQ ID NO: 26 | mut1 | MVRIKEGSANKPSDD |
| SEQ ID NO: 27 | mut3 | MVRGKPGSANKPSDD |
| SEQ ID NO: 28 | mut4 | MERIKPGSANKPSDD |
| SEQ ID NO: 29 | mut5 | AVRIKPGSANKPSDD |
| n=native, s= synthetic, scr =scrambled, same AA, ( )= number of AA, Ab=antibody, X = any amino acid, except arginine | | |

In some embodiments of the present disclosure, a PIF peptide (or analog) is provided. In some embodiments, the PIF analog binds or associates with human insulin degrading enzyme (IDE - SEQ ID NO:30) at least 1%, 2%, 3%, 4%, 5%, 6%, 7%, 8%, 9%, or 10% or higher than native or wild-type PIF sequences. In some embodiments, the PIF analog may have a binding affinity for insulin degrading enzyme (IDE) that has at least 1%, 2%, 3%, 4%, 5%, 6%, 7%, 8%, 9%, or 10% or higher than native or wild-type PIF sequences. In some embodiments, the PIF analog may have a binding affinity for insulin degrading enzyme that has from about 1% to about 30% or higher than the affinity native or wild-type PIF sequences have for IDE. In some embodiments, the PIF analog may have a binding affinity for insulin degrading enzyme that has from about 1% to about 10% or higher than the affinity native or wild-type PIF sequences have for IDE. In some embodiments, the PIF analog may have a binding affinity for insulin degrading enzyme that has from about 1% to about 20% or higher than the affinity native or wild-type PIF sequences have for IDE. In some embodiments, the PIF analog may have a binding affinity for insulin degrading enzyme that has from about 10% to about 20% or higher than the affinity native or wild-type PIF sequences have for IDE. Such PIF peptides in therapeutically effective amounts may be useful for treating any of the diseases or disorder disclosed herein.

IDE sequence:



















 (SEQ ID NO:30).

In another embodiment, a pharmaceutical composition comprising a PIF peptide is provided. In preferred embodiments, the pharmaceutical composition comprises a therapeutically effective amount of a PIF peptide or a pharmaceutically acceptable salt thereof.

In another embodiment, a method of treating Duchenne's Muscular Dystrophy is provided. In a preferred embodiment, the method comprises administering an effective amount of a PIF peptide to a subject in need thereof. In a further embodiment, a method for treating Duchenne's Muscular Dystrophy comprising administering a therapeutically effective amount of a PIF peptide in combination with one or more immunotherapeutic, antiepileptic, diuretic, or blood pressure controlling drugs or compounds to a subject in need thereof is provided. Such a combination may enhance the effectiveness of the treatment of either component alone, or may provide less side effects and/or enable a lower dose of either component.

In one embodiment of the present invention, a PIF peptide is provided. Such PIF peptides may be useful for treating or ameliorating dystrophy-related disorders, such as DMD.

In another embodiment, a pharmaceutical composition comprising a PIF peptide or a pharmaceutically acceptable salt thereof is provided. In preferred embodiments, the pharmaceutical composition comprises a therapeutically effective amount of a PIF peptide or a pharmaceutically acceptable salt thereof.

In another embodiment, a method of treating or preventing disorders is provided. In a preferred embodiment, the method comprises administering a therapeutically effective amount of a PIF peptide to a subject in need thereof. The methods are particularly useful in treating or preventing immune-mediated disorders, including, but not limited to, graft-versus-host disease type 1 diabetes, multiple sclerosis, ulcerative colitis, Crohn's disease, rheumatoid arthritis and the like.

For therapeutic treatment of the specified indications, a PIF peptide may be administered as such, or can be compounded and formulated into pharmaceutical compositions in unit dosage form for parenteral, transdermal, rectal, nasal, local intravenous administration, or, preferably, oral administration. Such pharmaceutical compositions are prepared in a manner well known in the art and comprise at least one active PIF peptide associated with a pharmaceutically carrier. The term "active compound", as used throughout this specification, refers to at least one compound selected from compounds of the formulas or pharmaceutically acceptable salts thereof.

In such a composition, the active compound is known as "active ingredient." In making the compositions, the active ingredient will usually be mixed with a carrier, or diluted by a carrier, or enclosed within a carrier that may be in the form of a capsule, sachet, paper or other container. When the carrier serves as a diluent, it may be a solid, semisolid, or liquid material that acts as a vehicle, excipient of medium for the active ingredient. Thus, the composition can be in the form of tablets, pills, powders, lozenges, sachets, cachets, elixirs, emulsion, solutions, syrups, suspensions, soft and hard gelatin capsules, sterile injectable solutions, and sterile packaged powders.

The terms "pharmaceutical preparation" or "pharmaceutical composition" includes preparations suitable for administration to mammals, e.g., humans. When the compounds of the present disclosure are administered as pharmaceuticals to mammals, e.g., humans, they can be given per se or as a pharmaceutical composition containing, for example, from about 0.1 to about 99.5% of active ingredient in combination with a pharmaceutically acceptable carrier.

The phrase "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans. In a preferred embodiment, the therapeutic composition is not immunogenic when administered to a subject for therapeutic purposes.

The phrase "pharmaceutically acceptable carrier" is art recognized and includes a pharmaceutically acceptable material, composition or vehicle, suitable for administering compounds of the present disclosure to mammals. In some embodiments, compositions and pharmaceutical compositions of the disclosure comprise one or a plurality of pharmaceutically acceptbale carriers disclosed herein. The carriers include liquid or solid filler, diluent, excipient, solvent or encapsulating material, involved in carrying or transporting the subject agent from one organ, or portion of the body, to another organ, or portion of the body. Each carrier must be "acceptable" in the sense of being compatible with the other ingredients of the formulation and not injurious to the patient. Some examples of materials which can serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution or Lactated Ringer's solution; ethyl alcohol; phosphate buffer solutions; and other non-toxic compatible substances employed in pharmaceutical formulations. Suitable pharmaceutical carriers are described in "Remington's Pharmaceutical Sciences" by E. W. Martin, which is incorporated herein by reference in its entirety. In some embodiments, the pharmaceutically acceptable carrier is sterile and pyrogen-free water. In some embodiments, the pharmaceutically acceptable carrier is Ringer's Lactate, sometimes known as lactated Ringer's solution.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, .alpha.-tocopherol, and the like; and metal chelating agents, such as citric acid, ethylenediamine tetraacetic acid (EDTA), sorbitol, tartaric acid, phosphoric acid, and the like.

Formulations of the present disclosure include those suitable for oral, nasal, topical, buccal, sublingual, rectal, vaginal and/or parenteral administration. The formulations may conveniently be presented in unit dosage form and may be prepared by any methods well known in the art of pharmacy. The amount of active ingredient that can be combined with a carrier material to produce a single dosage form will generally be that amount of the compound that produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

Some examples of suitable carriers, excipients, and diluents include lactose, dextrose, sucrose, sorbitol, mannitol, starches, gum acacia, calcium phosphate alginates, calcium salicate, microcrystalline cellulose, polyvinylpyrrolidone, cellulose, tragacanth, gelatin, syrup, methyl cellulose, methyl- and propylhydroxybenzoates, tale, magnesium stearate, water, and mineral oil. The formulations can additionally include lubricating agents, wetting agents, emulsifying and suspending agents, preserving agents, sweetening agents or flavoring agents. The compositions may be formulated so as to provide quick, sustained, or delayed release of the active ingredient after administration to the patient by employing procedures well known in the art.

For oral administration, a compound can be admixed with carriers and diluents, molded into tablets, or enclosed in gelatin capsules. The mixtures can alternatively be dissolved in liquids such as 10% aqueous glucose solution, isotonic saline, sterile water, or the like, and administered intravenously or by injection.

The local delivery of inhibitory amounts of active compound for the treatment of dystrophy-related can be by a variety of techniques that administer the compound at or near the targeted site. Examples of local delivery techniques are not intended to be limiting but to be illustrative of the techniques available. Examples include local delivery catheters, site specific carriers, implants, direct injection, or direct applications, such as topical application.

Local delivery by an implant describes the surgical placement of a matrix that contains the pharmaceutical agent into the affected site. The implanted matrix releases the pharmaceutical agent by diffusion, chemical reaction, or solvent activators.

For example, in some aspects, the disclosure is directed to a pharmaceutical composition comprising a PIF peptide, and a pharmaceutically acceptable carrier or diluent, or a therapeutically effective amount of pharmaceutical composition comprising a PIF peptide or salt thereof.

The compounds of the present disclosure can be administered in the conventional manner by any route where they are active. Administration can be systemic, topical, or oral. For example, administration can be, but is not limited to, parenteral, subcutaneous, intravenous, intramuscular, intraperitoneal, transdermal, oral, buccal, ocular routes, intravaginally, by inhalation, by depot injections, or by implants. Thus, modes of administration for the compounds of the present disclosure (either alone or in combination with other pharmaceuticals) can be, but are not limited to, subligual, injectable (including short-acting, depot, implant and pellet forms injected subcutaneously or intramuscularly), or by use of vaginal creams, suppositories, pessaries, vaginal rings, rectal suppositories, intrauterine devices, and transdermal forms such as patches and creams.

Specific modes of administration will depend on the indication. The selection of the specific route of administration and the dose regimen is to be adjusted or titrated by the clinician according to methods known to the clinician in order to obtain the optimal clinical response. The amount of compound to be administered is that amount which is therapeutically effective. The dosage to be administered will depend on the characteristics of the subject being treated, e.g., the particular mammal or human treated, age, weight, health, types of concurrent treatment, if any, and frequency of treatments, and can be easily determined by one of skill in the art (e.g., by the clinician).

Pharmaceutical formulations containing the compounds of the present disclosure and a suitable carrier can be solid dosage forms which include, but are not limited to, tablets, capsules, cachets, pellets, pills, powders and granules; topical dosage forms which include, but are not limned to, solutions, powders, fluid emulsions, fluid suspensions, semi-solids, ointments, pastes, creams, gels and jellies, and foams; and parenteral dosage forms which include, but are not limited to, solutions, suspensions, emulsions, and dry powder; comprising an effective amount of a polymer or copolymer of the present disclosure. It is also known in the art that the active ingredients can be contained in such formulations with pharmaceutically acceptable diluents, fillers, disintegrants, binders, lubricants, surfactants, hydrophobic vehicles, water soluble vehicles, emulsifiers, buffers, humectants, moisturizers, solubilizers, preservatives and the like. The means and methods for administration are known in the art and an artisan can refer to various pharmacologic references for guidance. For example, Modern Pharmaceutics, Banker & Rhodes, Marcel Dekker, Inc. (1979); and Goodman & Gilman's The Pharmaceutical Basis of Therapeutics, 6th Edition, MacMillan Publishing Co., New York (1980) can be consulted.

The compounds of the present disclosure can be formulated for parenteral administration by injection, e.g., by bolus injection or continuous infusion. The compounds can be administered by continuous infusion subcutaneously over a predetermined period of time. Formulations for injection can be presented in unit dosage form, e.g., in ampoules or in multi-dose containers, with an added preservative. The compositions can take such forms as suspensions, solutions or emulsions in oily or aqueous vehicles, and can contain formulatory agents such as suspending, stabilizing and/or dispersing agents.

For oral administration, the compounds can be formulated readily by combining these compounds with pharmaceutically acceptable carriers well known in the art. Such carriers enable the compounds of the disclosure to be formulated as tablets, pills, dragees, capsules, liquids, gels, syrups, slurries, suspensions and the like, for oral ingestion by a patient to be treated. Pharmaceutical preparations for oral use can be obtained by adding a solid excipient, optionally grinding the resulting mixture, and processing the mixture of granules, alter adding suitable auxiliaries, if desired, to obtain tablets or dragee cores. Suitable excipients include, but are not limited to, fillers such as sugars, including, but not limited to, lactose, sucrose, mannitol, and sorbitol; cellulose preparations such as, but not limited to, maize starch, wheat starch, rice starch, potato starch, gelatin, gum tragecanth, methyl cellulose, hydroxypropylmethyl-celllose, sodium carboxymethylcellulose, and polyvinylpyrrolidone (PVP). If desired, disintegrating agents can be added, such as, but not limited to, the cross-linked polyvinyl pyrrolidone, agar, or alginic acid or a salt thereof such as sodium alginate.

Dragee cores can be provided with suitable coatings. For this purpose, concentrated sugar solutions can be used, which can optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments can be added to the tablets or dragee coatings for identification or to characterize different combinations of active compound doses.

Pharmaceutical preparations which can be used orally include, but are not limited to, push-fit capsules made of gelatin, as well as soft, scaled capsules made of gelatin and a plasticizer, such as glycerol or sorbitol. The push-fit capsules can contain the active ingredients in admixture with filler such as, e.g., lactose, binders such as, e.g., starches, and/or lubricants such as, e.g., talc or magnesium stearate and, optionally, stabilizers. In soft capsules, the active compounds can be dissolved or suspended in suitable liquids, such as fatty oils, liquid paraffin, or liquid polyethylene glycols. In addition, stabilizers can be added. All formulations for oral administration should be in dosages suitable for such administration.

For buccal administration, the compositions can take the form of, e.g., tablets or lozenges formulated in a conventional manner.

For administration by inhalation, the compounds for use according to the present disclosure are conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit can be determined by providing a valve to deliver a metered amount. Capsules and cartridges of, e.g., gelatin for use in an inhaler or insufflator can be formulated containing a powder mix of the compound and a suitable powder base such as lactose or starch.

The compounds of the present disclosure can also be formulated in rectal compositions such as suppositories or retention enemas, e.g., containing conventional suppository bases such as cocoa butter or other glycerides.

In addition to the formulations described previously, the compounds of the present disclosure can also be formulated as a depot preparation. Such long acting formulations can be administered by implantation (for example subcutaneously or intramuscularly) or by intramuscular injection.

Depot injections can be administered at about 1 month to about 6 months or longer intervals. Thus, for example, the compounds can be formulated with suitable polymeric or hydrophobic materials (for example as an emulsion in an acceptable oil) or ion exchange resins, or as sparingly soluble derivatives, for example, as a sparingly soluble salt.

In transdermal administration, the compounds of the present disclosure, for example, can be applied to a plaster, or can be applied by transdermal, therapeutic systems that are consequently supplied to the organism.

Pharmaceutical compositions of the compounds also can comprise suitable solid or gel phase carriers or excipients. Examples of such carriers or excipients include but are not limited to calcium carbonate, calcium phosphate, various sugars, starches, cellulose derivates, gelatin, and polymers such as, e.g., polyethylene glycols.

For parenteral administration, analog can be, for example, formulated as a solution, suspension, emulsion or lyophilized powder in association with a pharmaceutically acceptable parenteral vehicle. Examples of such vehicles are water, saline, Ringer's solution, dextrose solution, and 5% human serum albumin. Liposomes and nonaqueous vehicles such as fixed oils may also be used. The vehicle or lyophilized powder may contain additives that maintain isotonicity (e.g., sodium chloride, mannitol) and chemical stability (e.g., buffers and preservatives). The formulation is sterilized by commonly used techniques. For example, a parenteral composition suitable for administration by injection is prepared by dissolving 1.5% by weight of analog in 0.9% sodium chloride solution.

The present invention relates to routes of administration include intramuscular, sublingual, intravenous, intraperitoneal, intrathecal, intravaginal, intraurethral, intradermal, intrabuccal, via inhalation, via nebulizer and via subcutaneous injection. Alternatively, the pharmaceutical composition may be introduced by various means into cells that are removed from the individual. Such means include, for example, microprojectile bombardment and liposome or other nanoparticle device.

Solid dosage forms for oral administration include capsules, tablets, pills, powders and granules. In solid dosage forms, the analogs are generally admixed with at least one inert pharmaceutically acceptable carrier such as sucrose, lactose, starch, or other generally regarded as safe (GRAS) additives. Such dosage forms can also comprise, as is normal practice, an additional substance other than an inert diluent, e.g., lubricating agent such as magnesium state. With capsules, tablets, and pills, the dosage forms may also comprise a buffering agent. Tablets and pills can additionally be prepared with enteric coatings, or in a controlled release form, using techniques know in the art.

Liquid dosage forms for oral administration include pharmaceutically acceptable emulsions, solutions, suspensions and syrups, with the elixirs containing an inert diluent commonly used in the art, such as water. These compositions can also include one or more adjuvants, such as wetting agent, an emulsifying agent, a suspending agent, a sweetening agent, a flavoring agent or a perfuming agent.

In other embodiments of the invention thecomposition of the disclosure the pharmaceutical compositions of the disclosure are used to treat a patient suffering from, or susceptible to Duchenne's Muscular Dystrophy or a dystrophy-related disorder.

One of skill in the art will recognize that the appropriate dosage of the compositions and pharmaceutical compositions may vary depending on the individual being treated and the purpose. For example, the age, body weight, and medical history of the individual patient may affect the therapeutic efficacy of the therapy. Further, a lower dosage of the composition may be needed to produce a transient cessation of symptoms, while a larger dose may be needed to produce a complete cessation of symptoms associated with the disease, disorder, or indication. A competent physician can consider these factors and adjust the dosing regimen to ensure the dose is achieving the desired therapeutic outcome without undue experimentation. It is also noted that the clinician and/or treating physician will know how and when to interrupt, adjust, and/or terminate therapy in conjunction with individual patient response. Dosages may also depend on the strength of the particular analog chosen for the pharmaceutical composition.

The dose of the composition or pharmaceutical compositions may vary. The dose of the composition may be once per day. In some embodiments, multiple doses may be administered to the subject per day. In some embodiments, the total dosage is administered in at least two application periods. In some embodiments, the period can be an hour, a day, a month, a year, a week, or a two-week period. In an additional embodiment of the invention, the total dosage is administered in two or more separate application periods, or separate doses over the course of about an hour, 2 hours, 3 hours, 4 hours, 5 hours, 6 hours, 7 hours, 8 hours, 9 hours, 10 hours, 11 hours, 12 or more hours. a day, a month, a year, a week, or a two-week period.

In some embodiments, subjects can be administered the composition in which the composition is provided in a daily dose range of about 0.0001 mg/kg to about 5000 mg/kg of the weight of the subject. The dose administered to the subject can also be measured in terms of total amount of PIF peptide or PIF analog or pharmaceutically acceptable salt thereof administered per day. In some embodiments, a subject is administered from about 0.001 to about 3000 milligrams of PIF peptide or PIF analog or pharmaceutically acceptable salt thereof per day. In some embodiments, a subject is administered up to about 2000 milligrams of PIF peptide or PIF analog or pharmaceutically acceptable salt thereof per day. In some embodiments, a subject is administered up to about 1800 milligrams of PIF peptide or PIF analog or pharmaceutically acceptable salt thereof per day. In some embodiments, a subject is administered up to about 1600 milligrams of PIF peptide or PIF analog or pharmaceutically acceptable salt thereof per day. In some embodiments, a subject is administered up to about 1400 milligrams of PIF peptide or PIF analog or pharmaceutically acceptable salt thereof per day. In some embodiments, a subject is administered up to about 1200 milligrams of PIF peptide or PIF analog or pharmaceutically acceptable salt thereof per day. In some embodiments, a subject is administered up to about 1000 milligrams of PIF peptide or PIF analog or pharmaceutically acceptable salt thereof per day. In some embodiments, a subject is administered up to about 800 milligrams of PIF peptide or PIF analog or pharmaceutically acceptable salt thereof per day. In some embodiments, a subject is administered from about 0.001 milligrams to about 700 milligrams of PIF peptide or PIF analog or pharmaceutically acceptable salt thereof per dose. In some embodiments, a subject is administered up to about 700 milligrams of PIF peptide or PIF analog per dose. In some embodiments, a subject is administered up to about 600 milligrams of PIF peptide or PIF analog or pharmaceutically acceptable salt thereof per dose. In some embodiments, a subject is administered up to about 500 milligrams of PIF peptide or PIF analog or pharmaceutically acceptable salt thereof per dose. In some embodiments, a subject is administered up to about 400 milligrams of PIF peptide or PIF analog or pharmaceutically acceptable salt thereof per dose. In some embodiments, a subject is administered up to about 300 milligrams of PIF peptide or PIF analog or pharmaceutically acceptable salt thereof per dose. In some embodiments, a subject is administered up to about 200 milligrams of PIF peptide or PIF analog or pharmaceutically acceptable salt thereof per dose. In some embodiments, a subject is administered up to about 100 milligrams of PIF peptide or PIF analog or pharmaceutically acceptable salt thereof per dose. In some embodiments, a subject is administered up to about 50 milligrams of PIF peptide or PIF analog or pharmaceutically acceptable salt thereof per dose.

In some embodiments, subjects can be administered the composition in which the composition comprising a PIF peptide or PIF analog or pharmaceutically acceptable salt thereof is administered in a daily dose range of about 0.0001 mg/kg to about 5000 mg/kg of the weight of the subject. In some embodiments, the composition comprising a PIF analog or pharmaceutically acceptable salt thereof is administered in a daily dosage of up to about 450 mg/kg of the weight of the subject. In some embodiments, the composition comprising a PIF peptide or PIF analog or pharmaceutically acceptable salt thereof is administered in a daily dosage of up to about 400 mg/kg of the weight of the subject. In some embodiments, the composition comprising a PIF peptide or PIF analog or pharmaceutically acceptable salt thereof is administered in a daily dosage of up to about 350 mg/kg of the weight of the subject. In some embodiments, the composition comprising a PIF peptide or PIF analog or pharmaceutically acceptable salt thereof is administered in a daily dosage of up to about 300 mg/kg of the weight of the subject. In some embodiments, the composition comprising a PIF peptide or PIF analog or pharmaceutically acceptable salt thereof is administered in a daily dosage of up to about 250 mg/kg of the weight of the subject. In some embodiments, the composition comprising PIF peptide or a PIF analog or pharmaceutically acceptable salt thereof is administered in a daily dosage of up to about 200 mg/kg of the weight of the subject. In some embodiments, the composition comprising PIF peptide or a PIF analog or pharmaceutically acceptable salt thereof is administered in a daily dosage of up to about 150 mg/kg of the weight of the subject. In some embodiments, the composition comprising a PIF peptide or a PIF analog or pharmaceutically acceptable salt thereof is administered in a daily dosage of up to about 100 mg/kg of the weight of the subject. In some embodiments, the composition comprising a PIF peptide or a PIF analog or pharmaceutically acceptable salt thereof is administered in a daily dosage of up to about 50 mg/kg of the weight of the subject. In some embodiments, the composition comprising PIF peptide or a PIF analog or pharmaceutically acceptable salt thereof is administered in a daily dosage of up to about 25 mg/kg of the weight of the subject.

In some embodiments, the composition comprising a PIF peptide or a PIF analog or pharmaceutically acceptable salt thereof is administered in a daily dosage of up to about 10 mg/kg of the weight of the subject. In some embodiments, the composition comprising PIF peptide or a PIF analog or pharmaceutically acceptable salt thereof is administered in a daily dosage of up to about 5 mg/kg of the weight of the subject. In some embodiments, the composition comprising a PIF peptide or a PIF analog or pharmaceutically acceptable salt thereof is administered in a daily dosage of up to about 2.0 mg/kg of the weight of the subject. In some embodiments, the composition comprising a PIF peptide or a PIF analog or pharmaceutically acceptable salt thereof is administered in a daily dosage of up to about 3.0 mg/kg of the weight of the subject. In some embodiments, the composition comprising PIF peptide or a PIF analog or pharmaceutically acceptable salt thereof is administered in a daily dosage of up to about 1 mg/kg of the weight of the subject. In some embodiments, the composition comprising a PIF peptide or a PIF analog or pharmaceutically acceptable salt thereof is administered in a daily dosage of up to about 0.1 mg/kg of the weight of the subject. In some embodiments, the composition comprising a PIF analog or pharmaceutically acceptable salt thereof is administered in a daily dosage of up to about 0.01 mg/kg of the weight of the subject. In some embodiments, the composition comprising a PIF analog or pharmaceutically acceptable salt thereof is administered in a daily dosage of up to about 0.001 mg/kg of the weight of the subject. The dose administered to the subject can also be measured in terms of total amount of a PIF peptide or PIF analog administered per day.

In some embodiments, a subject in need thereof is administered from about 1 ng to about 500 µg of analog or pharmaceutically salt thereof per day. In some embodiments, a subject in need thereof is administered from about 1 ng to about 10 ng of analog or pharmaceutically salt thereof per day. In some embodiments, a subject in need thereof is administered from about 10 ng to about 20 ng of analog or pharmaceutically salt thereof per day. In some embodiments, a subject in need thereof is administered from about 10 ng to about 100 ng of analog or pharmaceutically salt thereof per day. In some embodiments, a subject in need thereof is administered from about 100 ng to about 200 ng of analog or pharmaceutically salt thereof per day. In some embodiments, a subject in need thereof is administered from about 200 ng to about 300 ng of analog or pharmaceutically salt thereof per day. In some embodiments, a subject in need thereof is administered from about 300 ng to about 400 ng of analog or pharmaceutically salt thereof per day. In some embodiments, a subject in need thereof is administered from about 400 ng to about 500 ng of analog or pharmaceutically salt thereof per day. In some embodiments, a subject in need thereof is administered from about 500 ng to about 600 ng of analog or pharmaceutically salt thereof per day. In some embodiments, a subject in need thereof is administered from about 600 ng to about 700 ng of analog or pharmaceutically salt thereof per day. In some embodiments, a subject in need thereof is administered from about 800 ng to about 900 ng of analog or pharmaceutically salt thereof per day. In some embodiments, a subject in need thereof is administered from about 900 ng to about 1 µg of analog or pharmaceutically salt thereof per day. In some embodiments, a subject in need thereof is administered from about 1 µg to about 100 µg of analog or pharmaceutically salt thereof per day. In some embodiments, a subject in need thereof is administered from about 100 µg to about 200 µg of analog or pharmaceutically salt thereof per day. In some embodiments, a subject in need thereof is administered from about 200 µg to about 300 µg of analog or pharmaceutically salt thereof per day. In some embodiments, a subject in need thereof is administered from about 300 µg to about 400 µg of analog or pharmaceutically salt thereof per day. In some embodiments, a subject in need thereof is administered from about 400 µg to about 500 µg of analog or pharmaceutically salt thereof per day. In some embodiments, a subject in need thereof is administered from about 500 µg to about 600 µg of analog or pharmaceutically salt thereof per day. In some embodiments, a subject in need thereof is administered from about 600 µg to about 700 µg of analog or pharmaceutically salt thereof per day. In some embodiments, a subject in need thereof is administered from about 800 µg to about 900 µg of analog or pharmaceutically salt thereof per day. In some embodiments, a subject in need thereof is administered from about 900 µg to about 1 mg of analog or pharmaceutically salt thereof per day.

In some embodiments, a subject in need thereof is administered from about .0001 to about 3000 milligrams of a PIF peptide or PIF analog or pharmaceutically salt thereof per day. In some embodiments, a subject is administered up to about 2000 milligrams of a PIF peptide or PIF analog or pharmaceutically salt thereof day. In some embodiments, a subject is administered up to about 1800 milligrams of a PIF peptide or PIF analog or pharmaceutically salt thereof per day. In some embodiments, a subject is administered up to about 1600 milligrams of a PIF peptide or PIF analog or pharmaceutically salt thereof per day. In some embodiments, a subject is administered up to about 1400 milligrams of a PIF peptide or PIF analog or pharmaceutically salt thereof per day. In some embodiments, a subject is administered up to about 1200 milligrams of a PIF peptide or PIF analog or pharmaceutically salt thereof per day. In some embodiments, a subject is administered up to about 1000 milligrams of a PIF peptide or PIF analog or pharmaceutically salt thereof per day. In some embodiments, a subject is administered up to about 800 milligrams of a PIF peptide or PIF analog or pharmaceutically salt thereof per day. In some embodiments, a subject is administered from about 0.0001 milligrams to about 700 milligrams of a PIF peptide or PIF analog or pharmaceutically salt thereof per dose. In some embodiments, a subject is administered up to about 700 milligrams of a PIF peptide or PIF analog or pharmaceutically salt thereof per dose. In some embodiments, a subject is administered up to about 600 milligrams of a PIF peptide or PIF analog or pharmaceutically salt thereof per dose. In some embodiments, a subject is administered up to about 500 milligrams of a PIF peptide or PIF analog or pharmaceutically salt thereof per dose. In some embodiments, a subject is administered up to about 400 milligrams of a PIF peptide or PIF analog or pharmaceutically salt thereof per dose. In some embodiments, a subject is administered up to about 300 milligrams of a PIF peptide or PIF analog or pharmaceutically salt thereof per dose. In some embodiments, a subject is administered up to about 200 milligrams of a PIF peptide or PIF analog or pharmaceutically salt thereof per dose. In some embodiments, a subject is administered up to about 100 milligrams of a PIF peptide or PIF analog or pharmaceutically salt thereof per dose. In some embodiments, a subject is administered up to about 50 milligrams of a PIF peptide or PIF analog or pharmaceutically salt thereof per dose. In some embodiments, a subject is administered up to about 25 milligrams of a PIF peptide or PIF analog or pharmaceutically salt thereof per dose. In some embodiments, a subject is administered up to about 15 milligrams of a PIF peptide or PIF analog or pharmaceutically salt thereof per dose. In some embodiments, a subject is administered up to about 50, 60, 70, 80, 90, or 100 milligrams of a PIF peptide or PIF analog or pharmaceutically salt thereof per dose.

In some embodiments, a subject is administered up to about 10 milligrams of a PIF peptide or PIF analog or pharmaceutically salt thereof per dose. In some embodiments, a subject is administered up to about 5 milligrams of a PIF peptide or PIF analog or pharmaceutically salt thereof per dose. In some embodiments, a subject is administered up to about 1 milligram of a PIF peptide or PIF analog or pharmaceutically salt thereof per dose. In some embodiments, a subject is administered up to about 0.1 milligrams of a PIF peptide or PIF analog or pharmaceutically salt thereof per dose. In some embodiments, a subject is administered up to about 0.001 milligrams of a PIF peptide or PIF analog or pharmaceutically salt thereof per dose.

The dose administered to the subject can also be measured in terms of total amount of a PIF peptide or PIF analog or pharmaceutically salt thereof administered per ounce of liquid prepared. In some embodiments, the PIF peptide or PIF analog or pharmaceutically salt thereof is at a concentration of about 2.5 grams per ounce of solution. In some embodiments, the PIF peptide or PIF analog or pharmaceutically salt thereof is at a concentration of about 2.25 grams per ounce of solution. In some embodiments, the PIF peptide or PIF analog or pharmaceutically salt thereof is at a concentration of about 2.25 grams per ounce of solution. In some embodiments, the PIF peptide or PIF analog or pharmaceutically salt thereof is at a concentration of about 2.0 grams per ounce of solution. In some embodiments, the PIF peptide or PIF analog or pharmaceutically salt thereof is at a concentration of about 1.9 grams per ounce of solution. In some embodiments, the PIF peptide or PIF analog or pharmaceutically salt thereof is at a concentration of about 1.8 grams per ounce of solution. In some embodiments, the PIF analog or pharmaceutically salt thereof is at a concentration of about 1.7 grams per ounce of solution. In some embodiments, the PIF peptide or PIF analog or pharmaceutically salt thereof is at a concentration of about 1.6 grams per ounce of solution. In some embodiments, the PIF peptide or PIF analog or pharmaceutically salt thereof is at a concentration of about 1.5 grams per ounce of solution. In some embodiments, the PIF peptide or PIF analog or pharmaceutically salt thereof is at a concentration of about 1.4 grams per ounce of solution. In some embodiments, the PIF peptide or PIF analog or pharmaceutically salt thereof is at a concentration of about 1.3 grams per ounce of solution. In some embodiments, the PIF peptide or PIF analog or pharmaceutically salt thereof is at a concentration of about 1.2 grams per ounce of solution. In some embodiments, the PIF peptide or PIF analog or pharmaceutically salt thereof is at a concentration of about 1.1 grams per ounce of solution. In some embodiments, the PIF peptide or PIF analog or pharmaceutically salt thereof is at a concentration of about 1.0 grams per ounce of solution.

In some embodiments, the PIF peptide or PIF analog or pharmaceutically salt thereof is at a concentration of about 0.9 grams per ounce of solution. In some embodiments, the PIF peptide or PIF analog or pharmaceutically salt thereof is at a concentration of about 0.8 grams per ounce of solution. In some embodiments, the PIF peptide or PIF analog or pharmaceutically salt thereof is at a concentration of about 0.7 grams per ounce of solution. In some embodiments, the PIF peptide or PIF analog or pharmaceutically salt thereof is at a concentration of about 0.6 grams per ounce of solution. In some embodiments, the PIF peptide or PIF analog or pharmaceutically salt thereof is at a concentration of about 0.5 grams per ounce of solution. In some embodiments, the PIF peptide or PIF analog or pharmaceutically salt thereof is at a concentration of about 0.4 grams per ounce of solution. In some embodiments, the PIF peptide or PIF analog or pharmaceutically salt thereof is at a concentration of about 0.3 grams per ounce of solution. In some embodiments, the PIF peptide or PIF analog or pharmaceutically salt thereof is at a concentration of about 0.2 grams per ounce of solution. In some embodiments, the PIF peptide or PIF analog or pharmaceutically salt thereof is at a concentration of about 0.1 grams per ounce of solution. In some embodiments, the PIF peptide or PIF analog or pharmaceutically salt thereof is at a concentration of about 0.01 grams per ounce of solution. In some embodiments, the PIF peptide or PIF analog or pharmaceutically salt thereof is at a concentration of about 0.001 grams per ounce of solution prepared. In some embodiments, the PIF peptide or PIF analog or pharmaceutically salt thereof is at a concentration of about 0.0001 grams per ounce of solution prepared. In some embodiments, the PIF peptide or PIF analog or pharmaceutically salt thereof is at a concentration of about 0.00001 grams per ounce of solution prepared. In some embodiments, the PIF peptide or PIF analog or pharmaceutically salt thereof is at a concentration of about 0.000001 grams per ounce of solution prepared.

Dosage may be measured in terms of mass amount of analog per liter of liquid formulation prepared. One skilled in the art can increase or decrease the concentration of the analog in the dose depending upon the strength of biological activity desired to treat or prevent any above-mentioned disorders associated with the treatment of subjects in need thereof. For instance, some embodiments of the invention can include up to 0.00001 grams of analog per 5 mL of liquid formulation and up to about 10 grams of analog per 5 mL of liquid formulation. In some embodiments, the pharmaceutical compositions comprising a PIF analog or any of such compositions in any of the disclosed methods are free of SEQ ID NO: 1. In some embodiments, the pharmaceutical compositions comprising a PIF analog or any of such compositions in any of the disclosed methods are free of SEQ ID NO:2. In some embodiments, the pharmaceutical compositions comprising a PIF analog or any of such compositions in any of the disclosed methods are free of SEQ ID NO:3. In some embodiments, the pharmaceutical compositions comprising a PIF analog or any of such compositions in any of the disclosed methods are free of SEQ ID NO:4. In some embodiments, the pharmaceutical compositions comprising a PIF analog or any of such compositions in any of the disclosed methods are free of SEQ ID NO:5. In some embodiments, the pharmaceutical compositions comprising a PIF analog or any of such compositions in any of the disclosed methods are free of SEQ ID NO:6. In some embodiments, the pharmaceutical compositions comprising a PIF analog or any of such compositions in any of the disclosed methods are free of SEQ ID NO:7. In some embodiments, the pharmaceutical compositions comprising a PIF analog or any of such compositions in any of the disclosed methods are free of SEQ ID NO:8. In some embodiments, the pharmaceutical compositions comprising a PIF analog or any of such compositions in any of the disclosed methods are free of SEQ ID NO:9. In some embodiments, the pharmaceutical compositions comprising a PIF analog or any of such compositions in any of the disclosed methods are free of SEQ ID NO:10. In some embodiments, the pharmaceutical compositions comprising a PIF analog or any of such compositions in any of the disclosed methods are free of SEQ ID NO:11. In some embodiments, the pharmaceutical compositions comprising a PIF analog or any of such compositions in any of the disclosed methods are free of SEQ ID NO:12. In some embodiments, the pharmaceutical compositions comprising a PIF analog or any of such compositions in any of the disclosed methods are free of SEQ ID NO:13. In some embodiments, the pharmaceutical compositions comprising a PIF analog or any of such compositions in any of the disclosed methods are free of SEQ ID NO:14. In some embodiments, the pharmaceutical compositions comprising a PIF analog or any of such compositions in any of the disclosed methods are free of SEQ ID NO:15. In some embodiments, the pharmaceutical compositions comprising a PIF analog or any of such compositions in any of the disclosed methods are free of SEQ ID NO:16. In some embodiments, the pharmaceutical compositions comprising a PIF analog or any of such compositions in any of the disclosed methods are free of SEQ ID NO:17. In some embodiments, the pharmaceutical compositions comprising a PIF analog or any of such compositions in any of the disclosed methods are free of SEQ ID NO:18. In some embodiments, the pharmaceutical compositions comprising a PIF analog or any of such compositions in any of the disclosed methods are free of SEQ ID NO:19. In some embodiments, the pharmaceutical compositions comprising a PIF analog or any of such compositions in any of the disclosed methods are free of SEQ ID NO:20. In some embodiments, the pharmaceutical compositions comprising a PIF analog or any of such compositions in any of the disclosed methods are free of SEQ ID NO:21. In some embodiments, the pharmaceutical compositions comprising a PIF analog or any of such compositions in any of the disclosed methods are free of SEQ ID NO:22. In some embodiments, the pharmaceutical compositions comprising a PIF analog or any of such compositions in any of the disclosed methods are free of SEQ ID NO:23. In some embodiments, the pharmaceutical compositions comprising a PIF analog or any of such compositions in any of the disclosed methods are free of SEQ ID NO:24. In some embodiments, the pharmaceutical compositions comprising a PIF analog or any of such compositions in any of the disclosed methods are free of SEQ ID NO:25. In some embodiments, the pharmaceutical compositions comprising a PIF analog or any of such compositions in any of the disclosed methods are free of SEQ ID NO:26. In some embodiments, the pharmaceutical compositions comprising a PIF analog or any of such compositions in any of the disclosed methods are free of SEQ ID NO:27. In some embodiments, the pharmaceutical compositions comprising a PIF analog or any of such compositions in any of the disclosed methods are free of SEQ ID NO:28. In some embodiments, the pharmaceutical compositions comprising a PIF analog or any of such compositions in any of the disclosed methods are free of SEQ ID NO:29.

In some embodiments the pharmaceutical compositions of the claimed invention comprises a therapeutically effective amount of at least one or a plurality of active agents other than the PIF peptide, analog or pharmaceutically acceptable salt thereof. In some embodiments the active agent is covalently linked to the PIF peptide or PIF analog disclosed herein optionally by a protease cleavable linker (including by not limited to Pro-Pro or Cituline-Valine di-α-amino acid linkers). In some embodiments, the one or plurality of active agents is one or a combination of compounds chosen from: an anti-inflammatory compound, alpha-adrenergic agonist, antiarrhythmic compound, analgesic compound, and/or an anesthetic compound.

**Table Y**

| Examples of anti-inflammatory compounds include: |
|---|
| aspirin |
| celecoxib |
| diclofenac |
| diflunisal |
| etodolac |
| ibuprofen |
| indomethacin |
| ketoprofen |
| ketorolac nabumetone |
| naproxen |
| oxaprozin |
| piroxicam |
| salsalate |
| sulindac |
| tolmetin |

| Examples of alpha-adrenergic agonists include: |
|---|
| Methoxamine |
| Methy lnorepinephrine |
| Midodrine |
| Oxymetazoline |
| Metaraminol |
| Phenylephrine |
| Clonidine (mixed alpha2-adrenergic and imidazoline-Il receptor agonist) |
| Guanfacine, (preference for alpha2A-subtype of adrenoceptor) |
| Guanabenz (most selective agonist for alpha2-adrenergic as opposed to imidazoline-I1) |
| Guanoxabenz (metabolite of guanabenz) |
| Guanethidine (peripheral alpha2-receptor agonist) |
| Xylazine, |
| Tizanidine |
| Medetomidine |
| Methyldopa |
| Fadolmidine |
| Dexmedetomidine |

| Examples of antiarrhythmic compound include: |
|---|
| Amiodarone (Cordarone, Pacerone) |
| Bepridil Hydrochloride (Vascor) |
| Disopyramide (Norpace) |
| Dofetilide (Tikosyn) |
| Dronedarone (Multaq) |
| Flecainide (Tambocor) |
| Ibutilide (Corvert) |
| Lidocaine (Xylocaine) |
| Procainamide (Procan, Procanbid) |
| Propafenone (Rythmol) |
| Propranolol (Inderal) |
| Quinidine (many trade names) |
| Sotalol (Betapace) |
| Tocainide (Tonocarid) |

| Examples of analgesic compound include: |
|---|
| codeine |
| hydrocodone (Zohydro ER), |
| oxycodone (OxyContin, Roxicodone), |
| methadone |
| hydromorphone (Dilaudid, Exalgo), |
| morphine (Avinza, Kadian, MSIR, MS Contin), and |
| fentanyl (Actiq, Duragesic) |

| Examples of anesthetic compounds include: |
|---|
| Desflurane |
| Isoflurane |
| Nitrous oxide |
| Sevoflurane |
| Xenon |

The compounds of the present disclosure can also be administered in combination with, sequentially with other active ingredients, such as, for example, adjuvants, or other compatible drugs or compounds where such combination is seen to be desirable or advantageous in achieving the desired effects of the methods described herein.

### Methods

International Application Serial Number PCT/US2012/027480 is incorporated by reference in its entirety and any of the methods disclosed therein may be performed with the PIF analogs of this application in place of or in addition to the experiments performed with native PIF sequences. Comparative experimentation may demonstrate that the PIF analogs of this application may share one or more properties tested in International Application Serial Number PCT/US2012/027480.

The methods disclosed herein can be used with any of the compounds, compositions, preparations, and kits disclosed herein.

The disclosure also relates to methods for treating Duchenne's Muscular Dystrophy comprising administering an effective amount of the compositions described herein to a subject in need thereof.

In an embodiment, the composition is administered once a day to a subject in need thereof. In another embodiment, the composition is administered every other day, every third day or once a week. In another embodiment, the composition is administered twice a day. In still another embodiment, the composition is administered three times a day or four times a day. In a further embodiment, the composition is administered at least once a day for at least 1, 2, 3, 4, 5, 6, 7, 8, 9, 10, 11 or 12 weeks. In still a further embodiment, the composition is administered at least once a day for a longer term such as at least 4, 6, 8, 10, 12 or 24 months. Administration in some embodiments includes but is not limited to a dosage of 10-50 mg of composition at a frequency of minimum 1, 2, 3 or 4 times per day. Optionally, administration continues until all symptoms are resolved and cleared by medical personnel via standardized testing such as SCAT 2.

In some embodiments, the composition is administered at least once a day until the condition has ameliorated to where further treatment is not necessary. In another embodiment, the composition is administered until all symptoms of Duchenne's Muscular Dystrophy are resolved. In further embodiments, the composition is administered for at least 1, 2, 3, 6, 8, 10 or 12 or 24 months after the subject is asymptomatic.

The compositions of the present disclosure are useful and effective when administered to treat Duchenne's Muscular Dystrophy. The amount of each component present in the composition will be the amount that is therapeutically effective, i.e., an amount that will result in the effective treatment of the condition (e.g., Duchenne's Muscular Dystrophy) when administered. The therapeutically effective amount will vary depending on the subject and the severity and nature of the injury and can be determined routinely by one of ordinary skill in the art.

In some embodiments, the disclosure relates to a method of treating or preventing any of the indications set forth in US Pat. Nos. 8,222,211, 7,723,289, 7,723,290, 8,454,967, 9,097,725, (each of which are incorporated by reference in their entireties) comprising administering compositions or pharmaceutical compositions comprising any one or plurality of PIF peptides, analogs, or pharmaceutically acceptable salts thereof disclosed herein.

In some methods, the disclosure relates to a method of stimulating the differentiation and/or proliferation of stem cells in a subject in need thereof comprising administering compositions or pharmaceutical compositions comprising any one or plurality of PIF peptides, analogs, or pharmaceutically acceptable salts thereof disclosed herein.

In some embodiments, the disclosure relates to any of the methods disclosed in US Pat. Nos. 7,273,708, 7,695,977, 7,670,852, 7,670,851, 7,678,582, 7,670,850, 8,012,700 (each of which are incorporated by reference in their entireties) comprising administering compositions or pharmaceutical compositions comprising any one or plurality of PIF peptides, analogs, or pharmaceutically acceptable salts thereof disclosed herein.

This disclosure also incorporates by reference in their entireties US Pat. Nos. 7,789,289, 7,723,290, 8,222,211, and 8,454,967.

In some embodiments, the disclosure relates to a method of treating dystrophy-related disorder in a subject by administering at least one or a plurality of compositions disclosed herein comprising PIF peptide, an analog thereof, or a pharmaceutically acceptable salt thereof to the subject.

In some embodiments, the disclosure relates to a method of treating dystrophy-related disorder in a subject by administering a therapeutically effective amount or dose of one or a plurality of compositions disclosed herein comprising at least one PIF peptide, an analog thereof, or a pharmaceutically acceptable salt thereof to the subject.

In some embodiments, the disclosure relates to a method of treating dystrophy-related disorder by administering at least one or a plurality of compositions disclosed herein comprising PIF peptide, an analog thereof, or a pharmaceutically acceptable salt thereof to a subject in need thereof.

In some embodiments, the disclosure relates to a method of treating dystrophy-related disorder by administering a therapeutically effective amount or dose of one or a plurality of compositions disclosed herein comprising at least one PIF peptide, an analog thereof, or a pharmaceutically acceptable salt thereof to a subject in need thereof.

In some embodiments, the disclosure relates to a method of treating Duchenne's Muscular Dystrophy by administering at least one or a plurality of compositions disclosed herein comprising PIF peptide, an analog thereof, or a pharmaceutically acceptable salt thereof.

In some embodiments, the disclosure relates to a method of preventing a dystrophy-related disorder by administering a therapeutically effective amount or dose of one or a plurality of compositions disclosed herein comprising at least one PIF peptide, an analog thereof, or a pharmaceutically acceptable salt thereof.

In some embodiments, the disclosure relates to a method of preventing a dystrophy-related disorder by administering a therapeutically effective amount or dose of one or a plurality of compositions disclosed herein comprising at least one PIF peptide, an analog thereof, or a pharmaceutically acceptable salt thereof to a subject.

In some embodiments, the disclosure relates to a method of preventing a dystrophy-related disorder by administering a therapeutically effective amount or dose of one or a plurality of compositions disclosed herein comprising at least one PIF peptide, an analog thereof, or a pharmaceutically acceptable salt thereof to a subject in need thereof.

In some embodiments, the disclosure relates to a method of preventing Duchenne's Muscular Dystrophy by administering a therapeutically effective amount or dose of one or a plurality of compositions disclosed herein comprising at least one PIF peptide, an analog thereof, or a pharmaceutically acceptable salt thereof.

In some embodiments, the disclosure relates to a method of preventing Duchenne's Muscular Dystrophy by administering a therapeutically effective amount or dose of one or a plurality of compositions disclosed herein comprising at least one PIF peptide, an analog thereof, or a pharmaceutically acceptable salt thereof to a subject.

In some embodiments, the disclosure relates to a method of preventing Duchenne's Muscular Dystrophy by administering a therapeutically effective amount or dose of one or a plurality of compositions disclosed herein comprising at least one PIF peptide, an analog thereof, or a pharmaceutically acceptable salt thereof to a subject in need thereof.

In some embodiments, the disclosure relates to a method of treating Duchenne's Muscular Dystrophy in a subject in need thereof by administration to the subject of a pharmaceutical composition comprising a therapeutically effective amount or dose of at least: (i) one PIF peptide, an analog thereof, or a pharmaceutically acceptable salt thereof; and (ii) a pharmaceutically acceptable carrier.

In some embodiments, the disclosure relates to a pharmaceutical composition comprising a therapeutically effective amount or dose of at least one PIF peptide, an analog thereof, or a pharmaceutically acceptable salt thereof, and a pharmaceutically acceptable carrier for the treatment of a dystrophy-related disorder, such as Duchenne's Muscular Dystrophy, in a subject.

In some embodiments, the disclosure relates to the use of a therapeutically effective amount or dose of any one or plurality of compositions disclosed herein comprising at least one PIF peptide, an analog thereof, or a pharmaceutically acceptable salt thereof, and a pharmaceutically acceptable carrier in the manufacture of a medicament for the treatment of Duchenne's Muscular Dystrophy.

In some embodiments, the disclosure relates to the use of a pharmaceutical composition comprising a therapeutically effective amount or dose at least one PIF peptide, an analog thereof, or a pharmaceutically acceptable salt thereof, and a pharmaceutically acceptable carrier in the manufacture of a medicament for the Duchenne's Muscular Dystrophy.

In some embodiments, the disclosure relates to a method of inducing an immunomodulation effect in a subject in need thereof, when subject has been or is suspected of having Duchenne's Muscular Dystrophy.

In some embodiments, the disclosure relates to a method of treating fibrosis by administering at least one or a plurality of compositions disclosed herein comprising PIF peptide, an analog thereof, or a pharmaceutically acceptable salt thereof to a subject in need thereof.

In some embodiments, the disclosure relates to a method of treating fibrosis by administering a therapeutically effective amount or dose of one or a plurality of compositions disclosed herein comprising at least one PIF peptide, an analog thereof, or a pharmaceutically acceptable salt thereof to a subject in need thereof.

In some embodiments, the disclosure relates to a method of preventing fibrosis by administering at least one or a plurality of compositions disclosed herein comprising PIF peptide, an analog thereof, or a pharmaceutically acceptable salt thereof to a subject in need thereof.

In some embodiments, the disclosure relates to a method of preventing fibrosis by administering a therapeutically effective amount or dose of one or a plurality of compositions disclosed herein comprising at least one PIF peptide, an analog thereof, or a pharmaceutically acceptable salt thereof to a subject in need thereof.

In some embodiments, methods of treating fibrosis result in a reduction of fibrosis as compared to the amount of fibrosis in the subject as measured before administration of any of the disclosed compositions. In some embodiments, fibrosis may be measured by muscle motility, muscle strength, muscle tone, and/or muscle stability. In some embodiments, fibrosis may be measured by muscle electrical activity, such as by electromyography. In some embodiments, fibrosis may be measured using a Grade of fibrosis scale based on adverse effects, such as NCI-CTCAE-V4, shown in Appendix 1.

In some embodiments, the methods disclosed herein will result in about 5% reduction in fibrosis, about 10% reduction in fibrosis, about 20% reduction in fibrosis, about 30% reduction in fibrosis, about 40% reduction in fibrosis, about 50% reduction in fibrosis, about 60% reduction in fibrosis, about 70% reduction in fibrosis, about 80% reduction in fibrosis, about 90% reduction in fibrosis, about 95% reduction in fibrosis, or about 100% reduction in fibrosis as compared to the amount of fibrosis in the subject as measured before administration of any of the disclosed compositions.

In some embodiments, the methods comprise methods of improving muscle weakness in a subject comprising administering to the subject in need thereof, a pharmaceutical composition comprising a pharmaceutically effective amount of a PIF peptide, salt thereof or peptidomimetic thereof and a pharmaceutically acceptable carrier. In some embodiments, the muscle weakness is improved by at least about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, or about 30% or more as compared to the score of muscle weakness of the subject measured before treatment was administered. In some embodiments, muscle weakness is measured by the Oxford Scale and/or the ambulatory index (AI).

### Muscle strength grading scale (Oxford Scale)

| Grade | Description |
|---|---|
| 0/5 | No contraction |
| 1/5 | Visible/palpable muscle contraction but no movement |
| 2/5 | Movement with gravity eliminated |
| 3/5 | Movement against gravity only |
| 4/5 | Movement against gravity with some resistance |
| 5/5 | Movement against gravity with full resistence |

An adaptation of Modified Oxford Grading System may be used to evaluate the strength of the muscles by using manipulation or palpation. This scale is divided in fifteen categories, as follow:
0= "0": No active muscular contraction
1="1-"
2="1": Very slight muscular contraction
3="1+":
4="2-"
5="2": Full-motion overcome the force of gravity
6="2+"
7="3-"
8="3": Full-motion against gravity
9="3+"
10="4-"
11="4": Full-motion against slight resistance
12="4+"
13="5-"
14="5": Full-motion against strong resistance
15="5+"

The AI is a rating scale developed by Hauser et al (1983) to assess mobility by evaluating the time and degree of assistance required to walk 25 feet. Scores range from 0 (asymptomatic and fully active) to 10 (bedridden). The patient is asked to walk a marked 25-foot course as quickly and safely as possible. The examiner records the time and type of assistance (e.g., cane, walker, crutches) needed.

Administration time will vary depending upon the ability of the patient. Total administration time should be approximately 1-5 minutes. The AI is administered in person by a trained examiner. The examiner need not be a physician or nurse.

### Scoring

Although the patient's walking is timed, the time is not used directly but is utilized in conjunction with other factors to rate the patient on an ordinal scale with 11 gradations.

### Hauser Ambulation Index

0 = Asymptomatic; fully active.
1 = Walks normally, but reports fatigue that interferes with athletic or other demanding activities.
2 = Abnormal gait or episodic imbalance; gait disorder is noticed by family and friends; able to walk 25 feet (8 meters) in 10 seconds or less.
3 = Walks independently; able to walk 25 feet in 20 seconds or less.
4 = Requires unilateral support (cane or single crutch) to walk; walks 25 feet in 20 seconds or less.
5 = Requires bilateral support (canes, crutches, or walker) and walks 25 feet in 20 seconds or less; *or* requires unilateral support but needs more than 20 seconds to walk 25 feet.
6 = Requires bilateral support and more than 20 seconds to walk 25 feet; may use wheelchair^{∗} on occasion.
7 = Walking limited to several steps with bilateral support; unable to walk 25 feet; may use wheelchair^{∗} for most activities.
8 = Restricted to wheelchair; able to transfer self independently.
9 = Restricted to wheelchair; unable to transfer self independently.
   ^{∗}The use of a wheelchair may be determined by lifestyle and motivation. It is expected that patients in Grade 7 will use a wheelchair more frequently then those in Grades 5 or 6. Assignment of a grade in the range of 5 to 7, however, is determined by the patient's ability to walk a given distance, and not by the extent to which the patient uses a wheelchair.

### Kits

According to some embodiments of the invention, the formulation may be supplied as part of a kit. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:1. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:2. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:3. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:4. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:5. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:6. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:7. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:8. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:9. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:10. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:11. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:12. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:13. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:14. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:15. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:16. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:17. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:18. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:19. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:20. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:21. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:22. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:23. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:24. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:25. In some embodiments, the kit comprises comprising a

PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:26. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:27. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:28. In some embodiments, the kit comprises comprising a PIF peptide and/or a PIF analog or pharmaceutically acceptable salt thereof, the PIF peptide and/or a PIF analog or pharmaceutically acceptable salt thereof comprises a non-natural amino acid or is at least 70% homologous to SEQ ID NO:29. In another embodiment, the kit comprises a pharmaceutically acceptable salt of an analog with a rehydration mixture. In another embodiment, the pharmaceutically acceptable salt of an analog are in one container while the rehydration mixture is in a second container. The rehydration mixture may be supplied in dry form, to which water or other liquid solvent may be added to form a suspension or solution prior to administration. Rehydration mixtures are mixtures designed to solubilize a lyophilized, insoluble salt of the invention prior to administration of the composition to a subject takes at least one dose of a purgative. In another embodiment, the kit comprises a pharmaceutically acceptable salt in orally available pill form.

The kit may contain two or more containers, packs, or dispensers together with instructions for preparation and administration. In some embodiments, the kit comprises at least one container comprising the pharmaceutical composition or compositions described herein and a second container comprising a means for delivery of the compositions such as a syringe . In some embodiments, the kit comprises a composition comprising an analog in solution or lyophilized or dried and accompanied by a rehydration mixture. In some embodiments, the analog and rehydration mixture may be in one or more additional containers.

The compositions included in the kit may be supplied in containers of any sort such that the shelf-life of the different components are preserved, and are not adsorbed or altered by the materials of the container. For example, suitable containers include simple bottles that may be fabricated from glass, organic polymers, such as polycarbonate, polystyrene, polypropylene, polyethylene, ceramic, metal or any other material typically employed to hold reagents or food; envelopes, that may consist of foil-lined interiors, such as aluminum or an alloy. Other containers include test tubes, vials, flasks, and syringes. The containers may have two compartments that are separated by a readily removable membrane that upon removal permits the components of the compositions to mix. Removable membranes may be glass, plastic, rubber, or other inert material.

Kits may also be supplied with instructional materials. Instructions may be printed on paper or other substrates, and/or may be supplied as an electronic-readable medium, such as a floppy disc, CD-ROM, DVD-ROM, zip disc, videotape, audio tape, or other readable memory storage device. Detailed instructions may not be physically associated with the kit; instead, a user may be directed to an internet web site specified by the manufacturer or distributor of the kit, or supplied as electronic mail.

In another embodiment, a packaged kit is provided that contains the pharmaceutical formulation to be administered, i.e., a pharmaceutical formulation containing PIF peptide and/or a PIF analog or pharmaceutically acceptable salt thereof, a container (e.g., a vial, a bottle, a pouch, an envelope, a can, a tube, an atomizer, an aerosol can, etc.), optionally sealed, for housing the formulation during storage and prior to use, and instructions for carrying out drug administration in a manner effective to treat any one or more of the indications disclosed herein. The instructions will typically be written instructions on a package insert, a label, and/or on other components of the kit.

Depending on the type of formulation and the intended mode of administration, the kit may also include a device for administering the formulation (e.g., a transdermal delivery device). The administration device may be a dropper, a swab, a stick, or the nozzle or outlet of an atomizer or aerosol can. The formulation may be any suitable formulation as described herein. For example, the formulation may be an oral dosage form containing a unit dosage of the active agent, or a gel or ointment contained within a tube. The kit may contain multiple formulations of different dosages of the same agent. The kit may also contain multiple formulations of different active agents. The kit may contain a number of therapeutically effective dosages in separate containers or syringes necessary to treat one or more symptoms of a dystrophy-re;ated disorder. In some embodiments, the kit contains about 1, 2, 3, 4, or 5 or more dosages in 1, 2, 3, 4, or five containers (such as a syringe), that enable administration of any of the dosages into the subject in need thereof.

The present kits will also typically include means for packaging the individual kit components, i.e., the pharmaceutical dosage forms, the administration device (if included), and the written instructions for use. Such packaging means may take the form of a cardboard or paper box, a plastic or foil pouch, etc.

This disclosure and embodiments illustrating the method and materials used may be further understood by reference to the following non-limiting examples.

### EXAMPLES

### EXAMPLE 1

### Duchenne's Muscular Dystrophy

Prelmplantation factor (PIF), a 15-amino acid peptide secreted by viable mammalian embryos, is found in maternal circulation. Synthetic PIF (sPIF) is currently used in clinical trials in autoimmune hepatitis. Duchenne muscular dystrophy (DMD) is a progressive lethal, X-linked disease of skeletal and cardiac muscles caused by mutation of the dystrophin gene, which leads to muscle degeneration. Loss of function of dystrophin leads to damage and ultimately to muscle fibers waste and to the impairment of satellite cells to undergo asymmetric division, which is essential for muscle regeneration. Degenerating muscles accumulate connective tissue, a process commonly referred to as fibrosis.

Pre-Implantation Factor or PIF is a fifteen amino acid linear peptide secreted by viable human, bovine and murine embryos (Stamatkin et al 2011, Stamatkin et al 2011b, Barnea et al 2012, Ramu et al 2013). In singly cultured bovine and murine embryos increased levels of PIF in the media correlate with embryos development whereas PIF is absent in non-viable embryos (Stamatkin et al 2011). PIF plays an essential role in human pregnancy, as it primes the endometrium for implantation, promotes trophoblast invasion and regulates systemic immune response (Barnea et al 2012, Paidas et al 2010, Barnea et al 2012b, Duzyj et al 2010). Relevant to PIF's immune regulatory features, translational aspects to treatment of non-pregnant autoimmune and transplantation models were documented (Weiss et al 2011, Weiss et al 2012, Azar et al 2013). PIF also promotes trophoblast invasion, and orchestrates maternal systemic immune response (Barnea et al 2012, Duzyj et al, Roussev et al 2013). Pathway analysis in autoimmunity and transplantation models demonstrate that sPIF, administered as a single agent to non-pregnant mice, acts by reducing oxidative stress and protein misfolding (Weiss et al 2011, Weiss et al 2012, Paidas et al 2012, Azer et al 2013, Shainer et al 2013).

Tregs are derived from the CD4 lineage of T cells and are produced naturally in the thymus, express IL-10 receptor (CD25+) and the forkhaed box P3 transcription factor (Foxp3+). CD4+ T cells become Tregs by the Foxp3 expression induced by the cytokines increase in the microenvironment. Activated Tregs suppress the response of effector T cell indirectly by inhibiting the dendritic cells or the other antigen presenting cells (APC) from triggering effector T cell proliferation (Tang et al 2006). Tregs induce immune tolerance through the production of IL-10 and Transforming Growth Factor-β, anti-inflammatory cytokines or Th2, which inhibit T helper cell activation. (von Boemer et al 2006). The absence or depletion of Tregs leads to multi-systemic autoimmunity in mice and humans (Bruckner et al 2010). It has been reported that Tregs are critical for tumor growth, since they may provide an immunologically protected micro environment (Bergman et al 2007, Strauss et al 2007, Bergmann et al 20011, Wang et al 2012). The presence of Tregs in eutopic and ectopic endometrium of women affected by Duchenne's Muscular Dystrophy has been reported (Budiu et al 2009, Berbic et al 2010).

### Material and Methods

Synthetic PIF15 (MVRIKPGSANKPSDD) (SEQ ID NO: 13) and a scrambled peptide same amino acid sequence synthesized by solid-phase peptide synthesis (Peptide Synthesizer, Applied Biosystems) employing Fmoc (9-fluorenylmethoxycarbonyl) chemistry at Bio-Synthesis, Inc. (Lewisville, TX). Final purification was carried out by reversed-phase HPLC and identity was verified by matrix-assisted laser desorption/ionization time-of-flight mass spectrometry and amino acid analysis at >95% purity.

### Results

Our results so far documented that PIF treatment promoted differentiation of mouse myoblasts and human satellite cells as demonstrated by increases in MyoD and MyHC expression and a decrease in Pax-7 levels. PIF treatment also increased the myoblasts fusion into multinucleated myotubes. Additionally, PIF treatment increased levels of utrophin, a dystrophin protein homologue via downregulation of let-7. We further examined the therapeutic effects of PIF administration in DMD using mdx mice. PIF administration significantly decreased the serum levels of CK, a marker of muscle damage and the level of collagen type IV in the mdx mice diaphragms, indicating a significant decrease in muscle fibrosis. Considering the known anti-inflammatory effects of PIF, together with its current effects on cell regeneration and inhibition of muscle damage and fibrosis, we propose that PIF can be a potential therapeutic agent for the treatment of DMD.

Duchenne muscular dystrophy (DMD) is a common, genetic neuromuscular disease associated with the progressive deterioration of muscle function, X-linked recessive disorder that affects 1 in 3,500 males caused by mutations in the dystrophin gene which is largest known gene. The gene can be altered addition/deletion or even by point mutation which adds to the variability of manifestations of the disease. The vast majority of DMD patients lack the dystrophin protein. Becker muscular dystrophy is associated with a milder dystrophin protein defect. Genetic screening may improve early identification of carriers.

Preclinical studies use a variety of models (Allamand, V. & Campbell, K. P. Hum. Mol. Genet. 9, 2459-2467 (2000).) Although dogs may have the DMD phenotype they are not practical for studies. The mdx mouse is the most widely used model due to availability and ease to use. (Bulfield, G.et al. Proc. Natl. Acad. Sci. USA 81, 1189-1192 (1984).

A potential gene therapy that could initiated at birth by inserting the missing utrophin by overexpression in dmx mice which targets the sarcolema would help to restore dystrophin associated proteins. (Khurana and Davies, Nat Review Drug Dis. 2:379-390 (2003). Therefore attempts to increase utrophin endogenously expression by drug therapy could help to move the field forward. The limitations of current DMD therapy are the muscle although can differentiate usually is a terminal symmetric differentiation and therefore a limited reservoir is created. The second element is inflammation where the attempt to repair lead to fibrosis and severe and progressive muscular dysfunction. The third element is the neuromuscular junction where although early does not occur - later the disuse atrophy invariably affect the associated nerve conduction which further affects the individuals wellbeing. In that respect the data generated with PIF in an attempt to address the different DMD pathologies, both in vitro and in vivo

**Figure 1****: PIF promotes muscle differentiation**. C2C12 mouse myoblast are rapidly differentiating cells. To confirm, PIF effect was also tested on human satellite cells. The markers that are associated with muscle differentiation increase significantly in a dose dependent manner. They include MyHC- beta-myosin heavy chain binds actin and involved in kinetic energy transduction. MYOG- myogenin is an inducer of myogenesis. In addition the increase in MYOD- Myogen differentiating factor is a further enhancer of muscle differentiation. Importantly, it is also associated with muscle regeneration. Overall, the 3 proteins upregulated provide a strong supporting evidence that PIF enables muscle cells differentiation through a direct action. Additional action of PIF on these cells is the upregulation of the utrophin protein, this is an important current therapeutic target for gene delivery. Ability to increase utrophin that is present in the neuromuscular junction beyond the muscle, could also improve the neuro-muscular functionality. This increase also demonstrates that PIF promoted myotubules. (Westemblot)

**Figure 8****. PIF enhances the differentiation and fusion of mouse myoblasts.** PIF effect was examined on the ability of myoblasts to different and fuse in order to develop to muscle. Data showed by microphotographic evaluation that PIF effect is dose dependent. This was confirmed the increase in the fusion index percentage. The increase in mygenin levels documented that muscle differentiation has taken place. (Westemblot).

**Figure 2****. PIF downregulates let-7 and increases H19/ miR675 expression.** We reported that PIF in the brain in vivo murine model downregulates let-7 - leading to neuroprotection and reversing advanced hypoxic and ischemic damage. (Muller et al. PNAS 2014). Here we show that PIF in myoblasts lead to a similar decrease in let-7 expression which is coupled with reciprocal increase in H19 expression. Acting as a molecular sponge, H19 inhibits microRNA (miRNA) let-7. The implication of this finding goes beyond DMD since increased let-7/decreased H19 alters glucose metabolism in muscle of human subjects with type-2 diabetes and insulin resistant rodents. (Yuan Gao et al. Nucleic acids 42 13799, 2014). Further, to define the specific mechanism of action of PIF, the microRNA involved were investigated downstream of H19, showing increased expression of two variants of miR-675-3p and 5p. Knockdown of H19 RNA in myoblast cells and H19 knockout mouse satellite cells decreases differentiation. H19 exon1 encodes two miR-675-3p and miR-675-5p both are induced during skeletal muscle differentiation. The inhibition of myogenesis by H19 depletion during myoblast differentiation is rescued by exogenously added miR-675-3p and miR-675-5 (Dey BK, Genes Dev. 28:491-501 (2014)). This data documents that PIF acting through the myoblast differentiation pathway lead to muscle differentiation.

**Figure 3****. PIF effective in DMD model reduces CPK levels to normal.** The mdx C57BL/1 mouse model is induced by dystrophin nonsense mutation in exon 23. This leads to impaired asymmetric division of satellite cells leading to muscle exhaustion as no effective myoblast reservoir is maintained. The effect of PIF was tested in this model. PIF SC injection in PBS or intramuscular were administered twice daily for 2 weeks followed by 5 weeks observation without further therapy. The marker used for determining efficacy was measurement of CPK in the plasma of these mice. The data showed that by day 49, in the end of the experiment, the CPK levels where within normal limits. This provides a strong indication that the muscle was not affected. In contrast in control levels of CPK progressively increased until day 49 of the study.

**Figure 4****. PIF decreases tissue fibrosis in mdx mice.** Increased collagen expression due the inflammatory change leads to the development of muscle fibrosis. This is critical for the diaphragm that enables adequate expansion of the lungs to regulate breathing. We find that PIF based on collagen IV IHC confirmed by DAPI staining that the peptide reduced markedly this fibrosis marker. This was evidenced both following SQ and combined with intramuscular injection.

**Figure 5****. PIF increases utrophin the quadriceps of mdx mice**. We showed that in cultured myoblasts PIF promotes their differentiation evidenced also by the increase in utrophin levels. Here we confirm the same observation by IHC utrophin in the quadriceps muscle. Data shows that compared with control PIF increases the utrophin protein using also a DAPI staining. This was noted both with PIF SC and when combined with intramuscular injection.

**Figure 6****. PIF increases utrophin the diaphragm of mdx mice**. We showed that in cultured myoblasts PIF promotes their differentiation evidenced also by the increase in utrophin levels. Here we confirm the same observation by IHC utrophin in the diaphragm muscle. Data shows that compared with control PIF increases the utrophin protein using also a DAPI staining. This was noted both with PIF SC and when combined with intramuscular injection.

**Figure 7****. PIF potential therapeutic agent for the treatment of DMD.** This cartoon provides an integrated view of PIF's potential therapeutic role. From induction of myoblasts and satellite cells asymmetric differentiation to muscle to mechanistic insight decrease Let-7/increased H19 and downstream miR-675 3p/5p. Further the protective effect is confirmed in vivo in mdx model where PIF reduces CPK to that in normal levels. This is coupled by reduction in muscle fibrosis, and increased the critical utrophin in both quadriceps and diaphragm. (N=8/group)

Our results documented that PIF treatment promoted differentiation of mouse myoblasts and human satellite cells as demonstrated by increases in MyoD and MyHC expression and a decrease in Pax-7 levels. PIF treatment also increased the myoblasts fusion into multinucleated myotubes. Additionally, PIF treatment increased levels of utrophin, a dystrophin protein homologue via downregulation of let-7. We further examined the therapeutic effects of PIF administration in DMD using mdx mice. PIF administration significantly decreased the serum levels of CK, a marker of muscle damage and the level of collagen type IV in the mdx mice diaphragms, indicating a significant decrease in muscle fibrosis. Considering the known anti-inflammatory effects of PIF, together with its current effects on cell regeneration and inhibition of muscle damage and fibrosis, we propose that PIF can be a potential therapeutic agent for the treatment of DMD.

### Discussion

In this study we showed for the first time that PIF is effective in reversing Duchenne's Muscular Dystrophy lesions.

### EXAMPLE 2

### Treatment of Muscle Fibrosis and Muscle Differentiation

### Materials and methods for second series of experiments

### Materials

The following antibodies were employed: Myosin heavy chain (sc-376157), myoD (sc-760), Pax-7 (sc-81975), β-Actin HRP-conjugated antibody (sc-47778HRP), GAPDH (sc-26778) from Santa Cruz Biotechnology (Dallas, TX, USA); Collagen I (Ab21286), Collagen IV (ab19808) from Abcam, Cambridge, UK; utrophin (NCL-DRP2) from Leica Biosystems Inc. (Buffalo Grove, IL, USA); donkey anti-rabbit Cy-3-conjugated antibody (#711-166-152), donkey anti-mouse Cy-3-conjugated antibody (#715-165-151) from Jackson ImmunoResearch Laboratories, Inc. (West Grove, PA, USA); goat anti-mouse Alexa488-conjugated antibody (1:200, Molecular Probes Inc., Carlsbad, CA, USA, #A-11029), goat anti-rabbit HRP-conjugated antibody (#172-1019), goat anti-mouse HRP-conjugated antibody (#170-6516) from Bio-Rad Laboratories (Hercules, CA, USA); DAPI (D1306), and Red CMTPX Dye CellTracker (#C34552) from Thermo Fischer Scientific (Oregon City, OR, USA).

### Cultures of human satellite and C2C12 cells

Satellite cell preparations were consistently >90% pure, as indicated by the expression of Pax7 and alpha7-integrin and the absence of myogenin or myosin heavy chain (MyHC) expression. Isolated satellite cells were maintained on collagen-coated plates, and they continued to proliferate without differentiation when cultured in the presence of basic FGF (bFGF). Following the removal of bFGF and culture in low-serum medium (differentiation condition), satellite cells quickly exited the cell cycle and initiated myogenic differentiation that was confirmed by the increased expression of MyHC.

C2C12 myoblasts were maintained at subconfluent densities in DME supplemented with 10% FBS (Invitrogen, Paisley, UK). Myogenic differentiation was induced by changing subconfluent cells to DME containing 2% heat-inactivated horse serum.

### Transfection of miRNA mimics and shRNAs

RNA duplexes corresponding to miR-675 were obtained from by Sigma (St. Louis, MO). Transfection of the cells with the miRNA duplexes was carried out with siIMPORTER (Millipore) in 6-well plates according to the manufacturer's instructions. H19 shRNA was obtained from obtained from Dharmacon and miR-675 antagomiRs were obtained from SBI.

### Western blot analysis

Cells lysates (30 µg protein) were resolved by SDS-PAGE and transferred to nitrocellulose membranes as previously described. Following incubation with the primary and secondary antibodies the immunoreactive bands were visualized by the ECL Western blotting detection kit (Amersham, Arlington Heights, IL).

### Fusion analysis

C2C12 cells were plated in DMEM supplemented with 10% FBS, in a 35 mm imaging dish with an ibidi Polymer Coverslip Bottom at high density (80%) and allowed to proliferate for 48 hours. The medium was then replaced with a differentiation medium containing 2% horse serum, and the cells were cultured for an additional 10 days. sPIF (300 nM) was added to the culture medium at the beginning of the differentiation phase and every 72 hr thereafter.

The fusion index was quantified as the ratio of DAPI-stained nuclei in MyHC-positive multinucleated cells with ≥3 nuclei to total number of stained nuclei. Analysis was performed using ImageJ software of 49 fields in each of 2 independent culture dishes per group.

### Animal studies

All animal experiments were performed in accordance with the guidelines of the Israel Board for Animal Experiments and in compliance with The Israel Animal Welfare Act and Ethics Committee. The mdx mice (4-6 weeks males) were housed in cages under conditions of constant photoperiod (12/12 h light/dark) with free access to food and water. Mice were treated with sPIF, 0.75 mg/kg twice a week for two weeks either subcutaneously or by combination of subcutaneous and intramuscular injection into the Tibialis anterior (TA) muscle. The control mice were injected with saline. Two weeks later, tissues from the diaphragm, heart, QC and TA muscles were collected for histological and immunofluorescence analyses.

### Preparation of sections and immunofluorescence

Muscle biopsies were frozen in isopentane cooled with liquid nitrogen. Cryo-sections were prepared, fixed and blocked with 0.2% (w/v) gelatin and 0.2% (v/v) Tween20 (Sigma Aldrich, P1379) in PBS and immunostained with anti-utrophin antibody (1:50) alone, or double-immunostained with collagen type I (1:100) or collagen type IV (1:50) antibodies.

Cell nuclei were stained with DAPI (1:1000). As secondary antibodies goat anti-mouse IgG antibodies with Alexa Fluor dye were used for collagen type I detection and Cy3 anti-goat for utrophin detection. Microscope observations and image acquisition were performed with Leica SP8 Confocal and Zeiss Axioimager fluorescent microscopes and analyzed using CellProfiler software.

### Results

### sPIF increases the differentiation of human and mouse myoblasts

In this study we employed two in vitro systems: mouse C2C12 cells and human myoblasts derived from healthy donors and DMD patients. Lack of dystrophin has been reported to decrease the asymmetric division (Dumont et al 2015) and muscle differentiation of satellite cells (Keefe et al 2015). We first examined the effects of sPIF on myoblast differentiation and fusion. Myoblasts were cultured in a medium containing 2% horse serum for 10 days to allow cell differentiation. sPIF was added to the cells at the beginning of the culture and every two days thereafter. Treatment of C2C12 cells with sPIF significantly increased the expression of MyoD, MyHC and myogenin (Fig. 9A) and also enhanced cell fusion (Fig. 9B). Similarly, sPIF increased the expression of MyHC in myoblasts derived from both healthy donors and DMD patients (Fig. 9C). The internalization of sPIF in muscle cells was followed using a FITF-labeled sPIF and confocal microscopy. As presented in Figure 9D, sPIF entered the cells within 30-60 min of incubation and accumulated in the cytoplasm.

### sPIF increases myoblast differentiation via the induction of H19 and miR-675

To analyze the mechanisms involved in the effects of sPIF on muscle differentiation, we focused on the H19/miR-675 pathway. H19 plays an essential role in muscle differentiation and regeneration and its biological functions are mediated via miR-675-3p and miR-675-5p that are encoded by exon 1 of H19 (Dey et al 2014). Myoblasts derived from DMD patients showed significantly reduced expression of H19 and miR-675 in comparison to healthy donors (Figure 10A). Treatment of DMD myoblasts with sPIF significantly upregulated the expression of both H19 (Figure 10B) and miR-675 (Figure 10C) in these cells.

To demonstrate that the induction of miR-675 played a role in sPIF-induced myoblast differentiation we performed two experiments. First, we transfected DMD myoblasts with a siRNA duplex that targets H19 and showed that silencing of H19 decreased both miR-675 induction in the myoblasts and their differentiation by sPIF (Figure 10D). We next transfected the DMD-myoblasts with miR-675-3p and miR-675-3p mimics and found that the expression of MyHC was significantly increased after 5 days of treatment (Figure 10E). We also examined the effects of sPIF on the differentiation of myoblasts transfected with a miR-675 antagomiR and found the effects to be markedly abrogated, whereas, no significant decrease in sPIF effects was observed in myoblast transfected with a control antagomiR (Figure 10F). These results indicate that the induction by H19 and the subsequent induction of miR-675 by sPIF mediated the observed increased differentiation of the human myoblasts.

### sPIF increases utrophin expression via the H19/let-7 and miR-675/PTEN/AKT pathway

In addition to promoting muscle differentiation, sPIF treatment also increased the expression of utrophin in the mouse (Fig. 11A) and human muscle cells (Figs. 11A,B). The induction of utrophin in the human myoblasts was partly dependent on the induction of H19 since silencing of this IncRNA abrogated the increased utrophin expression (Fig. 11C). H19 has been reported to act as a sponge of let-7 (Kallen et al 2013) which targets the 3'-UTR of utrophin (Basu et al 2011; Mishra et al 2017), suggesting that the upregulation of H19 may interfere with the ability of let-7 to target utrophin. We then examined the effects of let-7 silencing on utrophin expression in DMD myoblasts and found an increased utrophin expression in let-7 silenced myoblasts (Figure 11D). Since sPIF was recently reported to downregulate let-7 expression in neural and lymphoid cells by decreasing its biogenesis, we examined the effects of sPIF on let-7 expression also in muscle cells. Treatment of human myoblasts with sPIF decreased the expression of let-7 (Fig. 11E), and overexpression of let-7 abrogated the upregulation of utrophin by sPIF (Fig. 11F). These results suggest that sPIF induced upregulation of utrophin expression via both directly decreasing let-7 expression and by inhibiting the targeting of utrophin by let-7 via H19 upregulation.

### sPIF decreases creatine kinase (CK) levels and muscle tissue fibrosis in mdx mice

We further studied the therapeutic effects of sPIF on mdx mice. sPIF was administered twice a day subcutaneous (0.75 mg/kg) for 2 weeks and mice were sacrificed 2 weeks later. We found that treatment of the mice with sPIF significantly decreased serum CK levels when examined 2 weeks post administration (Fig. 12A), suggesting that sPIF treatment exerts a therapeutic impact in this disease model.

Muscle fibrosis is one of the hallmark processes in DMD, reflecting muscle degeneration and correlating with patient's functional deterioration (Mann et al 2011; Li et al 2004). In mdx mice, fibrosis is mainly observed in the diaphragm and heart (Barbin et al 2016; Levi et al 2015). We therefore analyzed the effects of sPIF on fibrosis in these tissues. Mice treated with sPIF for 2 weeks were sacrificed 2 weeks later, and the diaphragm tissues were analyzed for collagen I and collagen IV expression using immunofluorescence staining. The expression of collagen I in the diaphragm of mice treated with sPIF was significantly lower as compared with tissues of control mice (Fig. 12B). In contrast, sPIF did not induce a significant decrease in collagen I or collagen IV expression in cardiac tissues (data not shown).

### sPIF increases utrophin expression in DMD muscle tissues

Recent studies reported inverse correlation of fibrosis and utrophin expression in both patient biopsies and mdx mice (Levi et al 2015). We found that sPIF increased utrophin expression in cultured cells and therefore examined the effects of sPIF on utrophin expression in mdx muscle. Indeed, two weeks of sPIF treatment significantly increased utrophin expression in the quadriceps (Fig. 13A), diaphragm (Fig. 13B) and cardiac (Fig. 13C) tissues of the mdx mice.

### Discussion

The results of this study demonstrate that sPIF promotes muscle differentiation in vitro and exerts a therapeutic impact in mdx mice by decreasing the levels of fibrosis and inducing utrophin expression. sPIF represents a therapeutic agent for the treatment of DMD and other dystrophin-related disorders.

### EXAMPLE 3

This study using sPIF a dose dependent design will evaluate safety and tolerability in patients with Duchenne's Muscular Dystrophy. The primary endpoint will be improved muscular activity. Secondary endpoints will include monitoring decreased levels of circulating CPK.

| **Arms** | **Assigned Interventions** |
|---|---|
| Active Comparator: SAD Normal sPIF | Drug: sPIF |
| Within each cohort (at least 3 patients/cohort; at least 9 subjects in total), patients with normal uterine function tests will be randomized in a 2:1 ratio (active drug : placebo) to receive a single dose of sPIF or placebo as follows: | Other Name: synthetic PreImplantation Factor in Ringer's lactate |
| Cohort 1: single dose 0.1 mg/kg sPIF Day 1 given subcutaneously (SQ) | |
| Cohort 2: single dose 0.5 mg/kg sPIF Day 1 given SQ | |
| Cohort 3: single dose 1.0 mg/kg sPIF Day 1 given SQ | |
| Placebo Comparator: SAD Normal Placebo | Drug: Placebo |
| Within each cohort (at least 3 patients/cohort; at least 9 subjects in total), patients with normal uterine function will be randomized in a 2:1 ratio (active drug : placebo) to receive a single dose of sPIF or placebo as follows: | Other Name: Ringer's lactate to mimic sPIF solution for injection |
| Cohort 1: single dose placebo Day 1 given SQ | |
| Cohort 2: single dose placebo Day 1 given SQ | |
| Cohort 3: single dose placebo Day 1 given SQ | |
| Active Comparator: SAD Abormal sPIF | Drug: sPIF |
| Within each cohort (at least 3 patients/cohort; at least 9 subjects in total), subjects with abnormal uterine function will be randomized in a 2:1 ratio (active drug : placebo) to receive a single dose of sPIF or placebo as follows: | Other Name: synthetic PreImplantation Factor |
| Cohort 1: single dose 0.1 mg/kg sPIF Day 1-5 given SQ | |
| Cohort 2: single dose 0.5 mg/kg sPIF Day 1-5 given SQ | |
| Cohort 3: single dose 1.0 mg/kg sPIF Day 1-5 given SQ | |
| Placebo Comparator: single ascending dose (SAD) Abnormal LFTs Placebo | Drug: Placebo |
| Within each cohort (at least 3 patients/cohort; at least 9 subjects in total), subjects with abnormal uterine function tests will be randomized in a 2:1 ratio (active drug : placebo) to receive a single dose of sPIF or placebo as follows: | Other Name: Ringer's lactate to mimic sPIF solution for injection |
| Cohort 1: single dose placebo Day 1-5 given SQ | |
| Cohort 2: single dose placebo Day 1-5 given SQ | |
| Cohort 3: single dose placebo Day 1-5 given SQ | |
| Active Comparator: multiple ascending dose (MAD) Normal sPIF | Drug: sPIF |
| Within each cohort ( at least 3 patients/cohort), subjects with normal uterine function tests will be randomized in a 2:1 ratio (active drug : placebo) to multiple doses of sPIF administered subcutaneously once a day for 5 consecutive days (Days 1 to 5): | Other Name: synthetic PreImplantation Factor |
| Cohort 1: 0.1 mg/kg sPIF Days 1-5 given SQ | |
| Cohort 2: 0.5 mg/kg sPIF Days 1-5 given SQ | |
| Cohort 3: 1.0 mg/kg sPIF Days 1-5 given SQ | |
| Placebo Comparator: MAD Normal Placebo | Drug: Placebo |
| Within each cohort (at least 3 patients/cohort), subjects with normal liver function tests will be randomized in a 2:1 ratio (active drug : placebo) to multiple doses of placebo administered subcutaneously once a day for 5 consecutive days (Days 1 to 5): | Other Name: Ringer's lactate to mimic sPIF solution for injection |
| Cohort 1: placebo Days 1-5 given SQ | |
| Cohort 2: placebo Days 1-5 given SQ | |
| Cohort 3: placebo Days 1-5 given SQ | |
| Active Comparator: MAD Abnormal sPIF | Drug: sPIF |
| Within each cohort (at least 3 patients/cohort), subjects with abnormal uterine function tests will be randomized in a 2:1 ratio (active drug : placebo) to multiple doses of sPIF administered subcutaneously once a day for 5 consecutive days (Days 1 to 5): | Other Name: synthetic PreImplantation Factor |
| Cohort 1: 0.1 mg/kg sPIF Days 1-5 given SQ | |
| Cohort 2: 0.5 mg/kg sPIF Days 1-5 given SQ | |
| Cohort 3: 1.0 mg/kg sPIF Days 1-5 given SQ | |
| Placebo Comparator: MAD Abnormal Placebo | Drug: Placebo |
| Within each cohort (at least 3 patients/cohort), subjects with abnormal uterine function tests will be randomized in a 2:1 ratio (active drug : placebo) to multiple doses of placebo administered subcutaneously once a day for 5 consecutive days (Days 1 to 5): | Other Name: Ringer's lactate to mimic sPIF solution for injection |
| Cohort 1: placebo Days 1-5 given SQ | |
| Cohort 2: placebo Days 1-5 given SQ | |
| Cohort 3: placebo Days 1-5 given SQ | |

After a sufficient observation period, the patients will be evaluated for improved muscular function. We expect that sPIF-treated cohorts will display improved muscle function and/or delayed onset of muscle degeneration. We also expect that the sPIF-treated cohorts will display fewer fibrotic lesions in muscle versus untreated patient populations.

### EXAMPLE 4

### Treatment of Fibrosis

The dystrophin gene mutation leads to muscle degeneration and impairment of satellite cells set to undergo asymmetric differentiation. sPIF promoted differentiation of mouse myoblasts and human satellite cell differentiation by increasing MyoD and MyHC expression and decreasing Pax-7 levels. sPIF treatment also increased the myoblasts fusion into multinucleated myotubes. Additionally, sPIF treatment increased levels of utrophin, a dystrophin protein homologue by downregulation microRNA et-7. sPIF administration for 2 weeks followed by 5 weeks post-therapy led to decreased serum CPK levels to baseline prime marker. Muscle analysis showed reduced collagen type IV in the diaphragm and quadriceps, reflecting decreased fibrosis formation. The data suggest that PIF peptides and salts thereof can reverse advanced fibrosis and, in some embodiments, fibrosis observed post-radiation.

### EXAMPLE 5

### sPIF protects against 50Gy chest wall radiation induced skin and cardiac fibrosis

Localized, high dose radiation lead to skin and surrounding organs fibrosis. Rats were exposed to the chest wall with high 50Gy radiation. sPIF was administered for 2 weeks, followed by twice weekly injections to week 20. sPIF reduced mortality, cardiac hypertrophy and fibrosis. In addition, sPIF normalized body weight, led to faster scar healing and hair growth on the site of irradiation. The daily activity of sPIF-treated rats was higher than the activity of the control rats. The observed increase in miR-21 expression may protect against the radiation induced injury. Thus, sPIF protects against long term induced fibrosis.

### EXAMPLE 6

### sPIF reverses GI tract mucosa damage following sublethal radiation damage

The oral-pharyngeal mucosa is highly vulnerable to radiation. Following sublethal radiation (6Gy), sPIF reversed colonic crypt and basal membrane damage, coupled with reduced nitric oxide synthetase (iNOS) and increased (B7H1) expression. Global upper GI gene pathway analysis revealed sPIF involvement in protein-RNA interactions, mitochondrial oxidative pathways, and responses to cellular stress. sPIF affects macrophage differentiation and function. sPIF protected irradiated macrophages and classically activated M1 macrophages, reducing inflammatory gene expression (iNOS, Cox2), promoting protective (Arg1) gene expression and inducing pro-tolerance cytokine secretion. sPIF administration at 48 hours post 6Gy sub-lethal radiation improved hematopoietic recovery and reduced systemic inflammatory cytokines level.

### EXAMPLE 7

### sPIF protects against lethal radiation followed by BMT

sPIF protects against semi-allogenic and totally allogenic bone marrow transplant (BMT) induced graft vs host disease where short term, low dose sPIF reduced mortality and prevented the development of GVHD for up to four months post-therapy (33, 32). It also reversed GVHD and promoted syngeneic bone marrow transplantation. The beneficial graft vs leukemia effect was maintained while GVHD was reduced. sPIF prevented development of liver inflammation as well as skin and colon ulceration, which leads to fibrosis. The reduced liver inflammation is due to reduced iNOS- oxidative stress, proinflammatory cytokines/chemokines and their receptor expression, coupled with reduced circulating IL-1a and IL-17 levels, prime proinflammatory cytokines. Thus, skin is protected by preventing fibrosis.

### EXAMPLE 8

### sPIF prevents surgery induced fibrosis

The primate model used with sPIF monotherapy led to successful allo-transplant of ovarian tissue long-term and demonstrated protection against fibrosis. This was evidenced by the total healing of the abdominal laparotomy scar with restored hair growth. Moreover, where the allograft was transplanted in the peritoneal cavity, a foreign object, there was no evidence of local inflammation and fibrosis development, even when observed at 7 months post therapy. Thus, this data substantiates the anti-fibrotic effect by sPIF.

### EXAMPLE 9

### High dose sPIF is safe in murine and canine model

The HED equivalents gave a safety factor of (21-32) and demonstrated that sPIF is cleared from the circulation within four hours, even at the highest dose administered 2 weeks post daily administration (4000 times above the therapeutic dose). This indicates that sPIF has a high safety margin. Despite the short circulating sPIF half-lifethe biological effect of this drug is dependent on binding to specific receptors which initiate and maintains action long term. This is evidenced up to 6 months post 2 weeks administration.

### EXAMPLE 10

### Single Ascending Dose sPIF in Autoimmune Hepatitis: Randomized, Double-Blind, Placebo-Controlled Study: NCT02239562

sPIF demonstrated safety and tolerability in AIH single ascending dose trial all enrolled patients. N=18 received (0.1, 0.5, 1 mg/kg in a 2:1 Rx/placebo ratio with normal or abnormal liver function). All completed the study and here were no clinically significant >1 adverse events. No drug to drug interaction observed, despite the concomitant use of steroids, immune suppressive agents. Ascending doses of sPIF produced a linear increase in the peptide serum levels associated with rapid <2 hours clearance. Exploratory analysis of 5/6 patients with abnormal liver function (ALT/AST) showed improvement and 2/6 cases led to normal levels of ALT up to 8 days. sPIF did not affect patients with normal liver function.

### Multiple Ascending Dose sPIF in Autoimmune Hepatitis: Randomized, Double-Blind, Placebo-Controlled Study

sPIF demonstrated safety and tolerability in AIH multiple ascending dose trial. N=18 patients dosed with increasing sPIF (0.1,0.5 and 1mg/kg) for 5 days 2:1 Rx/ placebo ratio with normal or abnormal liver function. All patients completed the study and there was no clinically significant grade 2, 3 or 4 adverse events. There was high safety up to day 29 with no drug-to-drug interaction and no antibody detection after sPIF administration was observed. sPIF, after 5 days, cleared rapidly from circulation within ~2hours. Exploratory analysis revealed improvement in (n=5/6 sPIF treated) one or more liver indices (ALT/AST/GGT/ALP) in those with abnormal liver function, with n=2/6 reaching normal ALT levels. sPIF did not affect patients with normal liver function. Serial cytokine analysis showed sPIF effect on IL8 consistent with liver disease. Phase 2 trials in liver/GI disease are being planned.

### EXAMPLE 11

### Phase II Clinical Trial

### Radiation induced injury head and neck disease manifestations.

In the past, the main role of radiation was treatment of tumors that could not be surgically removed. In other cases, it is used as a follow up treatment post-surgery, where the primary tumor is removed, and the surrounding metastasis are possibly treated. The aim of current radiotherapy is to focus only on the region being treated while avoiding radiation to the surrounding healthy tissues. Chemotherapy is used to increase the efficacy of radiation against the tumor. It can also be simultaneously used to treat distant metastasis. Single or combined therapies have short, medium and long-term negative consequences. The damage is due to the destruction of the endothelial cells that line the vessels. The sequence of events that follow the damage are ischemia, edema leading to inflammation and, ultimately, to fibrosis. These events can occur at various time points, ranging from months up to years later. The layers affected by radiation are skin, subcutaneous, fat, muscle, and glands, such as the parotid, thyroid and lymph nodes. Symptoms range from general soreness, open sores in the mouth or throat, dry mouth, trouble swallowing opening the mouth, changes in taste, nausea, earaches, tooth decay, swelling in the gums and throat, difficulty breathing, neck stiffness, hair loss, skin changes and jaw stiffness. Paralysis can develop following radiation of the nasopharyngeal region. Overall, the ultimate result is decreased functionality of all tissues in the head and neck region. Lymph node inflammation and possibly metastasis may develop. Since the cervical spine may be exposed to radiation, bone degeneration may develop. The result is fibrosis associated with local atrophy, which, even after several years, does not improve. Therefore, early diagnosis and intervention that would arrest the process of progressive fibrosis and associated dysfunction, would be the most beneficial.

Diagnostic methods are mostly carried out by imaging. The high-resolution ultrasound is used for fibrosis. CT and MRI tests are complementary since they also evaluate the muscle and the effect on bones. In addition, effects on the different glands such as parotid and thyroid can also be assessed. The use of CT scan and MRI is also used to evaluate possible tumor recurrence and metastasis (Amemiya et al 2005; Corvo 2007; Glastonbury et al 2010).

### Current Therapy

There are only two approved radioprotective compounds, Amifostine and Palifermin. Amifostine is a cytoprotectant, which helps reduce the damage to the salivary glands following radiotherapy. It also acts as an oxygen radical scavenger. It is administered together with radiation by IV infusion. It is associated with serious side effects including fatal skin reaction in xerostomia treatment (Kouvaria et al 2007). Palifermin, a recombinant human keratinocyte growth factor, helps to heal or prevent mouth sores and ulcers in people being treated with chemotherapy and stem cell treatment. The side effects of the drug are rash and pruritus, among others (Spielberger et al 2004). However, in a recent placebo controlled study on patients with head and neck cancer, the drug effect was similar to the placebo, although it reduced dysphagia (Le et al 2009).

Other experimental therapies with various success rates were also utilized. These include; Free radical scavengers/antioxidants, superoxide dismutase and superoxide dismutase mimetics, nitroxides and dietary antioxidants, cytokines/growth factors, angiotensin-converting enzyme inhibitors and apoptotic modulator all revealing limited efficacy. Immune suppressive agents may help locally but have serious systemic side effects. Overall, the improvement of the secondary effects of radiation on quality of life measures, resulting fibrosis and atrophy, remain an unmet need.

A comprehensive treatment regimen would achieve inflammation regulation, both locally and systemically, while providing immune regulation without suppression. Treatment would enable function restoration and wound healing, as well as empower/potentiate the immune system to better fight the cancer.

### Clinical Trial Synopsis

This is a Phase II open label, adaptive design, dose finding study in male and female patients with head and neck cancer. Recruited patients have fibrosis grade ≥2 and reduced quality of life (QOL) measures including, muscle stiffness, deglutition and saliva production after receiving radiotherapy or combined radio/chemotherapy while currently being under standard of care. The study will be initiated in a single center in France. Depending on enrollment, additional sites in France and abroad may be added.

### Dose Comparison Induction and Maintenance Phase

sPIF will be dosed at 0.5mg/kg or 1mg/kg daily for 14 days to N=7/group assessing safety and tolerability and clinical response. If well tolerated patients will transition to the Maintenance phase receiving their respective doses weekly up to 12 weeks evaluating effect on defined three QOL measures. At 12 weeks the primary objective is reducing fibrosis grade to 1 (30%) determined by ultrasound and post-therapy at 24 weeks. A cohort where >4/7 patients achieve the primary objective will constitute efficacy and enable enlarged cohort and increased injection interval up to 4 weeks. If needed, patients will transit to shorter dose intervals up to daily injections until >4/7 achieve therapeutic effect. If not achieved, dose escalation will ensue.

### Dose escalation Induction and Maintenance phase

sPIF will be dosed at the 1.5mg/kg starting dose daily for 14 days to N=7/group assessing safety and tolerability and clinical response. If well tolerated patients will transition to the Maintenance phase receiving the same weekly dose up to 12 weeks evaluating effect on three defined QOL measures. At 12 weeks effect on reducing fibrosis grade to 1 (30%) determined by ultrasound and post-therapy at 24 weeks will be assessed. A cohort where >4/7 patients constitute efficacy and enable enlarged cohort and increased injection interval up to 4 weeks. If needed, patients will transit to shorter dose intervals up to daily injections until >4/7 achieve therapeutic effect. If not achieved, further dose escalation in 0.5mg/kg increments will ensue.

In addition, anti-sPIF antibody, plasma sPIF levels, PBMC genes and serum cytokine levels will be determined. Overall, the goal is to establish effective induction/maintenance sPIF dosing to enlarge patients' cohort to substantiate both sPIF safety and efficacy in improving QOL measures and reduced fibrosis in addition to current standard of care.

### Study Objectives and Endpoints

Primary objectives are to determine efficacy of sPIF in reducing fibrosis of grade ≥ 2 to 1 that develops at 3-6 months post-radiotherapy (or radio/chemotherapy) of head and neck cancer leading to sustained improvement.

| **Primary Objective: Dose Comparison** | **Endpoint for primary objective** |
|---|---|
| • Determine effect of sPIF 0.5mg/kg and /1mg/kg | • Reduction in fibrosis score >2 to 1 by |
| dose on head and neck fibrosis injected up to 12 weeks. | 30% at 12 and 24 weeks |

| **Primary Objective: Dose Escalation** | **Endpoint(s) for primary objective(s)** |
|---|---|
| • Determine effect of 0.5mg/kg increments dosing on head and neck fibrosis injected up to 12 weeks. | • Reduction in fibrosis score >2 to 1 by 30% at 12 and 24 weeks |
| • Determine sPIF 0.5mg/kg dose increments which improve QOL measures administered up to 12 weeks | • Improved QOL from baseline stiffness/deglutition/saliva indices at 12 weeks |
| • Evaluate the safety and tolerability | • No SAE >3 observed |

### Population

The study population will consist of male and female patients aged 18 to 65 years old with fibrosis secondary to radiotherapy or combined radio and chemotherapy. Patients will be recruited from Bichat Hospital/ Hartmann Institute in Paris, France at the start of the study. Additional centers in the US and other countries may be added once effective dose is determined within a cohort. The goal is to screen/enroll a total of approximately 60 patients.

### Inclusion Criteria

- Grade 2 cutaneous or subcutaneous fibrosis (NCI-CTCAE v4 toxicity scale) induced by radiotherapy for head and neck cancer that is progressive for at least 3-6 months
- Neoplasms of the ENT sphere treated with radiotherapy +/- chemotherapy, in sustained remission
- Age ≥ 18 years
- Karnofsky PS> = 70

### Exclusion Criteria

- Long-term corticosteroid therapy
- Personal or family history of hereditary muscle disease
- Patient already included in another therapeutic trial with an experimental molecule,
- Pregnant or nursing women. Women of childbearing age must use a medically recognized contraceptive method during the treatment period and for 4 weeks following inclusion in the study
- Persons deprived of their liberty or under guardianship
- Inability to submit to the medical follow-up of the trial for geographical, social or psychological reasons.

### Treatment arms

The study is an open label adaptive design. Patients will be dosed at cohorts of (n =7) comparing 0.5mg/kg and 1mg /kg in the induction phase for 14 days, followed by the Maintenance phase evaluating efficacy by reduction in fibrosis at 12 weeks, and secondary objectives improved QOL by objective measures up to 12 weeks. The dose interval in the Maintenance phase can be increased or decreased until efficacy 4/7 patients improve the primary objective. If this is not reached, dose escalation will be pursued, increasing at 0.5mg/kg increments following the same Induction and Maintenance schedule and approach used in the comparative dosing study. Once an effective therapeutic response is shown in 4/7 per cohort, the study will be enlarged to recruit up to 60 patients and, using power analysis, the efficacy of the trial will be established.

Patients participating in the induction phase of the study (n = 5/cohort) will receive single daily doses for 14 days. If no efficacy is observed, the dose will be escalated for a period of 2 weeks.

### Head and neck fibrosis assessment

sPIF effect will be summarized by patient and dose level in each cohort using descriptive statistics and tabular summaries. This will be carried out at screening, prior to injection, and serially thereafter. The optimal sPIF induction/maintenance dose is aimed to be established with about 30% reduction in fibrosis documented at 12 weeks and maintained at 24 weeks. This is examined by using a Grade of fibrosis based on adverse effects NCI-CTCAE-V4 (**Appendix 1**) evaluation scale, and others shown below.

### APPENDIX 1.

### GRADE OF FIBROSIS

| Grade | Description |
|---|---|
| 1 | Mild induration, able to move skin parallel to plane (sliding) and perpendicular to skin (pinching up) |
| 2 | Moderate induration, able to slide skin, unable to pinch skin; limiting instrumental ADL |
| 3 | Severe induration; unable to slide or pinch skin; limiting joint or orifice movement (e.g. mouth, anus); limiting self care ADL |
| 4 | Generalized; associated with signs or symptoms of impaired breathing or feeding |
| 5 | Death |

### ADVERSE EFFECTS - SCHEDULE OF TIME & EVENTS: INDUCTION*

| | Screening | Pre-Dose | ^{∗}sPIF Dosing daily 14 days | | | | |
|---|---|---|---|---|---|---|---|
| | Day -28 | Day1 predose | Day1 | Day5 | Day 8 | Day 15 | Day 21 |
| Concomitant medication | X | X | | | X | x | X |
| Physical exam^{∗∗} | X | x^{∗∗} | | | x^{∗∗} | X^{∗∗} | x^{∗∗} |
| Vital signs | X | X | X | | X | x | X |
| Pregnancy test (Urine) if applicable | X | X | | | | | |
| CBC (with differential) | X | X | | | | | X |
| Serum chemistry | X | X | | | X | x | X |
| Liver function tests | X | X | | | X | x | X |
| Anti-sPIF antibody | x | | | | x | X | (84) |
| | X | | | | | | |
| PT (INR), PTT | X | | | | | | X |
| Quality of life questions | X | | | | | | X |
| Deglutition questions | X | X | | | | | X |
| Saliva index | X | X | | | | | |
| Urine analysis | X | | | | | | X |
| 12-Lead ECG | X | X | | | | | X |
| sPIF levels (plasma) | | | | | | | X |
| Cytokine, PBMC | | X | | | X | x | X |
| Chest X ray | X | | | | | | |
| Pharmacy dispensing^{∗} | | | X | X | X | x | |
| sPIF administration for consecutive 14 days | | | x | X | X | x | |
| Ultrasound for fibrosis | X | | | | | | |

| | | | | | | | |
|---|---|---|---|---|---|---|---|
| ^{∗}sPIF administration is for consecutive 14 days (**Induction phase**) ^{∗∗}As needed according to Adverse Effects assessment | | | | | | | |

Once that goal is achieved further recruitment of patients will be carried out to consolidate the numbers and reach statistical significance through power analysis. This primarily will be carried out at Bichat Hospital/Hartmann Institute but later other sites will also be involved to generate the additional supportive data. With respect of the statistical analysis the following will take place. Since it is an open label study where data can be analyzed in real time the proportion of patients that improve QOL/deglutition/saliva indices up to 12 weeks will be determined, at that point the effect on the fibrosis score will be analyzed-the primary efficacy criteria. All individual data will be listed as measured. All statistical summaries and analyses will be performed using SAS^{®} software (SAS Institute, Cary, North Carolina, USA). Binary/categorical outcome will be assessed by Chi-square tests appropriate to the table design to reach statistical significance, P<0.05. Namely how many patients out of the total have a sustained response at 12 weeks when the initial optimal dose is reached. At that point whether sPIF reduced head and neck fibrosis by 30% maximal thickness as determined by ultrasound at 12 and at 24 weeks post therapy will be determined. The data generated will be evaluated as follows. Out of 60 patients aimed to be recruited and evaluated found effective in the QOL/deglutition/saliva indices at 12 weeks the following assumption is made. As the study is designed the study will continue until > 4/7 patients will improve the fibrosis score - which will enable to increase the size of the cohort to reach statistical significance. The number of patients required is estimated to reach 60 to be able to carry out a power analysis. Until present there were no drop outs from the study in both SAD and MAD phases and all 36 patients once enrolled completed their scheduled medication and blood tests until 29 days. However, as in any study patients may not complete all tests, drop out or are lost for follow up. Our assumption that 80-90% of patients enrolled in the study will complete it. Therefore, if we assume that 60 patients will be required in total in the optimal dose cohort to be enrolled we plan to recruit 66-72 patients based on drop-out rate to enable power statistical analysis to be performed.

Although the present invention has been described in considerable detail with reference to certain preferred embodiments thereof, other versions are possible. Therefore the spirit and scope of the appended claims should not be limited to the description and the preferred versions contain within this specification. Any patent applications or other journal articles disclosed herein are incorporated by reference in their entireties.

### References

1. Stamatkin CW, Roussev RG, Stout M, Absalon-Medina V, Ramu S, Goodman C, Coulam CB, Gilbert RO, Godke RA, Barnea ER. PreImplantation Factor (PIF) correlates with early mammalian embryo development-bovine and murine models. Reprod Biol Endocrinol. 2011 May 15;9:63.
2. Stamatkin CW, Roussev RG, Stout M, Coulam CB, Triche E, Godke RA, Barnea ER. Preimplantation factor negates embryo toxicity and promotes embryo development in culture. Reprod Biomed Online. 2011b Oct;23(4):517-24.
3. Ramu S, Stamatkin C, Timms L, Ruble M, Roussev RG, Barnea ER. PreImplantation factor (PIF) detection in maternal circulation in early pregnancy correlates with live birth (bovine model). Reprod Biol Endocrinol. 2013 Nov 15;11:105
4. Barnea ER, Kirk D, Ramu S, Rivnay B, Roussev R, Paidas MJ. PreImplantation Factor (PIF) orchestrates systemic antiinflammatory response by immune cells: effect on peripheral blood mononuclear cells. Am J Obstet Gynecol. 2012, Oct;207(4):313.e1-11.
5. Barnea ER, Kirk D, Paidas MJ. Preimplantation factor (PIF) promoting role in embryo implantation: increases endometrial integrin-α2β3, amphiregulin and epiregulin while reducing betacellulin expression via MAPK in decidua. Reprod Biol Endocrinol. 2012b Jul 12;10:50.
6. Paidas MJ, Krikun G, Huang SJ, Jones R, Romano M, Annunziato J, Barnea ER. A genomic and proteomic investigation of the impact of preimplantation factor on human decidual cells. Am J Obstet Gynecol. 2010 May;202(5):459.
7. Duzyj CM, Barnea ER, Li M, Huang SJ, Krikun G, Paidas MJ. Preimplantation factor promotes first trimester trophoblast invasion. Am J Obstet Gynecol. 2010 Oct;203(4):402.
8. Weiss L, Bernstein S, et al. Preimplantation factor (PIF) analog prevents type I diabetes mellitus (TIDM) development by preserving pancreatic function in NOD mice. Endocrine. 2011;40:41-54.
9. Weiss L, Or R, et al. Preimplantation factor (PIF∗) reverses neuroinflammation while promoting neural repair in EAE model. J Neurol Sci. 2012;312:146-157.
10. Azar Y, Shainer R, Almogi-Hazan O, Bringer R, Compton SR, Paidas MJ, Barnea ER, Or R. Preimplantation factor reduces graft-versus-host disease by regulating immune response and lowering oxidative stress (murine model). Biol Blood Marrow Transplant. 2013 Apr;19(4):519-28
11. Roussev RG, Dons'koi BV, Stamatkin C, Ramu S, Chernyshov VP, Coulam CB, Barnea ER. Preimplantation factor inhibits circulating natural killer cell cytotoxicity and reduces CD69 expression: implications for recurrent pregnancy loss therapy. Reprod Biomed Online. 2013 Jan;26(1):79-87.
12. Paidas MJ, Annunziato J, Romano M, Weiss L, Or R, Barnea ER. Pregnancy and multiple sclerosis (MS): a beneficial association. Possible therapeutic application of embryo-specific pre-implantation factor (PIF∗). Am J Reprod Immunol. 2012 Dec;68(6):456-64.
13. Shainer R, Azar Y, et al. (2013) Immune Regulation and Oxidative Stress Reduction by Preimplantation Factor following Syngeneic or Allogeneic Bone Marrow Transplantation. Conference Papers in Medicine 2013: 1-8
14. Dumont, N. A. et al. Dystrophin expression in muscle stem cells regulates their polarity and asymmetric division. Nat. Med. (2015). doi:10.1038/nm.3990
15. Keefe, A. C. & Kardon, G. A new role for dystrophin in muscle stem cells. Nat. Med. 21, 1391-1393 (2015).
16. Dey, B. K., Pfeifer, K. & Dutta, A. The H19 long noncoding RNA gives rise to microRNAs miR-675-3p and miR-675-5p to promote skeletal muscle differentiation and regeneration. Genes Dev. (2014). doi:10.1101/gad.234419.113
17. Kallen, A. N. et al. The Imprinted H19 LncRNA Antagonizes Let-7 MicroRNAs. Mol. Cell (2013). doi:10.1016/j.molcel.2013.08.027
18. Basu, U. et al. Translational regulation of utrophin by miRNAs. PLoS One (2011). doi:10.1371/journal.pone.0029376
19. Mishra, M. K., Loro, E., Sengupta, K., Wilton, S. D. & Khurana, T. S. Functional improvement of dystrophic muscle by repression of utrophin: Let-7c interaction. PLoS One (2017). doi:10.1371/journal.pone.0182676
20. Mann, C. J. et al. Aberrant repair and fibrosis development in skeletal muscle. Skelet. Muscle 1, 21 (2011).
21. Li, Y. et al. Transforming growth factor-betal induces the differentiation of myogenic cells into fibrotic cells in injured skeletal muscle: a key event in muscle fibrogenesis. Am. J. Pathol. 164, 1007-19 (2004).
22. Barbin, I. C. C. et al. Diaphragm degeneration and cardiac structure in mdx mouse: potential clinical implications for Duchenne muscular dystrophy. J. Anat. 228, 784-791 (2016).
23. Levi, O. et al. Inhibition of muscle fibrosis results in increases in both utrophin levels and the number of revertant myofibers in Duchenne muscular dystrophy. Oncotarget 6, 23249-23260 (2015).
24. Amemiya K, Shibuya H, Yoshimura R, Okada N. The risk of radiation-induced cancer in patients with squamous cell carcinoma of the head and neck and its results of treatment. Br J Radiol. 2005;78(935):1028-33.
25. Corvo R. Evidence-based radiation oncology in head and neck squamous cell carcinoma. Radiother Oncol. 2007;85(1):156-70.
26. Glastonbury CM, Parker EE, Hoang JK. The postradiation neck: evaluating response to treatment and recognizing complications. AJR Am J Roentgenol. 2010;195(2):W164-71.
27. Kouvaris JR, Kouloulias VE, Vlahos LJ. Amifostine: the first selective-target and broad-spectrum radioprotector. Oncologist. 2007;12(6):738-47.
28. Spielberger R, Stiff P, Bensinger W, Gentile T, Weisdorf D, Kewalramani T, et al. Palifermin for oral mucositis after intensive therapy for hematologic cancers. N Engl J Med. 2004;351(25):2590-8.
29. Le QT, Kim HE, Schneider CJ, Murakozy G, Skladowski K, Reinisch S, et al. Palifermin reduces severe mucositis in definitive chemoradiotherapy of locally advanced head and neck cancer: a randomized, placebo-controlled study. J Clin Oncol. 2011;29(20):2808-14.

Aspects and features of the present disclosure are set out in the following numbered clauses which contain the subject matter of the claims of the parent application as filed.
1. A method of treating or preventing a dystrophin-related disorder in a subject in need thereof, the method comprising administering to the subject at least one pharmaceutical composition comprising:
   a therapeutically effective amount of a pre-implantation factor (PIF) peptide, mimetics thereof, analogs thereof, or a pharmaceutically acceptable salt thereof; and
   a pharmaceutically acceptable carrier.
2. The method of clause 1, wherein the pharmaceutically acceptable carrier is sterile and pyrogen-free water or sterile and pyrogen-free Lactated ringer's solution.
3. The method of clause 1, wherein the therapeutically effective dose is about 1.0 mg/kg, wherein kg is kilograms of the subject and mg is milligrams of the therapeutically effective dose.
4. The method of clause 1, wherein the therapeutically effective dose is about 2.0 mg/kg, wherein kg is kilograms of the subject and mg is milligrams of the therapeutically effective dose.
5. The method of clause 1, wherein the therapeutically effective dose is about 3.0 mg/kg, wherein kg is kilograms of the subject and mg is milligrams of the therapeutically effective dose.
6. The method of clause 1, wherein the therapeutically effective dose is about 4.0 mg/kg, wherein kg is kilograms of the subject and mg is milligrams of the therapeutically effective dose.
7. The method of clause 1, wherein the therapeutically effective dose is about 0.2 mg/kg, wherein kg is kilograms of the subject and mg is milligrams of the therapeutically effective dose.
8. The method of clause 1, wherein the therapeutically effective dose is about 0.3 mg/kg, wherein kg is kilograms of the subject and mg is milligrams of the therapeutically effective dose.
9. The method of clause 1, wherein the therapeutically effective dose is about 0.4 mg/kg, wherein kg is kilograms of the subject and mg is milligrams of the therapeutically effective dose.
10. The method of clause 1, wherein the therapeutically effective dose is about 0.5 mg/kg, wherein kg is kilograms of the subject and mg is milligrams of the therapeutically effective dose.
12. The method of clause 1, wherein the therapeutically effective dose is about 0.6 mg/kg, wherein kg is kilograms of the subject and mg is milligrams of the therapeutically effective dose.
13. The method of clause 1, wherein the therapeutically effective dose is about 0.7 mg/kg, wherein kg is kilograms of the subject and mg is milligrams of the therapeutically effective dose.
14. The method of clause 1, wherein the therapeutically effective dose is about 0.8 mg/kg, wherein kg is kilograms of the subject and mg is milligrams of the therapeutically effective dose.
15. The method of clause 1, wherein the step of administering to the subject at least one PIF peptide, or a mimetic, analog, or pharmaceutically acceptable salt thereof comprises administering a therapeutically effective dose of the at least one PIF peptide thereof, or a mimetic, analog, or pharmaceutically acceptable salt thereof.
16. The method of clause 1, the PIF peptide is one or a combination of any one or plurality of: SEQ ID NO:1, SEQ ID NO:2, SEQ ID NO:3, SEQ ID NO:4, SEQ ID NO:5, SEQ ID NO:6 SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, and/or SEQ ID NO:29, or pharmaceutically acceptable salt thereof.
17. The method of clause 1, the PIF peptide is SEQ ID NO:1 or a pharmaceutically acceptable salt thereof.
18. The method of clause 1, the PIF peptide is SEQ ID NO:2 or a pharmaceutically acceptable salt thereof.
19. The method of clause 1, the PIF peptide is SEQ ID NO:3 or a pharmaceutically acceptable salt thereof.
20. The method of clause 1, the PIF peptide is SEQ ID NO:4 or a pharmaceutically acceptable salt thereof.
21. The method of clause 1, the PIF peptide is SEQ ID NO:5 or a pharmaceutically acceptable salt thereof.
22. The method of clause 1, the PIF peptide is SEQ ID NO:6 or a pharmaceutically acceptable salt thereof.
23. The method of clause 1, the PIF peptide is SEQ ID NO:7 or a pharmaceutically acceptable salt thereof.
24. The method of clause 1, the PIF peptide is SEQ ID NO:8 or a pharmaceutically acceptable salt thereof.
25. The method of clause 1, the PIF peptide is SEQ ID NO:9 or a pharmaceutically acceptable salt thereof.
26. The method of clause 1, the PIF peptide is SEQ ID NO: 10 or a pharmaceutically acceptable salt thereof.
27. The method of clause 1, the PIF peptide is SEQ ID NO:11 or a pharmaceutically acceptable salt thereof.
28. The method of clause 1, the PIF peptide is SEQ ID NO:12 or a pharmaceutically acceptable salt thereof.
29. The method of clause 1, the PIF peptide is SEQ ID NO:13 or a pharmaceutically acceptable salt thereof.
30. The method of clause 1, the PIF peptide is SEQ ID NO:14 or a pharmaceutically acceptable salt thereof.
31. The method of clause 1, the PIF peptide is SEQ ID NO:15 or a pharmaceutically acceptable salt thereof.
32. The method of clause 1, the PIF peptide is SEQ ID NO:16 or a pharmaceutically acceptable salt thereof.
33. The method of clause 1, the PIF peptide is SEQ ID NO:17 or a pharmaceutically acceptable salt thereof.
34. The method of clause 1, the PIF peptide is SEQ ID NO:18 or a pharmaceutically acceptable salt thereof.
35. The method of clause 1, the PIF peptide is SEQ ID NO:19 or a pharmaceutically acceptable salt thereof.
36. The method of clause 1, the PIF peptide is SEQ ID NO:20 or a pharmaceutically acceptable salt thereof.
37. The method of clause 1, the PIF peptide is SEQ ID NO:21 or a pharmaceutically acceptable salt thereof.
38. The method of clause 1, the PIF peptide is SEQ ID NO:22 or a pharmaceutically acceptable salt thereof.
39. The method of clause 1, the PIF peptide is SEQ ID NO:23 or a pharmaceutically acceptable salt thereof.
40. The method of clause 1, the PIF peptide is SEQ ID NO:24 or a pharmaceutically acceptable salt thereof.
41. The method of clause 1, the PIF peptide is SEQ ID NO:25 or a pharmaceutically acceptable salt thereof.
42. The method of clause 1, the PIF peptide is SEQ ID NO:26 or a pharmaceutically acceptable salt thereof.
43. The method of clause 1, the PIF peptide is SEQ ID NO:27 or a pharmaceutically acceptable salt thereof.
44. The method of clause 1, the PIF peptide is SEQ ID NO:28 or a pharmaceutically acceptable salt thereof.
45. The method of clause 1, the PIF peptide is SEQ ID NO:29 or a pharmaceutically acceptable salt thereof.
46. The method of clause 1, wherein the step of administering to the subject at least one PIF peptide, or mimetic, analog or pharmaceutically acceptable salt thereof comprises administering a therapeutically effective dose of the PIF peptide, or mimetic, analog or pharmaceutically acceptable salt thereof from about 0.001 mg/kg to about 200 mg/kg.
47. The method of clause 1, wherein the step of administering to the subject at least one PIF peptide, or mimetic, analog or pharmaceutically acceptable salt thereof, comprises administering a therapeutically effective dose of the PIF peptide, or mimetic, analog or pharmaceutically acceptable salt thereof from about 0.5 mg/kg to about 5 mg/kg.
48. The method of any of clauses 1 - 47, wherein the at least the PIF peptide, or mimetic, analog or pharmaceutically acceptable salt thereof comprises a chemical targeting moiety and/or a radioactive moiety.
49. The method of clause 48, wherein the at least one PIF peptide, or mimetic, analog or pharmaceutically acceptable salt thereof comprises at least one radioactive moiety comprising at least one or a combination of the following isotopes: ²H, ³H, ¹³C, ¹⁴C, ¹⁵N, ¹⁶O, ¹⁷O, ³¹P, ³²P, ³⁵S, ¹⁸F, and ³⁶Cl.
50. The method of any of clauses 1-49, wherein the method further comprises administering at least one analgesic and/or one anti-inflammatory compound.
51. The method of clause 50, wherein the method further comprises administering at least one analgesic and or one anti-inflammatory compound before, after, or simultaneously with the administration of a therapeutically effective dose of at least one PIF peptide, or mimetic, analog or pharmaceutically acceptable salt thereof.
52. The method of any of clauses 1 - 51, wherein the pharmaceutical composition further comprises a therapeutically effective dose of one or a plurality of active agents.
53. The method of clause 52, wherein the one or plurality of active agents is one or a combination of compounds chosen from: an anti-inflammatory compound, alpha-adrenergic agonist, antiarrhythmic compound, analgesic compound, and an anesthetic compound.
54. The method of any of clauses 1 - 53, wherein the pharmaceutical composition is free of SEQ ID NO:1 or a pharmaceutically acceptable salt thereof.
55. A method of improving the clinical outcome in a subject suffering with, diagnosed with or suspected of having a dystrophin-related disorder comprising administering to the subject at least one pharmaceutical composition comprising:
   a therapeutically effective amount of a pre-implantation factor (PIF) peptide, an analog thereof, or a pharmaceutically acceptable salt thereof; and
   a pharmaceutically acceptable carrier.
56. The method of any of clauses 1 - 55, wherein the pharmaceutical composition is administered via parenteral injection, subcutaneous injection, intravenous injection, intramuscular injection, intraperitoneal injection, transdermally, orally, buccally, ocular routes, intravaginally, by inhalation, by depot injections, or by implants.
57. The method of any of clauses 1 - 55, wherein the pharmaceutical composition further comprises one or combination of active agents is selected from Table Y.
58. The method of either of clause 1 or 55, wherein the method further comprises administration of one or a plurality of compositions comprising active agents selected from Table Y sequentially or comtemporaneously.
59. A method administering to the subject at least one pharmaceutical composition comprising a therapeutically effective amount of a PIF peptide, mimetics thereof, analogs thereof, or a pharmaceutically acceptable salt thereof and a pharmaceutically acceptable carrier.
60. A method of treating or preventing muscle degeneration in a subject in need thereof, the method comprising administering to the subject at least one pharmaceutical composition comprising:
   a therapeutically effective amount of a pre-implantation factor (PIF) peptide, mimetics thereof, analogs thereof, or a pharmaceutically acceptable salt thereof; and
   a pharmaceutically acceptable carrier.
61. The method of clause 60, wherein the pharmaceutically acceptable carrier is sterile and pyrogen-free water or sterile and pyrogen-free Lactated ringer's solution.
62. The method of clause 60, wherein the therapeutically effective dose is about 1.0 mg/kg, wherein kg is kilograms of the subject and mg is milligrams of the therapeutically effective dose.
63. The method of clause 60, wherein the therapeutically effective dose is about 2.0 mg/kg, wherein kg is kilograms of the subject and mg is milligrams of the therapeutically effective dose.
64. The method of clause 60, wherein the therapeutically effective dose is about 3.0 mg/kg, wherein kg is kilograms of the subject and mg is milligrams of the therapeutically effective dose.
65. The method of clause 60, wherein the therapeutically effective dose is about 4.0 mg/kg, wherein kg is kilograms of the subject and mg is milligrams of the therapeutically effective dose.
66. The method of clause 60, wherein the therapeutically effective dose is about 0.2 mg/kg, wherein kg is kilograms of the subject and mg is milligrams of the therapeutically effective dose.
67. The method of clause 60, wherein the therapeutically effective dose is about 0.3 mg/kg, wherein kg is kilograms of the subject and mg is milligrams of the therapeutically effective dose.
68. The method of clause 60, wherein the therapeutically effective dose is about 0.4 mg/kg, wherein kg is kilograms of the subject and mg is milligrams of the therapeutically effective dose.
69. The method of clause 60, wherein the therapeutically effective dose is about 0.5 mg/kg, wherein kg is kilograms of the subject and mg is milligrams of the therapeutically effective dose.
70. The method of clause 60, wherein the therapeutically effective dose is about 0.6 mg/kg, wherein kg is kilograms of the subject and mg is milligrams of the therapeutically effective dose.
71. The method of clause 60, wherein the therapeutically effective dose is about 0.7 mg/kg, wherein kg is kilograms of the subject and mg is milligrams of the therapeutically effective dose.
72. The method of clause 60, wherein the therapeutically effective dose is about 0.8 mg/kg, wherein kg is kilograms of the subject and mg is milligrams of the therapeutically effective dose.
73. The method of clause 60, wherein the step of administering to the subject at least one PIF peptide, or a mimetic, analog, or pharmaceutically acceptable salt thereof comprises administering a therapeutically effective dose of the at least one PIF peptide thereof, or a mimetic, analog, or pharmaceutically acceptable salt thereof.
74. The method of clause 60, the PIF peptide is one or a combination of any one or plurality of: SEQ ID NO:1, SEQ ID NO:2, SEQ ID NO:3, SEQ ID NO:4, SEQ ID NO:5, SEQ ID NO:6 SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, and/or SEQ ID NO:29, or pharmaceutically acceptable salt thereof.
75. The method of clause 60, the PIF peptide is SEQ ID NO:1 or a pharmaceutically acceptable salt thereof.
76. The method of clause 60, the PIF peptide is SEQ ID NO:2 or a pharmaceutically acceptable salt thereof.
77. The method of clause 60, the PIF peptide is SEQ ID NO:3 or a pharmaceutically acceptable salt thereof.
78. The method of clause 60, the PIF peptide is SEQ ID NO:4 or a pharmaceutically acceptable salt thereof.
79. The method of clause 60, the PIF peptide is SEQ ID NO:5 or a pharmaceutically acceptable salt thereof.
80. The method of clause 60, the PIF peptide is SEQ ID NO:6 or a pharmaceutically acceptable salt thereof.
81. The method of clause 60, the PIF peptide is SEQ ID NO:7 or a pharmaceutically acceptable salt thereof.
82. The method of clause 60, the PIF peptide is SEQ ID NO:8 or a pharmaceutically acceptable salt thereof.
83. The method of clause 60, the PIF peptide is SEQ ID NO:9 or a pharmaceutically acceptable salt thereof.
84. The method of clause 60, the PIF peptide is SEQ ID NO: 10 or a pharmaceutically acceptable salt thereof.
85. The method of clause 60, the PIF peptide is SEQ ID NO:11 or a pharmaceutically acceptable salt thereof.
86. The method of clause 60, the PIF peptide is SEQ ID NO:12 or a pharmaceutically acceptable salt thereof.
87. The method of clause 60, the PIF peptide is SEQ ID NO: 13 or a pharmaceutically acceptable salt thereof.
88. The method of clause 60, the PIF peptide is SEQ ID NO:14 or a pharmaceutically acceptable salt thereof.
89. The method of clause 60, the PIF peptide is SEQ ID NO: 15 or a pharmaceutically acceptable salt thereof.
90. The method of clause 60, the PIF peptide is SEQ ID NO: 16 or a pharmaceutically acceptable salt thereof.
91. The method of clause 60, the PIF peptide is SEQ ID NO: 17 or a pharmaceutically acceptable salt thereof.
92. The method of clause 60, the PIF peptide is SEQ ID NO:18 or a pharmaceutically acceptable salt thereof.
93. The method of clause 60, the PIF peptide is SEQ ID NO: 19 or a pharmaceutically acceptable salt thereof.
94. The method of clause 60, the PIF peptide is SEQ ID NO:20 or a pharmaceutically acceptable salt thereof.
95. The method of clause 60, the PIF peptide is SEQ ID NO:21 or a pharmaceutically acceptable salt thereof.
96. The method of clause 60, the PIF peptide is SEQ ID NO:22 or a pharmaceutically acceptable salt thereof.
97. The method of clause 60, the PIF peptide is SEQ ID NO:23 or a pharmaceutically acceptable salt thereof.
98. The method of clause 60, the PIF peptide is SEQ ID NO:24 or a pharmaceutically acceptable salt thereof.
99. The method of clause 60, the PIF peptide is SEQ ID NO:25 or a pharmaceutically acceptable salt thereof.
100. The method of clause 60, the PIF peptide is SEQ ID NO:26 or a pharmaceutically acceptable salt thereof.
101. The method of clause 60, the PIF peptide is SEQ ID NO:27 or a pharmaceutically acceptable salt thereof.
102. The method of clause 60, the PIF peptide is SEQ ID NO:28 or a pharmaceutically acceptable salt thereof.
103. The method of clause 60, the PIF peptide is SEQ ID NO:29 or a pharmaceutically acceptable salt thereof.
104. The method of clause 60, wherein the step of administering to the subject at least one PIF peptide, or mimetic, analog or pharmaceutically acceptable salt thereof comprises administering a therapeutically effective dose of the PIF peptide, or mimetic, analog or pharmaceutically acceptable salt thereof from about 0.001 mg/kg to about 200 mg/kg.
105. The method of clause 60, wherein the step of administering to the subject at least one PIF peptide, or mimetic, analog or pharmaceutically acceptable salt thereof, comprises administering a therapeutically effective dose of the PIF peptide, or mimetic, analog or pharmaceutically acceptable salt thereof from about 0.5 mg/kg to about 5 mg/kg.
106. The method of any of clauses 1-105, wherein the subject is human.
107. A method of treating or preventing fibrosis in a subject in need thereof, the method comprising administering to the subject at least one pharmaceutical composition comprising:
   a therapeutically effective amount of a PIF peptide, mimetics thereof, analogs thereof, or a pharmaceutically acceptable salt thereof; and
   a pharmaceutically acceptable carrier.
108. The method of clause 107, wherein the fibrosis is muscle fibrosis.
109. The method of clause 107, the PIF peptide is one or a combination of any one or plurality of: SEQ ID NO:1, SEQ ID NO:2, SEQ ID NO:3, SEQ ID NO:4, SEQ ID NO:5, SEQ ID NO:6 SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, and/or SEQ ID NO:29, or pharmaceutically acceptable salt thereof.
110. The method of clause 107, wherein the step of administering to the subject at least one PIF peptide, or mimetic, analog or pharmaceutically acceptable salt thereof comprises administering a therapeutically effective dose of the PIF peptide, or mimetic, analog or pharmaceutically acceptable salt thereof from about 0.001 mg/kg to about 200 mg/kg.

## Claims

1. A pharmaceutical composition comprising:
a therapeutically effective amount of a pre-implantation factor (PIF) peptide, mimetics thereof, analogs thereof, or a pharmaceutically acceptable salt thereof; and
a pharmaceutically acceptable carrier for use in a method of treating or preventing a dystrophin-related disorder in a subject; and
wherein the PIF peptide is:
a) SEQ ID NO: 1 or a pharmaceutically acceptable salt thereof;
b) SEQ ID NO: 2 or a pharmaceutically acceptable salt thereof;
c) SEQ ID NO: 3 or a pharmaceutically acceptable salt thereof;
d) SEQ ID NO: 4 or a pharmaceutically acceptable salt thereof;
e) SEQ ID NO: 5 or a pharmaceutically acceptable salt thereof;
f) SEQ ID NO: 6 or a pharmaceutically acceptable salt thereof;
g) SEQ ID NO: 7 or a pharmaceutically acceptable salt thereof;
h) SEQ ID NO: 8 or a pharmaceutically acceptable salt thereof;
i) SEQ ID NO: 9 or a pharmaceutically acceptable salt thereof;
j) SEQ ID NO: 10 or a pharmaceutically acceptable salt thereof;
k) SEQ ID NO: 11 or a pharmaceutically acceptable salt thereof;
l) SEQ ID NO: 12 or a pharmaceutically acceptable salt thereof;
m) SEQ ID NO: 14 or a pharmaceutically acceptable salt thereof;
n) SEQ ID NO: 15 or a pharmaceutically acceptable salt thereof;
o) SEQ ID NO: 16 or a pharmaceutically acceptable salt thereof;
p) SEQ ID NO: 17 or a pharmaceutically acceptable salt thereof;
q) SEQ ID NO: 18 or a pharmaceutically acceptable salt thereof;
r) SEQ ID NO: 19 or a pharmaceutically acceptable salt thereof;
s) SEQ ID NO: 20 or a pharmaceutically acceptable salt thereof;
t) SEQ ID NO: 21 or a pharmaceutically acceptable salt thereof;
u) SEQ ID NO: 22 or a pharmaceutically acceptable salt thereof;
v) SEQ ID NO: 23 or a pharmaceutically acceptable salt thereof;
w) SEQ ID NO: 24 or a pharmaceutically acceptable salt thereof;
x) SEQ ID NO: 25 or a pharmaceutically acceptable salt thereof;
y) SEQ ID NO: 26 or a pharmaceutically acceptable salt thereof;
z) SEQ ID NO: 27 or a pharmaceutically acceptable salt thereof;
aa) SEQ ID NO: 28 or a pharmaceutically acceptable salt thereof; or
bb) SEQ ID NO: 29 or a pharmaceutically acceptable salt thereof.

2. A pharmaceutical composition comprising:
a therapeutically effective amount of a pre-implantation factor (PIF) peptide, mimetics thereof, analogs thereof, or a pharmaceutically acceptable salt thereof and
a pharmaceutically acceptable carrier for use in a method of treating or preventing muscle degeneration in a subject; and
wherein the PIF peptide is:
a) SEQ ID NO: 1 or a pharmaceutically acceptable salt thereof;
b) SEQ ID NO: 2 or a pharmaceutically acceptable salt thereof;
c) SEQ ID NO: 3 or a pharmaceutically acceptable salt thereof;
d) SEQ ID NO: 4 or a pharmaceutically acceptable salt thereof;
e) SEQ ID NO: 5 or a pharmaceutically acceptable salt thereof;
f) SEQ ID NO: 6 or a pharmaceutically acceptable salt thereof;
g) SEQ ID NO: 7 or a pharmaceutically acceptable salt thereof;
h) SEQ ID NO: 8 or a pharmaceutically acceptable salt thereof;
i) SEQ ID NO: 9 or a pharmaceutically acceptable salt thereof;
j) SEQ ID NO: 10 or a pharmaceutically acceptable salt thereof;
k) SEQ ID NO: 11 or a pharmaceutically acceptable salt thereof;
l) SEQ ID NO: 12 or a pharmaceutically acceptable salt thereof;
m) SEQ ID NO: 14 or a pharmaceutically acceptable salt thereof;
n) SEQ ID NO: 15 or a pharmaceutically acceptable salt thereof;
o) SEQ ID NO: 16 or a pharmaceutically acceptable salt thereof;
p) SEQ ID NO: 17 or a pharmaceutically acceptable salt thereof;
q) SEQ ID NO: 18 or a pharmaceutically acceptable salt thereof;
r) SEQ ID NO: 19 or a pharmaceutically acceptable salt thereof;
s) SEQ ID NO: 20 or a pharmaceutically acceptable salt thereof;
t) SEQ ID NO: 21 or a pharmaceutically acceptable salt thereof;
u) SEQ ID NO: 22 or a pharmaceutically acceptable salt thereof;
v) SEQ ID NO: 23 or a pharmaceutically acceptable salt thereof;
w) SEQ ID NO: 24 or a pharmaceutically acceptable salt thereof;
x) SEQ ID NO: 25 or a pharmaceutically acceptable salt thereof;
y) SEQ ID NO: 26 or a pharmaceutically acceptable salt thereof;
z) SEQ ID NO: 27 or a pharmaceutically acceptable salt thereof;
aa) SEQ ID NO: 28 or a pharmaceutically acceptable salt thereof; or
bb) SEQ ID NO: 29 or a pharmaceutically acceptable salt thereof.

3. The pharmaceutical composition for used as claimed in claim 1 or 2, wherein the pharmaceutically acceptable carrier is sterile and pyrogen-free water or sterile and pyrogen-free Lactated ringer's solution.

4. The pharmaceutical composition for used as claimed in claim 1 or 2, wherein the therapeutically effective dose is from about 0.001 mg/kg to about 200 mg/kg or from about 0.5 mg/kg to about 5 mg/kg, wherein kg is kilograms of the subject and mg is milligrams of the PIF peptide, or mimetic, analog or pharmaceutically acceptable salt thereof

5. The pharmaceutical composition for used as claimed in claim 1 or 2, wherein the therapeutically effective dose is:
a) about 1.0 mg/kg;
b) about 2.0 mg/kg;
c) about 3.0 mg/kg;
d) about 4.0 mg/kg;
e) about 0.2 mg/kg;
f) about 0.3 mg/kg;
g) about 0.4 mg/kg;
h) about 0.5 mg/kg;
i) about 0.6 mg/kg;
j) about 0.7 mg/kg; or
k) about 0.8 mg/kg,
wherein kg is kilograms of the subject and mg is milligrams of the PIF peptide, or mimetic, analog or pharmaceutically acceptable salt thereof.

6. The pharmaceutical composition for used as claimed in any of claims 1-5, wherein the at least the PIF peptide, or mimetic, analog or pharmaceutically acceptable salt thereof comprises a chemical targeting moiety and/or a radioactive moiety, optionally wherein the at least one PIF peptide, or mimetic, analog or pharmaceutically acceptable salt thereof comprises at least one radioactive moiety comprising at least one or a combination of the following isotopes: ²H, ³H, ¹³C, ¹⁴C, ¹⁵N, ¹⁶O, ¹⁷O, ³¹P, ³²P, ³⁵S, ¹⁸F, and ³⁶Cl.

7. The pharmaceutical composition for used as claimed in any of claims 1-6, wherein the pharmaceutical composition is suitable for administration with at least one analgesic and/or one anti-inflammatory compound, optionally wherein the at least one analgesic and/or one anti-inflammatory compound is suitable for administration before, after, or simultaneously with the administration of a therapeutically effective dose of at least one PIF peptide, or mimetic, analog or pharmaceutically acceptable salt thereof

8. The pharmaceutical composition for used as claimed in any of claims 1-7, wherein the pharmaceutical composition further comprises a therapeutically effective dose of one or a plurality of active agents, optionally wherein the one or plurality of active agents is one or a combination of compounds chosen from: an anti-inflammatory compound, alpha-adrenergic agonist, antiarrhythmic compound, analgesic compound, and an anesthetic compound.

9. The pharmaceutical composition for used as claimed in any of claims 1-8, wherein the pharmaceutical composition is free of SEQ ID NO: 1 or a pharmaceutically acceptable salt thereof.

10. A pharmaceutical composition comprising:
a therapeutically effective amount of a pre-implantation factor (PIF) peptide, an analog thereof, or a pharmaceutically acceptable salt thereof; and
a pharmaceutically acceptable carrier for use in a method of improving the clinical outcome in a subject suffering with, diagnosed with or suspected of having a dystrophin-related disorder; and
wherein the PIF peptide is:
a) SEQ ID NO: 1 or a pharmaceutically acceptable salt thereof;
b) SEQ ID NO: 2 or a pharmaceutically acceptable salt thereof;
c) SEQ ID NO: 3 or a pharmaceutically acceptable salt thereof;
d) SEQ ID NO: 4 or a pharmaceutically acceptable salt thereof;
e) SEQ ID NO: 5 or a pharmaceutically acceptable salt thereof;
f) SEQ ID NO: 6 or a pharmaceutically acceptable salt thereof;
g) SEQ ID NO: 7 or a pharmaceutically acceptable salt thereof;
h) SEQ ID NO: 8 or a pharmaceutically acceptable salt thereof;
i) SEQ ID NO: 9 or a pharmaceutically acceptable salt thereof;
j) SEQ ID NO: 10 or a pharmaceutically acceptable salt thereof;
k) SEQ ID NO: 11 or a pharmaceutically acceptable salt thereof;
l) SEQ ID NO: 12 or a pharmaceutically acceptable salt thereof;
m) SEQ ID NO: 14 or a pharmaceutically acceptable salt thereof;
n) SEQ ID NO: 15 or a pharmaceutically acceptable salt thereof;
o) SEQ ID NO: 16 or a pharmaceutically acceptable salt thereof;
p) SEQ ID NO: 17 or a pharmaceutically acceptable salt thereof;
q) SEQ ID NO: 18 or a pharmaceutically acceptable salt thereof;
r) SEQ ID NO: 19 or a pharmaceutically acceptable salt thereof;
s) SEQ ID NO: 20 or a pharmaceutically acceptable salt thereof;
t) SEQ ID NO: 21 or a pharmaceutically acceptable salt thereof;
u) SEQ ID NO: 22 or a pharmaceutically acceptable salt thereof;
v) SEQ ID NO: 23 or a pharmaceutically acceptable salt thereof;
w) SEQ ID NO: 24 or a pharmaceutically acceptable salt thereof;
x) SEQ ID NO: 25 or a pharmaceutically acceptable salt thereof;
y) SEQ ID NO: 26 or a pharmaceutically acceptable salt thereof;
z) SEQ ID NO: 27 or a pharmaceutically acceptable salt thereof;
aa) SEQ ID NO: 28 or a pharmaceutically acceptable salt thereof; or
bb) SEQ ID NO: 29 or a pharmaceutically acceptable salt thereof.

11. The pharmaceutical composition for use as claimed in any of claims 1-10, wherein the pharmaceutical composition is suitable for administration via parenteral injection, subcutaneous injection, intravenous injection, intramuscular injection, intraperitoneal injection, transdermally, orally, buccally, ocular routes, intravaginally, by inhalation, by depot injections, or by implants.

12. The pharmaceutical composition for use as claimed in claim 1 or 10, wherein the pharmaceutical composition is suitable for administration with one or a plurality of compositions comprising active agents selected from aspirin, celecoxib, diclofenac, diflunisal, etodolac, ibuprofen, indomethacin, ketoprofen, ketorolac nabumetone, naproxen, oxaprozin, piroxicam, salsalate, sulindac, tolmetin, Methoxamine, Methylnorepinephrine, Midodrine, Oxymetazoline, Metaraminol, Phenylephrine, Clonidine (mixed alpha2-adrenergic and imidazoline-Il receptor agonist), Guanfacine, (preference for alpha2A-subtype of adrenoceptor), Guanabenz (most selective agonist for alpha2-adrenergic as opposed to imidazoline-Il), Guanoxabenz (metabolite of guanabenz), Guanethidine (peripheral alpha2-receptor agonist), Xylazine, Tizanidine, Medetomidine, Methyldopa, Fadolmidine, Dexmedetomidine, Amiodarone (Cordarone, Pacerone), Bepridil Hydrochloride (Vascor), Disopyramide (Norpace), Dofetilide (Tikosyn), Dronedarone (Multaq), Flecainide (Tambocor), Ibutilide (Corvert), Lidocaine (Xylocaine), Procainamide (Procan, Procanbid), Propafenone (Rythmol), Propranolol (Inderal), Quinidine (many trade names), Sotalol (Betapace), Tocainide (Tonocarid), codeine, hydrocodone (Zohydro ER), oxycodone (OxyContin, Roxicodone), methadone, hydromorphone (Dilaudid, Exalgo), morphine (Avinza, Kadian, MSIR, MS Contin), fentanyl (Actiq, Duragesic), Desflurane, Isoflurane, Nitrous oxide, Sevoflurane, and Xenon sequentially or comtemporaneously.

13. The pharmaceutical composition for use as claimed in any of claims 1-12, wherein the subject is human.

14. A pharmaceutical composition comprising:
a therapeutically effective amount of a PIF peptide, mimetics thereof, analogs thereof, or a pharmaceutically acceptable salt thereof; and
a pharmaceutically acceptable carrier for use in a method of treating or preventing fibrosis in a subject in need thereof, optionally wherein the fibrosis is muscle fibrosis.

15. The pharmaceutical composition for used as claimed in claim 1, 2 or 14, wherein the PIF peptide is one or a combination of any one or plurality of: SEQ ID NO: 1, SEQ ID NO:2, SEQ ID NO:3, SEQ ID NO:4, SEQ ID NO:5, SEQ ID NO:6 SEQ ID NO:7, SEQ ID NO:8, SEQ ID NO:9, SEQ ID NO:10, SEQ ID NO:11, SEQ ID NO:12, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, and/or SEQ ID NO:29, or pharmaceutically acceptable salt thereof.

16. The pharmaceutical composition for used as claimed in claim 15, wherein the therapeutically effective amount of the PIF peptide, or mimetic, analog or pharmaceutically acceptable salt thereof is from about 0.001 mg/kg to about 200 mg/kg.
